# EUROPÄISCHE PATENTSCHRIFT

(11) **EP 1 891 011 B1**
(45) Veröffentlichungstag und Bekanntmachung des Hinweises auf die Patenterteilung: **08.12.2010**
(21) Anmeldenummer: 06754091.4
(22) Anmeldetag: 02.06.2006
(51) Int. Cl.: C07D 215/42, C07D 215/54, C07D 215/12, C07D 471/08, C07D 409/04, C07D 405/04, C07D 221/06, A61K 31/47, A61P 35/00

(54) **TETRAHYDROCHINOLINE ZUR VERWENDUNG ALS MODULATOREN DES MITOTISCHEN MOTOR- PROTEIN EG5**
TETRAHYDROQUINOLINES USED IN THE FORM OF MODULATORS OF MITOTIC MOTOR-PROTEINS EG5
TETRAHYDROQUINOLEINES COMME MODULATEURS DE LA PROTEINE MOTEUR MITOTIQUE EG5

(30) Priorität: 13.06.2005 DE 102005027168
(43) Veröffentlichungstag der Anmeldung: 27.02.2008
(73) Patentinhaber: Merck Patent GmbH, 64293 Darmstadt (DE)
(72) Erfinder: FINSINGER, Dirk, 64291 Darmstadt (DE); AMENDT, Christiane, 64367 Muehltal/Trautheim (DE); ZENKE, Frank, 64291 Darmstadt (DE); SCHIEMANN, Kai, 64342 Seeheim- Jugenheim (DE); BRUGE, David, 60329 Frankfurt (DE); BUCHSTALLER, Hans-Peter, 64347 Griesheim (DE); EMDE, Ulrich, 64291 Darmstadt (DE)
(86) Internationale Anmeldenummer: PCT/EP2006/005297
(87) Internationale Veröffentlichungsnummer: WO 2006/133821

(56) Entgegenhaltungen:
- EP-A- 1 395 563
- WO-A-97/30054
- WO-A-2004/078758
- V.N. GOGTE ET AL.: "Synthesis of tetrahydroquinolines involving rearrangement of N-arylazetidines as likely intermediates" TETRAHEDRON LETT., Bd. 39, 1969, Seiten 3319-3322, XP002396699
- STEVEN W. GOLDSTEIN ET AL.: "2-Substituted 1,2,3,4-tetrahydroquinolines from quinoline" SYNTHESIS, Bd. 3, 1989, Seiten 221-222, XP002396700
- KOHNO Y ET AL: "Synthesis and antirheumatic activity of novel tetrahydro-6-quinolinea-cetic acid derivatives" BIOORGANIC & MEDICINAL CHEMISTRY LETTERS, OXFORD, GB, Bd. 7, Nr. 12, 17. Juni 1997 (1997-06-17), Seiten 1519-1524, XP004136249 ISSN: 0960-894X
- ABBIATI G ET AL: "A valuable heterocyclic ring transformation: from isoxazolin-5(2H)-ones to quinolines" TETRAHEDRON, ELSEVIER SCIENCE PUBLISHERS, AMSTERDAM, NL, Bd. 59, Nr. 50, 8. Dezember 2003 (2003-12-08), Seiten 9887-9893, XP004474431 ISSN: 0040-4020
- MICHEL C. MAILLARD ET AL.: "Design, synthesis and pharmacological evaluation of conformationally constrained analogues of N,N-diaryl- and N-aryl-N-araalkylguanidines as potent inhibitors of neuronal Na+ channels" J. MED. CHEM., Bd. 41, 1998, Seiten 3048-3061, XP002396701
- WALLACE O B ET AL: "Tetrahydroquinoline-based selective estrogen receptor modulators (SERMs)" BIOORGANIC & MEDICINAL CHEMISTRY LETTERS, OXFORD, GB, Bd. 13, Nr. 11, 2003, Seiten 1907-1910, XP002301445 ISSN: 0960-894X

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen der Formel I und deren Verwendung zur Behandlung und Prophylaxe von Krankheiten, bei denen die Hemmung, Regulierung und/oder Modulation der mitotische Motor-Proteine, insbesondere des mitotischen Motor-Protein Eg5 eine Rolle spielt, ferner pharmazeutische Zusammensetzungen, die diese Verbindungen enthalten.

Im einzelnen betrifft die vorliegende Erfindung Verbindungen der Formel I, die die bevorzugt eines oder mehrere mitotische Motor-Proteine hemmen, regulieren und/oder modulieren, Zusammensetzungen, die diese Verbindungen enthalten, sowie Verfahren zu ihrer Verwendung zur Behandlung von Krankheiten und Leiden wie Angiogenese, Krebs, Tumorentstehung, -wachstum und -verbreitung, Arteriosklerose, Augenerkrankungen, choroidale Neovaskularisierung und diabetische Retinopathie, Entzündungserkrankungen, Arthritis, Neurodegeneration, Restenose, Wundheilung oder Transplantatabstossung. Insbesondere eignen sich die erfindungsgemäßen Verbindungen zur Therapie oder Prophylaxe von Krebserkrankungen.

Während der Mitose regulieren verschiedenen Kinesine die Ausbildung und Dynamik des Spindelapparates, der für eine korrekte und koordinierte Ausrichtung und Separation der Chromosomen verantwortlich ist. Es wurde beobachtet, dass eine spezifische Inhibierung eines mitotischen Motor-Proteins - Eg5 - zu einem Kollaps der Spindelfasern führt. Daraus resultiert, dass die Chromosomen nicht mehr korrekt auf die Tochterzellen aufgeteilt werden können. Dies führt zu mitotischem Arrest und kann in der Folge das Absterben der Zelle verursachen. Eine Hochregulierung des Motorproteins Eg5 wurde z.B. in Gewebe von Brust- Lungen- und Colon- Tumoren beschrieben. Da Eg5 eine für die Mitose spezifische Funktion einnimmt, sind hauptsächlich sich schnell teilende Zellen und nicht vollständig ausdifferenzierte Zellen von einer Eg5 Inhibierung betroffen. Darüber hinaus regelt Eg5 ausschließlich die Bewegung mitotischer Mikrotubuli (Spindelapparat) und nicht die des Cytoskeletts. Dies ist entscheidend für das Nebenwirkungsprofil der erfindungsgemäßen Verbindungen, da z.B. Neuropathien, wie sie bei Taxol beobachtet werden, nicht oder nur abgeschwächt auftreten. Daher ist die Inhibierung von Eg5 durch die erfindungsgemäßen Verbindungen ein relevantes Therapiekonzept für die Behandlung von malignen Tumoren.

Generell können alle soliden und nicht soliden Tumore mit den Verbindungen der Formel I behandelt werden, wie z.B. die Monozytenleukämie, Hirn-, Urogenital-, Lymphsystem-, Magen-, Kehlkopf- und Lungenkarzinom, darunter Lungenadenokarzinom und kleinzelliges Lungenkarzinom. Zu weiteren Beispielen zählen Prostata-, Bauchspeicheldrüsen- und Brustkarzinom.

Es wurde überraschend gefunden, daß die erfindungsgemäßen Verbindungen eine spezifische Inhibierung der mitotischen Moter-Proteine, insbesondere Eg5 bewirken. Die erfindungsgemäßen Verbindungen zeigen bevorzugt eine vorteilhafte biologische Aktivität, die in den zum Beispiel hierin beschrieben Assays leicht nachweisbar ist. In derartigen Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert werden kann.

Wie hierin besprochen, sind Wirkungen der erfindungsgemäßen Verbindung für verschiedene Erkrankungen relevant. Dementsprechend sind die erfindungsgemäßen Verbindungen nützlich bei der Prophylaxe und/oder Behandlung von Erkrankungen, die durch eine Inhibierung eines oder mehreren mitotischer Motor-Proteine, insbesondere Eg5, beeinflusst werden.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen in einem Xenotransplantat-Tumor-Modell eine vorteilhafte Wirkung aufweisen.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den Wirkstoffen zu ermöglichen, Zellproliferation zu inhibieren oder Zelltod zu induzieren, gewöhnlich zwischen ungefähr einer Stunde und einer Woche. Zum Testen in vitro können kultivierte Zellen aus einer Biopsieprobe oder etablierte Zell-Linien verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

### ZUSAMMENFASSUNG DER ERFINDUNG

Die Erfindung betrifft Verbindungen der Formel I 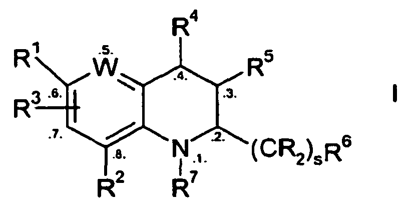 worin
- W: CH oder N,
- R¹, R², R³: unabhängig voneinander H, A, Aryl, Heteroaryl, Hal, - (CY₂)ₙ-SA, -(CY₂)ₙ-SCF₃, -(CY₂)ₙ-SCN, -(CY₂)ₙ-CF₃, -(CY₂)ₙ- OCF₃, Cycloalkyl, -SCH₃, -SCN, -CF₃, -OCF₃, -OA, -(CY₂)ₙ- OH, -(CY₂)ₙ-CO₂R, -(CY₂)ₙ-CN, -(CY₂)ₙ-Hal, (CH₂)ₙR, -(CY₂)ₙ-NR₂, (CY₂)ₙ-OR, (CY₂)ₙ-OCOA, -SCF₃, (CY₂)ₙ-CONR₂, -(CY₂)ₙ-NHCOA, -(CY₂)ₙ-NHSO₂A, SF₅, Si(CH₃)₃, CO-(CY₂)ₙ- CH₃, -(CY₂)ₙ-N-Pyrolidon, (CH₂)ₙNRCOOR, NRCOOR, NCO, CH₂(CH₂)ₙCOOR, NHCOOR, CH₂(CH₂)ₙOH, NR(CH₂)ₙOH, CH₂NH₂, (CH₂)ₙNR₂, CH(OH)R₂, CH₂NHCOR, (CH₂)ₙAryl, CH₂(CH₂)ₙHeteroaryl, (CH₂)ₙR¹, NH(CH₂)ₙCOOR, CH₂(CH₂)ₙX(CH₂)ₙAryl, CH₂(CH₂)ₙX(CH₂)ₙHeteroaryl, NH(CH₂)ₙCONR₂, XCONR(CH₂)ₙNR₂, N[(CH₂)ₙXCOOR]CO(CH₂)ₙAryl, N[(CH₂)ₙXR]CO(CH₂)ₙAryl, N[(CH₂)ₙXR]CO(CH₂)ₙXAryl, N[(CH₂)ₙXR]SO₂(CH₂)ₙAryl, N[(CH₂)ₙNRCOOR]CO(CH₂)ₙAryl, N[(CH₂)ₙNR₂]CO(CH₂)ₙAryl, N[(CH₂)ₙNR₂]CO(CH₂)ₙNRAryl, N[(CH₂)ₙNR₂]SO₂(CH₂)ₙAryl, N[(CH₂)ₙXR]CO(CH₂)ₙHeteroaryl, N[(CH₂)ₙXR]CO(CH₂)ₙXHeteroaryl, N[(CH₂)ₙXR]SO₂(CH₂)ₙHeteroaryl, N[(CH₂)ₙNRCOOR]CO(CH₂)ₙHeteroaryl, N[(CH₂)ₙNR₂]CO(CH₂)ₙHeteroaryl, N[(CH₂)ₙNR₂]CO(CH₂)ₙNRHeteroaryl wobei nicht benachbarte Gruppen CY₂ auch durch X ersetzt sein können, R¹ und R³ zusammen auch -N-C(CF₃)=N-, -N-CR=N-, -N-N=N-,
- Y: H, A, Hal, OR¹, N(R¹)₂, E-R¹
- A: Alkyl oder Cycloalkyl, worin eines oder mehrere H-Atome durch Hal und/oder eine oder mehrere nicht benachbarte CH₂ Gruppen durch X ersetzt sein können,
- Hal: F, Cl, Br oder I
- R: H oder A, bei geminalen Resten R zusammen auch -(CH₂)₅-, - (CH₂)₄-oder-(CH₂)ₙ-X-(CH₂)ₙ, oder-(CH2)ₙ₋Z-(CH₂)ₙ,
- R⁴: H
- R⁵: -(CY₂)ₙ-E-(CY₂)ₙ-XR¹, (CH₂)₂OH, (CH₂)₃OH, (CH₂)₂NRR¹, (CH₂)₃NRR¹, (CH₂)₂CH(OH)CH₂OH, (CH₂)₂CH(OH)CH₂NRR¹, (CH₂)₂CH(OH)CH₂NR(CH₂)₂NRR¹, (CH₂)₂CH(OH)CH₂NR⁵(CH₂)₂NR, (CH₂)₂CH(OH)CH₂-E-R¹, Q oder (CH₂)₂CH(OH)CH₂NR(CH₂)₂OR,
- E: -NR¹SO₂-, -SO₂NR¹-. -CONR¹-, -NR¹CO-, -COO-, -OOC-, NR¹CONR¹-, -OCONR¹-, -NR¹COO-, -CSNR¹-, -NR¹CS-, - NR¹CSNR¹-, -SCONR¹-, -NR¹COS-, -OCSNR¹-, NR¹CSO-, SCSNR¹-, -NR¹CSS oder eine Einfachbindung
- X: O, S oder NR¹,
- Q: (CH₂)ₚHal, CHO, COR^{a}, (CH₂)ₚR^{a}, (CH₂)ₚOCOR^{a}, (CH₂)ₚXR¹, (CH₂)ₚNCOR¹, (CH₂)ₚN(R¹)₂, (CH₂)ₚOR¹, (CH₂)ₚOCON(R¹)₂, (CH₂)ₚOCOOR¹, (CH₂)ₚNHCON(R¹)₂, (CH₂)ₚNHCOOR¹, (CH₂)ₚCN, (CH₂)ₚCOOR¹, (CH₂)ₚ-E- (CH₂)ₚR¹, (CH₂)ₚ-E-(CH₂)ₚR^{a}, wobei nicht benachbarte CH₂- Gruppen auch durch X ersetzt warden können.
- R^{a}: 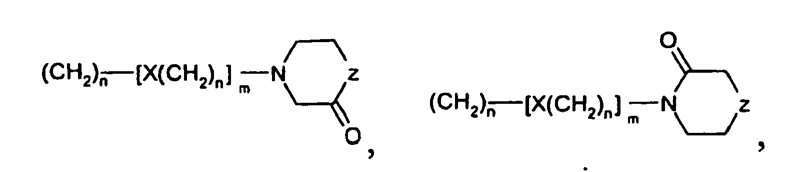 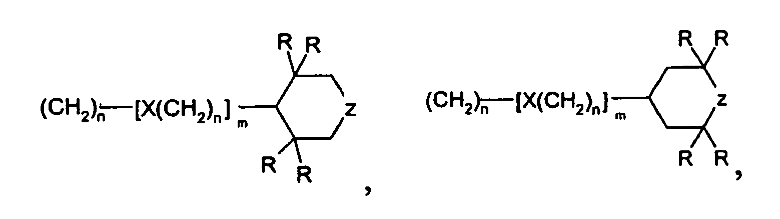 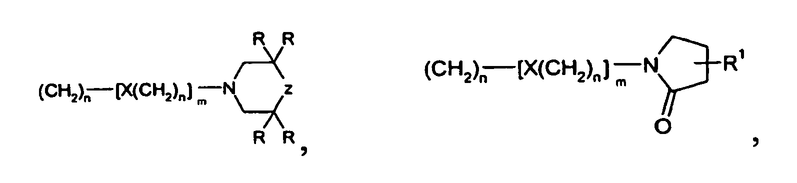 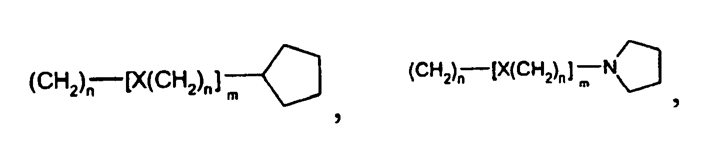 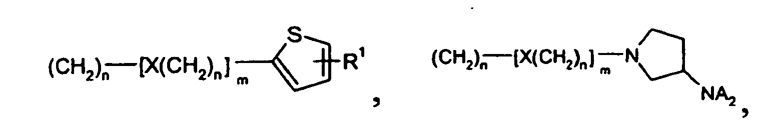 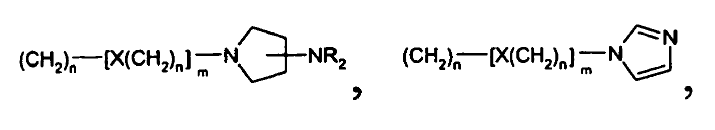 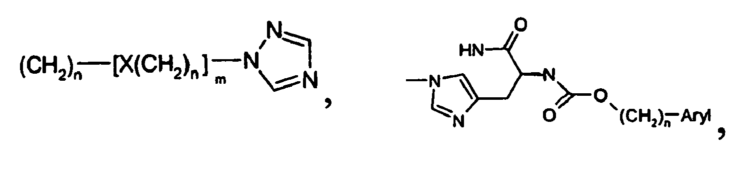 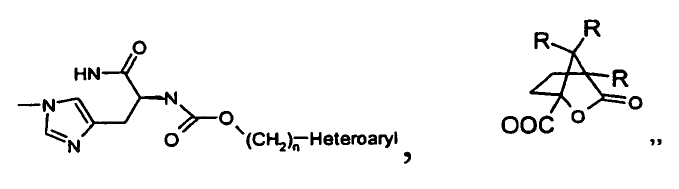 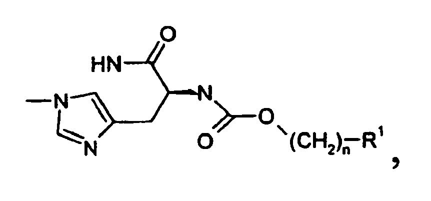 OR, NHR, NR₂, NR(CH₂)ₙAryl, NR(CH₂)ₙOR, COOR, N- Pyrrolidon-Rest, OCOR, NR(CH₂)ₙNR₂, N[(CH₂)ₙNR₂]CO(CH₂)ₙAryl, N[(CH₂)ₙNHCOOR]COAryl, R¹, N[CH₂(CH₂)ₙOR]₂, NR(CH₂)ₙNCOOR, X(CH₂)ₙX(CH₂)ₙXR, NR(CH₂)ₙX(CH₂)ₙOH, NR(CH₂)ₙO(CH₂)ₙOH, (CH₂)ₙCOOR, O(CO)NR(CH₂)ₙOR, O(CO)(CH₂)ₙNR₂, NR(CH₂)ₙNR₂, N[(CH₂)ₙNR₂]CO(CH₂)ₙAryl, N[(CH₂)ₙXR]CO(CH₂)ₙAryl, N[(CH₂)ₙXR]CO(CH₂)ₙHeteroaryl, N[(CH₂)ₙNR₂]CO(CH₂)ₙHeteroaryl, N[(CH₂)ₙNR₂]CO(CH₂)ₙR¹, N(R)(CH₂)ₙN(R)COOR, XCOO(CH₂)ₙNR₂, OSO₂A, OSO₂CF₃, OSO₂Ar, OCONR₂, OCH₂(CH₂)ₙNR₂
- Z: CH₂, X, CHCONH₂, CH(CH₂)ₙNR¹COOR¹, CHNR¹COOR¹, CHCON(R¹)₂, NCO, CH(CH₂)ₙCOOR¹, NCOOR¹, CH(CH₂)ₙOH, N(CH₂)ₙOH, CHNH₂, CH(CH₂)ₙN(R¹)₂, CH(CH₂)ₙN(R¹)₂, C(OH)R¹, CHNCOR¹, NCOR¹, N(CH₂)ₙAryl, N(CH₂)ₙHeteroaryl, CHR¹, NR¹, CH(CH₂)ₙAryl, CH(CH₂)ₙHeteroaryl, CH(CH₂)ₙR¹, N(CH₂)ₙCOOR¹, CH(CH₂)ₙX(CH₂)ₙR¹, CH(CH₂)ₙX(CH₂)ₙR^{a}, N(CH₂)ₙCON(R¹)₂, XCONR¹(CH₂)ₙN(R¹)₂, CO(CH₂)ₙR¹ , CO(CH₂)ₙR¹, CO(CH₂)ₙXR¹, SO₂(CH₂)ₙR¹, O(CH₂)ₙN(R¹)₂, X(CH₂)ₙN(R¹)₂, NCO(CH₂)ₙN(R¹)₂, CHR^{a}, NR^{a},
- R⁶: unsubstituiertes oder einfach oder mehrfach durch Aryl oder Heteroaryl, das durch Hal, NO₂, CN, A, OR, OCOR, COR, NR₂, CF₃, OCF₃, OCH(CF₃)₂ substituiert sein kann, Hal, NO₂, CN, OR, A, -(CY₂)ₙ-OR, -OCOR, -(CY₂)ₙ-CO₂R, -(CY₂)ₙ-CN, - NCOR, -COR oder -(CY₂)ₙ-NR₂ substituiertes Aryl oder Heteroaryl,
- R⁷: (C=O)-R, (C=O)NR₂, (C=O)-OR, H oder A, worin R⁵ und R⁷ zusammen auch -(CH₂)ₙ- bedeuten kann,
- m: 0, 1 oder 2
- n: 0, 1, 2, 3, 4, 5, 6 oder 7
und
- p: 0, 1, 2, 3, 4, oder 5, bevorzugt 2 oder 3
- S: 0, 1, 2, 3, 4, 5, 6 oder 7, bevorzugt 0
bedeuten,
sowie ihre Solvate, Tautomere, Salze und Stereoisomere, einschließlich deren Mischungen In allen Verhältnissen.

Gegenstand der Erfindung sind auch die optisch aktiven Formen, die Enantiomere, die Racemate, die Diastereomere sowie die Hydrate und Solvate dieser Verbindungen. Unter Solvaten der Verbindungen werden Anlagerungen von inerten Lösungsmittelmolekülen an die Verbindungen der Formel I verstanden, die sich aufgrund ihrer gegenseitigen Anziehungskraft ausbilden. Solvate sind z.B. Mono- oder Dihydrate oder Alkoholate.

Ähnliche Verbindungen sind z.B. in Tetrahedron Lett. 1988, 29, 5855-5858, Tetrahedron Lett. 2003, 44, 217-219, J. Org. Chem. 1997, 62, 4880-4882, J. Org. Chem. 1999, 64, 6462-6467, Chem. Lett. 1995, 423-424, J. Org. Chem. 2000, 65, 5009-5013, Chem. Lett. 2003, 32, 222-223. US2003149069A1 beschrieben, sind aber nicht im Zusammenhang mit Krebsbehandlungen genannt und/oder enthalten nicht die erfindungswesentlichen Merkmale.

Gleiches gilt für WO 2004/078758 A1 und deren fusionierte heterozyklische Verbindungen der Formel (I):

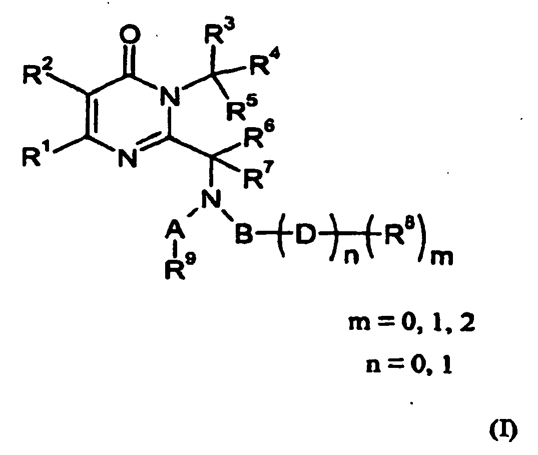

Die Radikale der Formel (I) sind als Markush-Gruppen definiert. Die Verbindungen können in pharmazeutischen Zusammensetzungen enthalten sein und in der Prophylaxe und Behandlung von Krebs Anwendung finden, ohne dass die Krebserkrankung spezifiziert ist

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird. Darüberhinaus bedeutet der Ausdruck "therapeutisch wirksame Menge" eine Menge, die in einem Menschen oder einem anderen Säuger mindestens eine der folgenden Wirkungen hervorruft (im Vergleich zu einem Subjekt, das diese Menge nicht erhalten hat):
Verbesserung der Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen oder zu verstärken.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I nach den Patentansprüchen sowie ihrer Salze, Solvate und Stereoisomeren, dadurch gekennzeichnet, daß man
eine Verbindung der Formel II 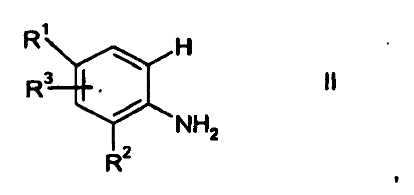 worin R¹, R² und R³ die oben angegebenen Bedeutungen haben,
mit einer Verbindung der Formel III 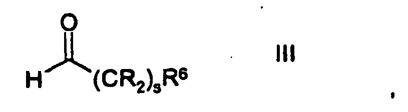 worin
R⁶ die oben angegebene Bedeutung aufweist,
und
mit einer Verbindung der Formel IV, 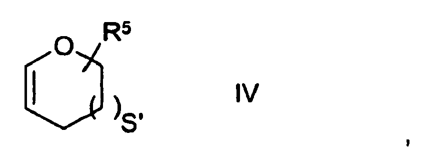 worin S' 0, 1 oder 2 bedeutet oder entsprechend substituierte Verbindungen,
bevorzugt in Gegenwart einer Protonensäure oder Lewis-Säure wie z.B. Trifluoressgsäure, Hexafluorisopropanol, Bismut (III)chlorid, Ytterbium(III)triflat, Scandium (III) triflat oder Cerammonium (IV)nitrat umsetzt.

Auf diese Weise werden die entsprechenden Tetrahydrochinoline erhalten, die durch Umsetzung mit wässriger Säure, wie z.B. Salzsäure in die analogen Chinoline umgewandelt werden. Je nach Reaktionsführung entstehen diese Chinoline auch direkt. Bevorzugte Chinoline sind die Verbindungen der Formel 1A.

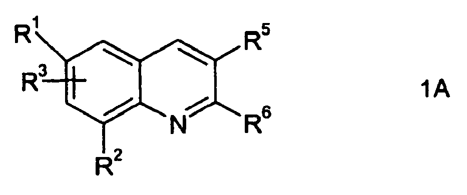

Die erhaltennen Chinoline werden vorzugsweise durch Anwendung von Wasserstoff in Gegenwart von Nickel oder Natrium in Gegenwart von Ethanol zu den Tetrahydrochinolinen reduziert, die dann beispielsweise nach Diastereomerentrennung durch Modifikation der Seitenketten wie z.B. R⁵ und Enantiomerentrennung in die Verbindungen der Formel I überführt werden, wie beispielhaft in nachfolgendem Schema dargestellt: 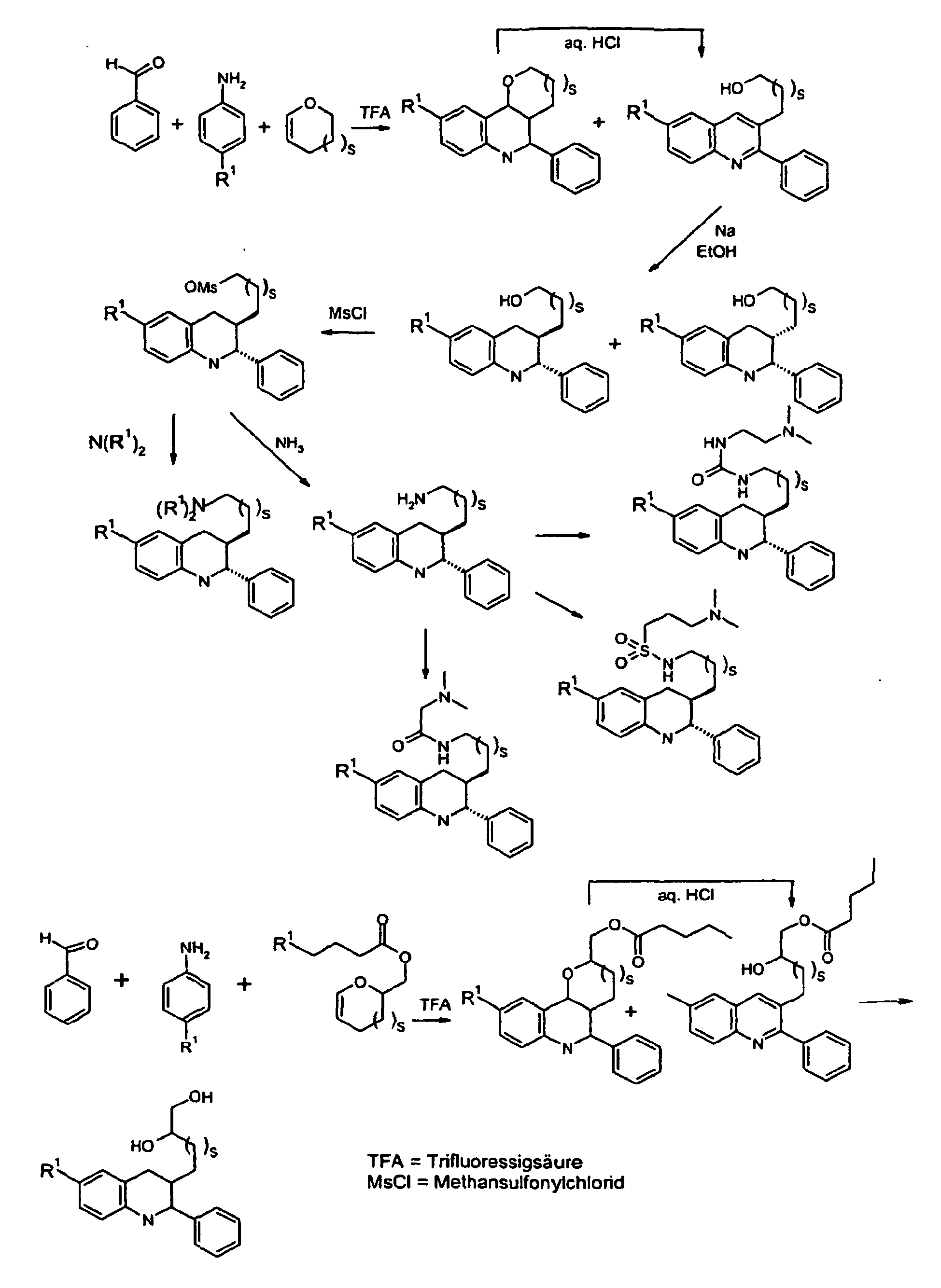 wobei R¹ die oben angegebene Bedeutung aufweist und s bevorzugt 0,1 oder 2 bedeutet, insbesondere 1.

Vorzugsweise werden die nach dem oben beschriebenen Verfahren gegebenenfalls erhaltenen Gemische von Diastereomeren und Enantiomeren der Verbindungen der Formel I durch Chromatographie oder Kristallisation aufgetrennt.

Gegebenenfalls werden die nach dem oben beschriebenen Verfahren erhaltenen Basen und Säuren der Formel I in ihre Salze umgewandelt.

Vor- und nachstehend haben die Reste R, R¹, R², R³, R⁴, R⁵, R⁶, R⁷, X, Y, E, Q, R^{a}, Z, W, p, s, m und n die bei der Formel I angegebenen Bedeutungen, sofern nicht ausdrücklich etwas anderes angegeben ist. Bei mehrfachem Auftreten einzelner Reste innerhalb einer Verbindung nehmen die Reste unabhängig voneinander die angegebenen Bedeutungen an.

A bedeutet Alkyl, ist bevorzugt unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1-, 2-, 3- oder 4-Methylpentyl, 1,1-, 1,2-, 1,3-, 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. A bedeutet auch Cycloalkyl.
Cycloalkyl bedeutet vorzugsweise Cyclopropyl, Cyclobutyl, Cylopentyl, Cyclohexyl oder Cycloheptyl, insbesondere aber Cyclopentyl.

E ist vorzugsweise -NR¹SO₂-, -SO₂NR¹-, -CONR¹-, -NR¹CO-, NR¹-CO-NR¹- oder-OCONR¹,
R¹ bedeutet vorzugsweise A, CF₃, OCF₃, SA, SCN, CH₂CN, -OCOA, Hal, SCF₃, bevorzugt auch t-Butyl, -CH(CH₃)CH₂CH₃, Isopropyl, Ethyl oder Methyl. Insbesondere bedeutet R¹ t-Butyl, Isopropyl, Ethyl, CF₃, Methyl, Br, Cl, SCF₃, CH(CH₃)CH₂CH₃, n-Propyl, OCH₃, SCH₃, n-Butyl, -SCN, CH₂CN. Besonders bevorzugt bedeutet R¹ t-Butyl, Isopropyl, Ethyl oder CF₃.
R² bedeutet bevorzugt Hal, A oder OA, insbesondere Br, Cyclopropyl, OCH₃. Weiterhin sind insbesondere bevorzugt H oder F.

R³ bedeutet vorzugsweise H oder A, insbesondere H. R³ steht bevorzugt in 5-position. Insbesondere bedeutet R³ H oder F.

In besonders bevorzugten Verbindungen der Formel I weisen R² und R³ gleichzeitig die Bedeutung H auf. In weiteren bevorzugten Verbindungen der Formel I weist einer der Reste R² und R³ die Bedeutung H und der andere Rest die Bedeutung F auf. R⁵ nimmt vorzugsweise eine der folgenden Bedeutungen an:
worin X, R¹, E, Y, n und R^{a} die oben angegebene Bedeutung aufweisen.

Besonders bevorzugt R⁵ eine der folgenden Bedeutungen an:
(CH₂)₂OH, (CH₂)₃OH, (CH₂)₂NRR¹, (CH₂)₃NRR¹, (CH₂)₂CH(OH)CH₂OH, (CH₂)₂CH(OH)CH₂NRR¹, (CH₂)₂CH(OH)CH₂NR(CH₂)₂NRR¹, (CH₂)₂CH(OH)CH₂NR⁵(CH₂)₂NR, (CH₂)₂CH(OH)CH₂-E-R¹, Q, (CH₂)₂CH(OH)CH₂NR(CH₂)₂OR,
worin R, R¹, E und Q die oben angegeben Bedeutung aufweisen.

R^{a} bedeutet vorzugsweise 1-Piperazinyl, N-Morpholinyl, NHR oder NR₂.

R⁶ bedeutet vorzugsweise unsubstituiertes oder einfach oder mehrfach durch Hal, CN, NO₂, OH, CF₃, OCH(CF₃)₂, OCOCH₃ oder A substituiertes Phenyl, 2-, 3- oder 4-Pyridyl, Pyrimidyl, Furyl oder Thienyl. Vorzugsweise ist R⁶ kein heteroaromatischer Rest. Insbesondere bedeutet R⁶ eine der folgenden Gruppen: 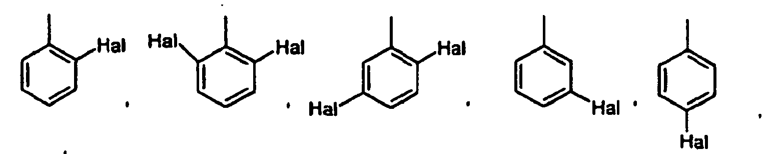 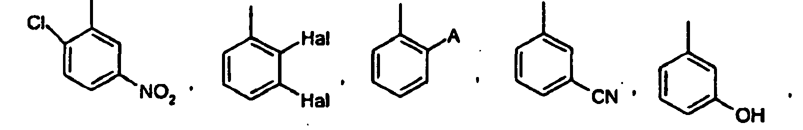 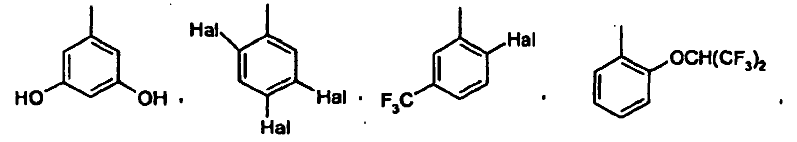 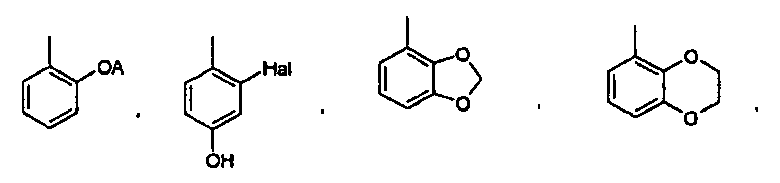 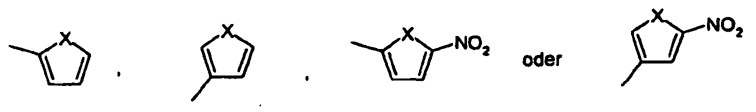 worin
- X: O, S oder NR und insbesondere O oder S bedeutet, A die oben angegebene Bedeutung aufweist, bevorzugt aber Methyl bedeutet und Hal bevorzugt F oder Cl bedeutet.

Weiterhin sind insbesondere Verbindungen der Formel I bevorzugt, worin R⁶ eine der folgenden Bedeutungen aufweist:

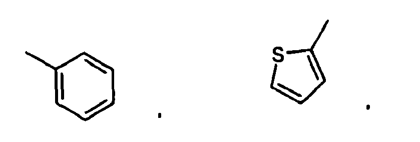

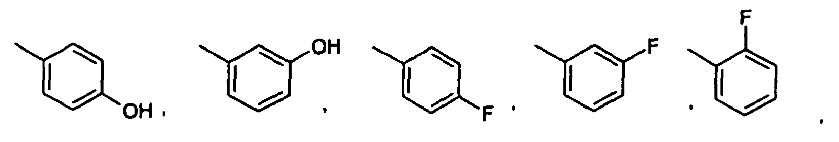

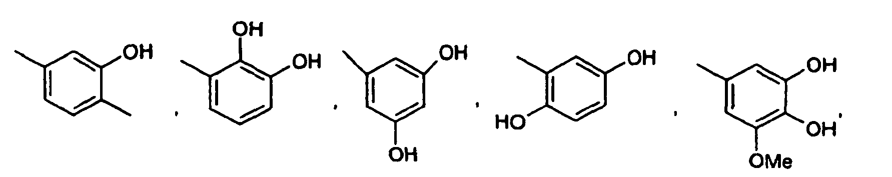

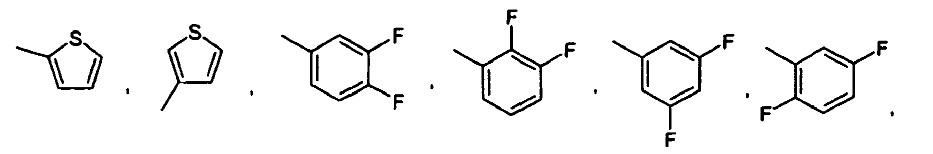

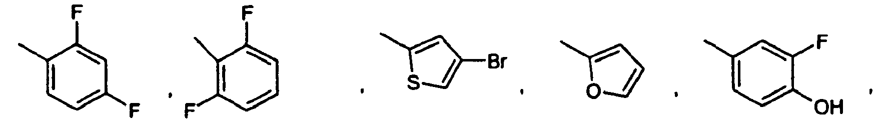

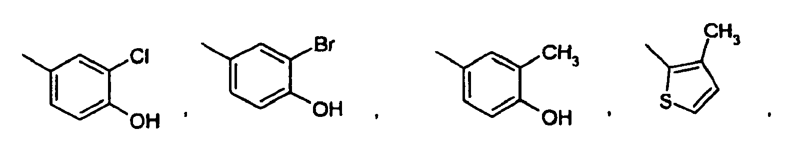

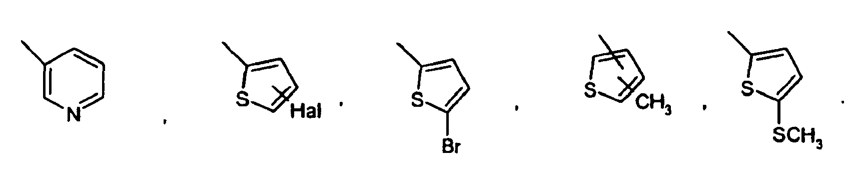

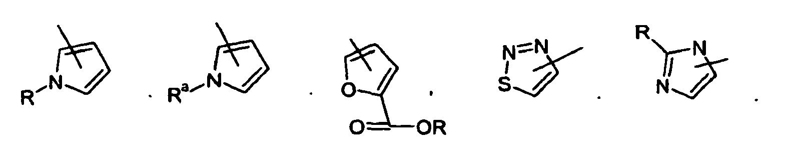

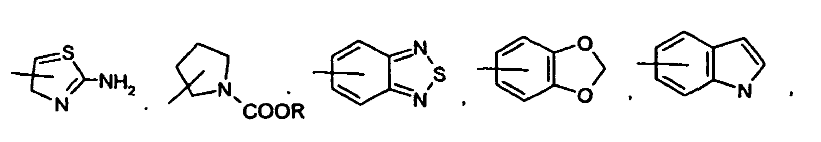

R⁷ bedeutet bevorzugt H oder A, insbesondere H.

Aryl bedeutet vorzugsweise unsubstituiertes oder ein-, zwei- oder dreifach durch Hal, A, OH, OA, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl.
Aryl bedeutet bevorzugt Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Methoxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-Iodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Heteroaryl bedeutet vorzugsweise einen ein- oder zweikernigen unsubstituierten oder ein-, zwei- oder dreifach durch Hal, A, NO₂, NHA, NA₂, OA, COOA oder CN substituierten aromatischen Heterocyclus mit einem oder mehreren N-, O- und/oder S-Atomen.

Heteroaryl bedeutet besonders bevorzugt einen einkemigen gesättigten oder aromatischen Heterocyclus mit einem N, S oder O-Atom, der unsubstituiert oder ein-, zwei- oder dreifach durch Hal, A, NHA, NA₂, NO₂, COOA oder Benzyl substituiert sein kann.

Ungeachtet weiterer Substitutionen, bedeutet unsubstituiertes Heteroaryl z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, - 4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-8enzodioxan-6-yl, 2,1,3-Benzothiadiazol-4- oder -5-yl oder 2,1,3-Benzoxadiazol-5-yl.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Besonders bevorzugte Verbindungen der Formel I sind die der Teilformeln IA bis IB: 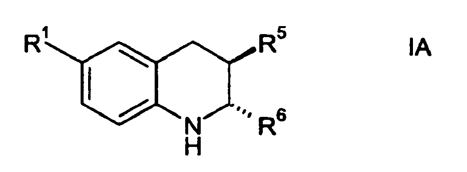 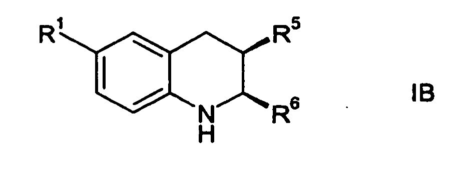 worin
R¹, R⁵, R⁶ die oben angegebenen Bedeutungen aufweisen, sowie deren Racemat oder andere Gemische der Enantiomeren. Verbindungen der Formel IA sind besonders bevorzugt.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln I1 bis I27 ausgedrückt werden: 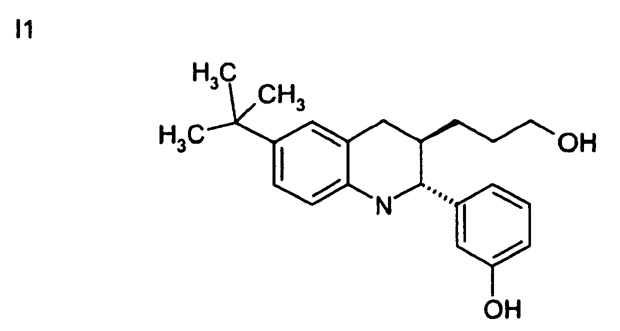 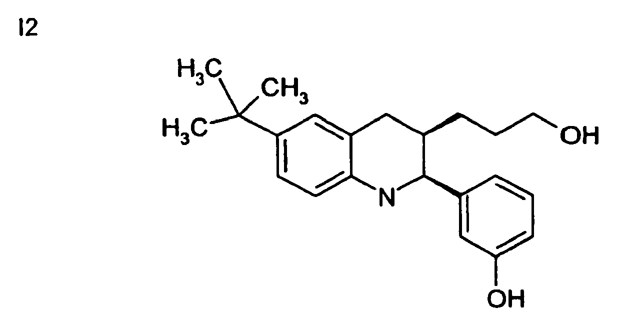 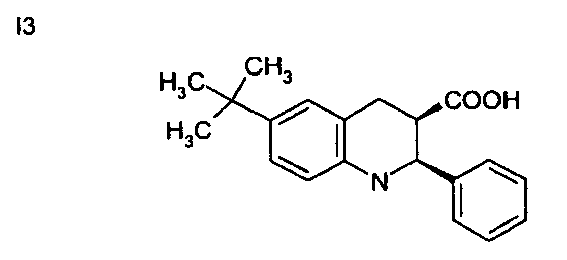 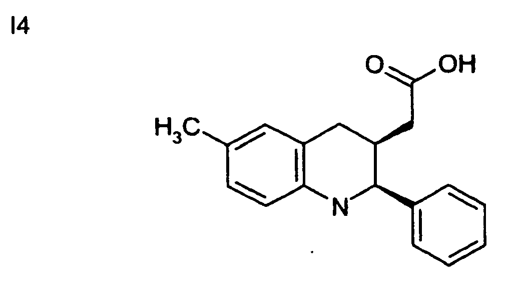 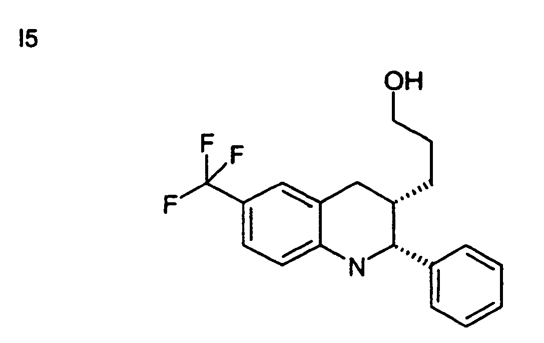 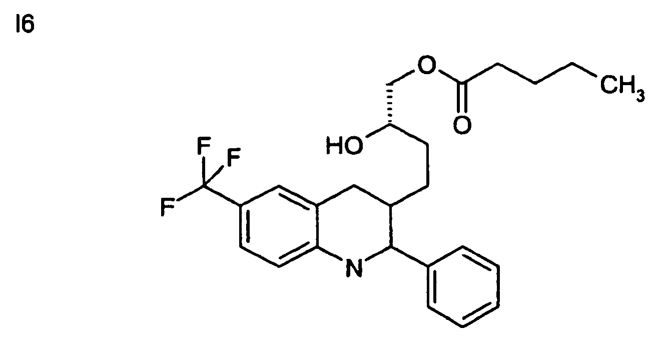 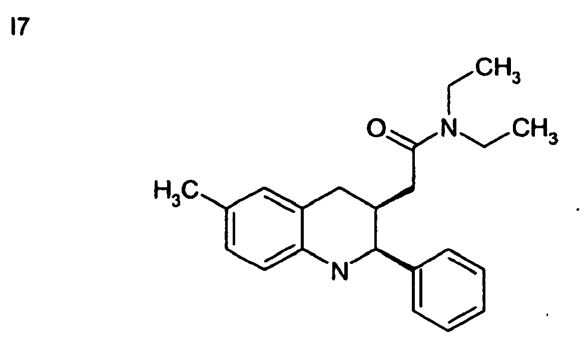 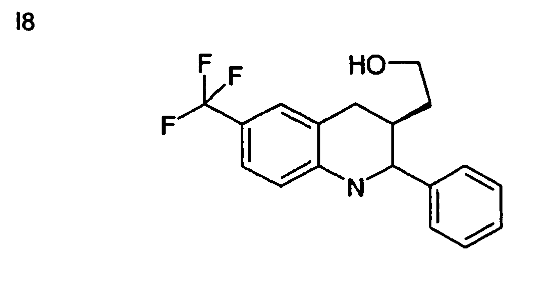 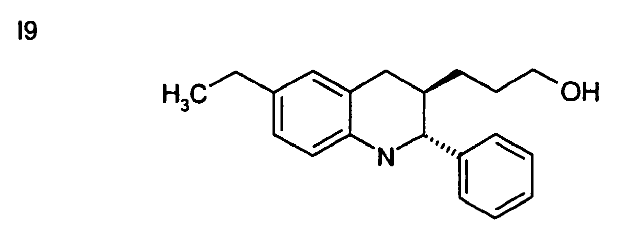 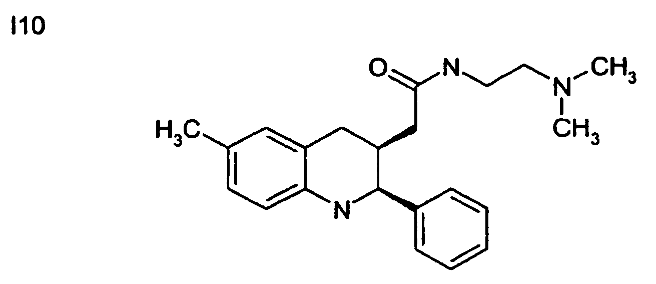 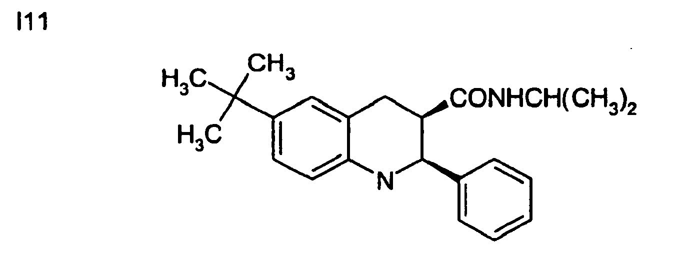 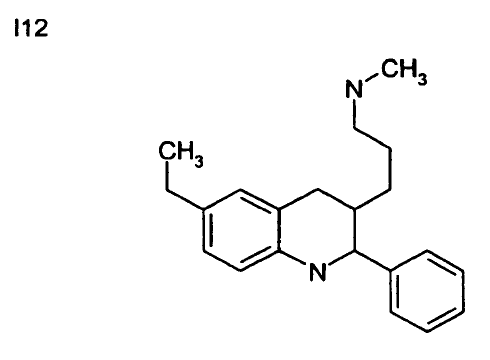 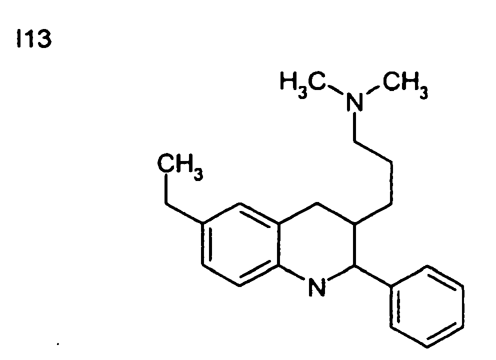 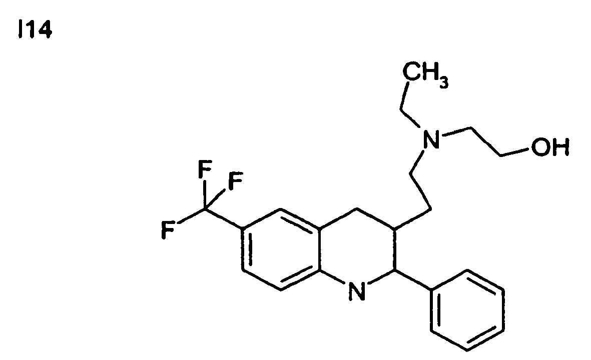 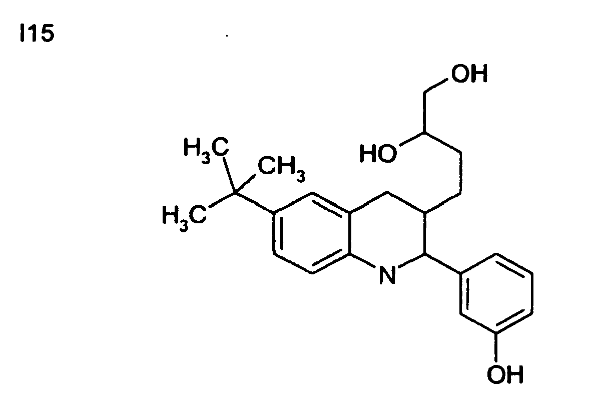 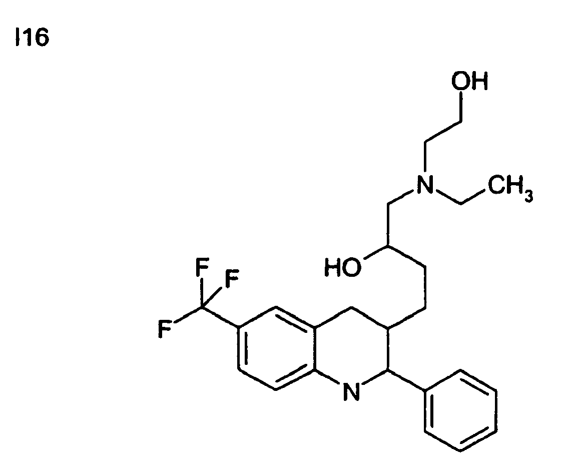 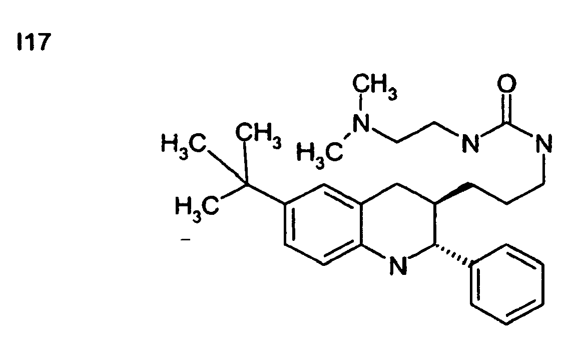 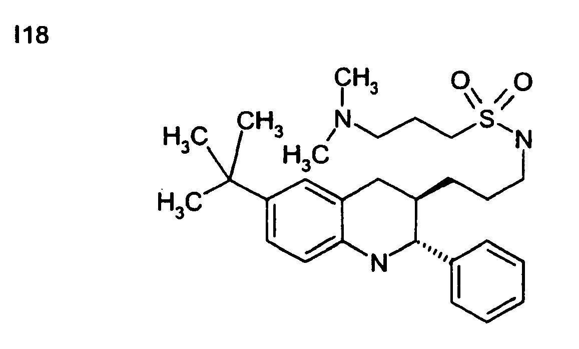 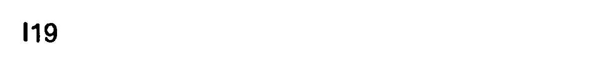 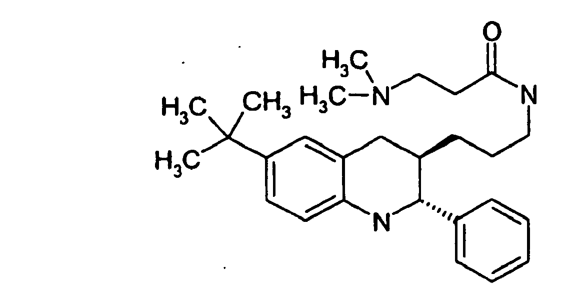 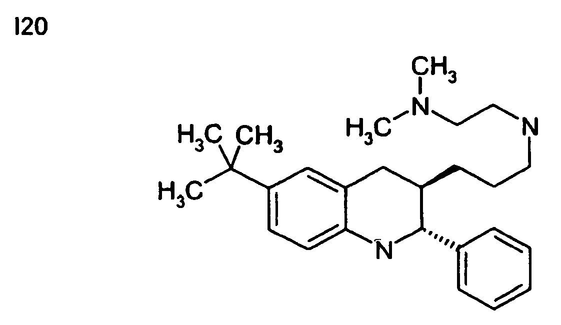 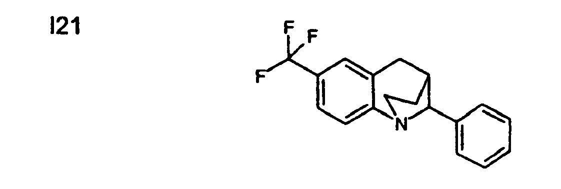 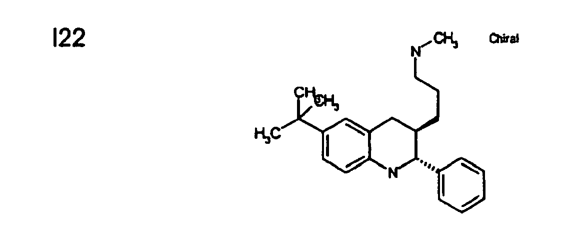 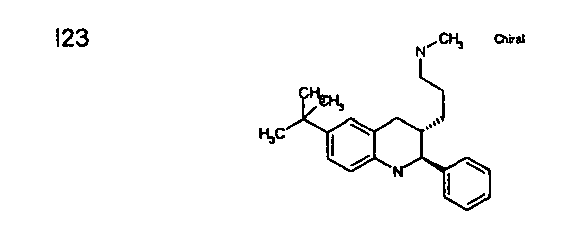 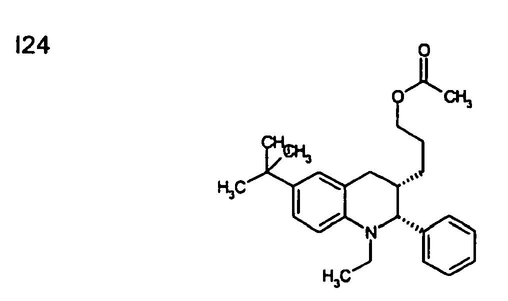 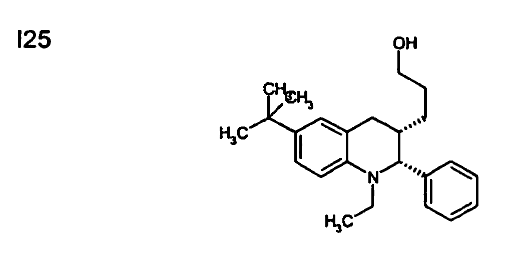 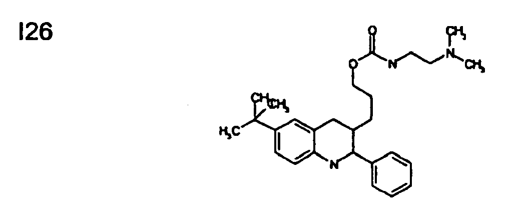 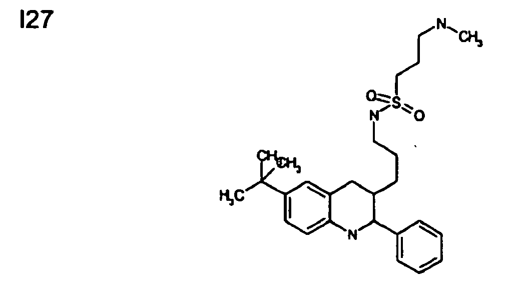 sowie deren Salze und Solvate, Diastereomere und Enantiomere sowie deren Gesmische in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Umsetzung erfolgt in der Regel in einem inerten Lösungsmittel, bevorzugt in Gegenwart einer Protonensäure oder Lewis-Säure wie TFA, HFIP, Bismut(III)-Salzen, Ytterhium(III)-Salzen oder CAN. Die Reaktionszeit liegt je nach den angewendeten Bedingungen zwischen einigen Minuten und 14 Tagen, die Reaktionstemperatur zwischen etwa 0° und 180°, normalerweise zwischen 0° und 100°, besonders bevorzugt zwischen 15° und 35°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan Nitrile wie Acetonitril; Schwefelkohlenstoff; Carbonsäuren wie Ameisensäure oder Essigsäure; Nitroverbindungen wie Nitromethan oder Nitrobenzol oder Gemische der genannten Lösungsmittel.

Verbindungen der Formel I worin R⁷ eine andere Bedeutung als H aufweist, werden vorzugsweise durch Alkylherung oder Azylierung aus den Verbindungen der Formel I hergestellt, worin R⁷ H bedeutet.

Gewünschtenfalls kann in einer Verbindung der Formel I eine funktionell abgewandelte Amino- und /oder Hydroxygruppe durch Solvolyse oder Hydrogenolyse nach üblichen Methoden in Freiheit gesetzt werden. Dies kann z.B. mit NaOH oder KOH in Wasser, Wasser-THF oder Wasser-Dioxan bei Temperaturen zwischen 0 und 100° erfolgen.

Die Reduktion eines Esters zum Aldehyd oder zum Alkohol, oder die Reduktion eines Nitrils zum Aldehyd oder Amin oder die Reduktion eines Chinolins zum Tetrahydrochinolin erfolgt nach Methoden wie sie dem Fachmann bekannt sind und in Standardwerken der organischen Chemie beschrieben sind. Für die Reduktion von Chinolinen wird bevorzugt Wasserstoff in Gegenwart von Metall-Katalysatoren wie z. B. Ni eingesetzt. Eine Reduktion mit Alkalimetall in Alkoholist ebenfalls möglich.

Insbesondere werden den Verbindungen der Formel I entsprechenden Chinoline hydriert, um die Verbindungen der Formel I zu erhalten.

Die Chinoline und deren Verwendung als Zwischenverbindungen sind ebenfalls Gegenstand der vorliegenden Anmeldung. Bevorzugt sind Chinoline der Formel 1A 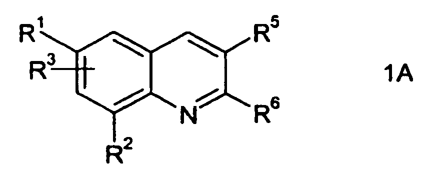 worin R¹, R², R³, R⁵ und R⁶ die oben angegebene Bedeutung aufweisen.

Insbesondere sind die folgenden Chinoline als Zwischenverbindungen bevorzugt:

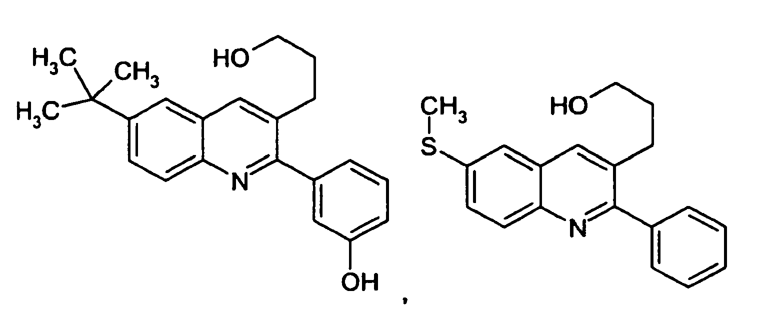

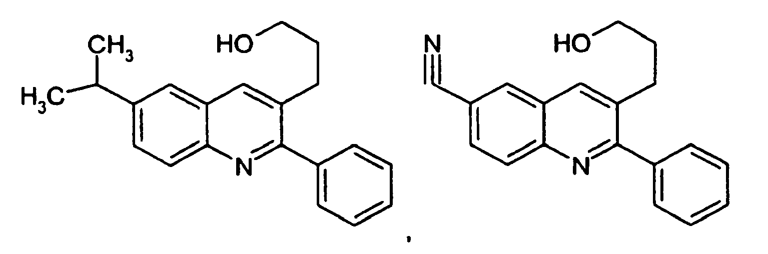

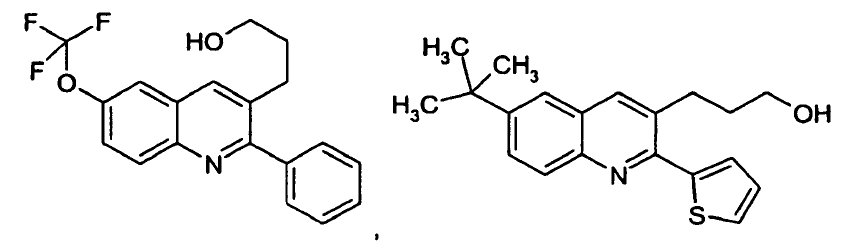

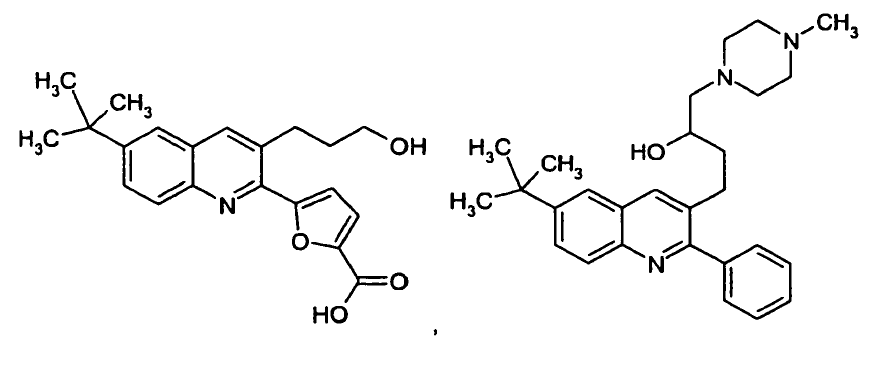

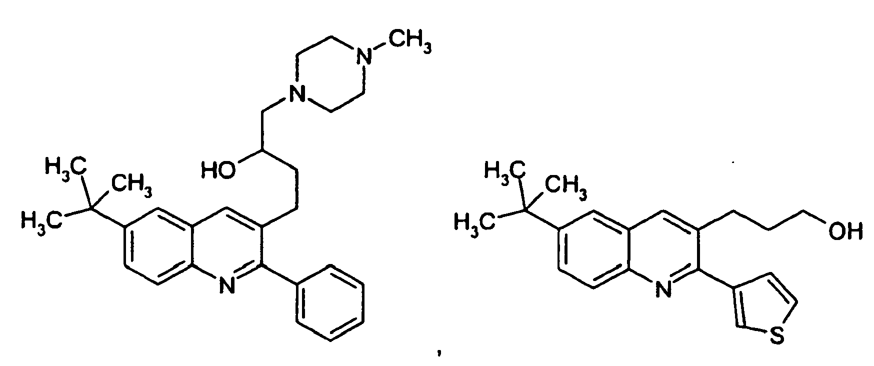

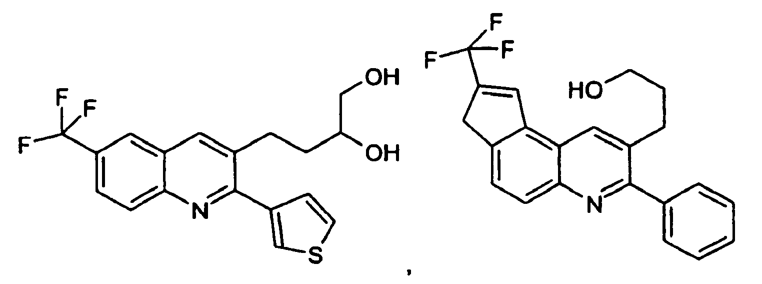

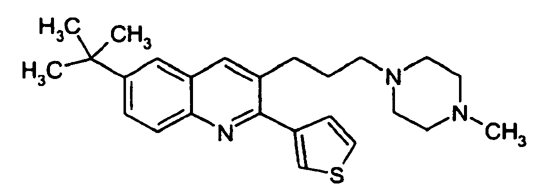

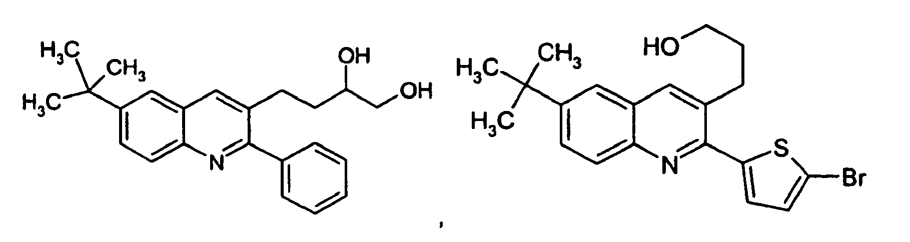

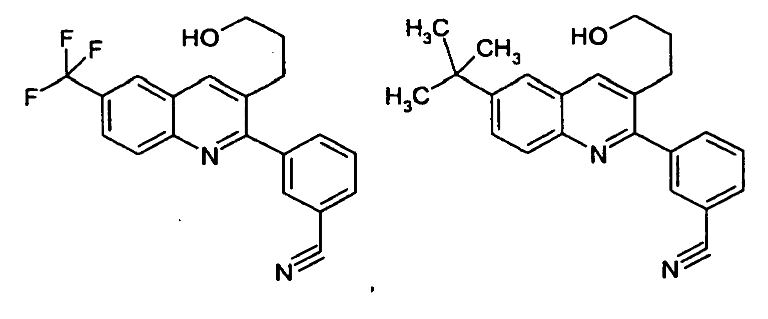

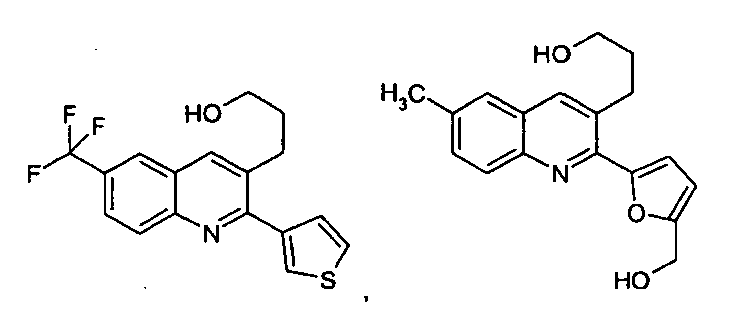

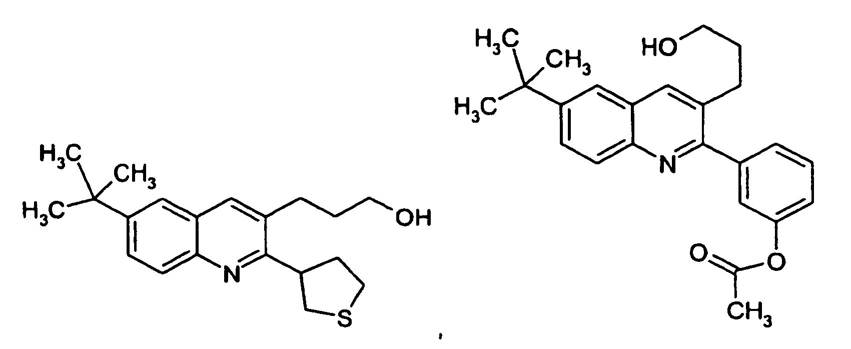

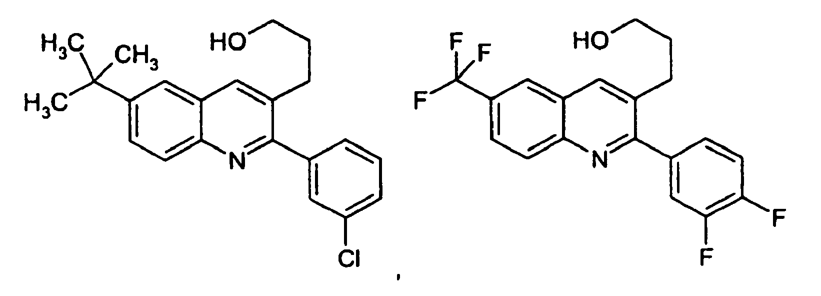

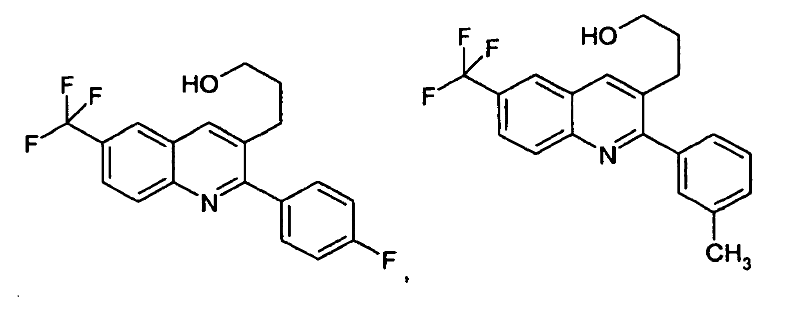

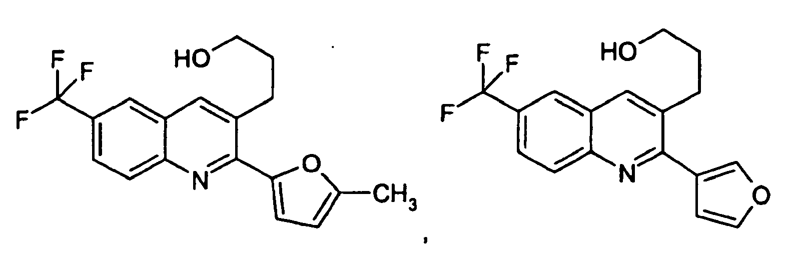

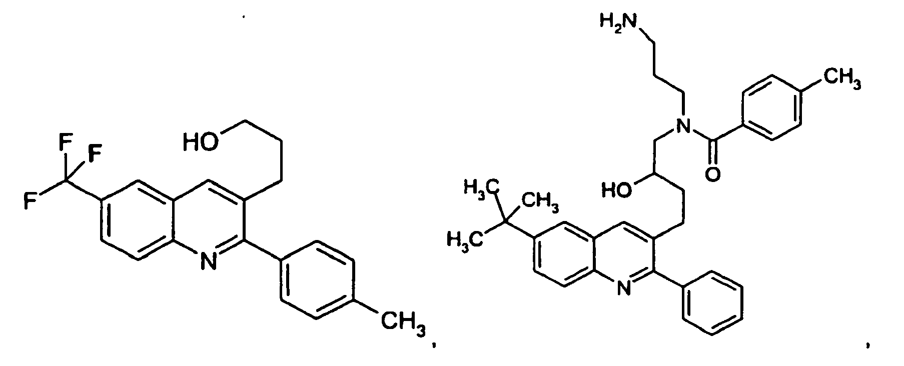

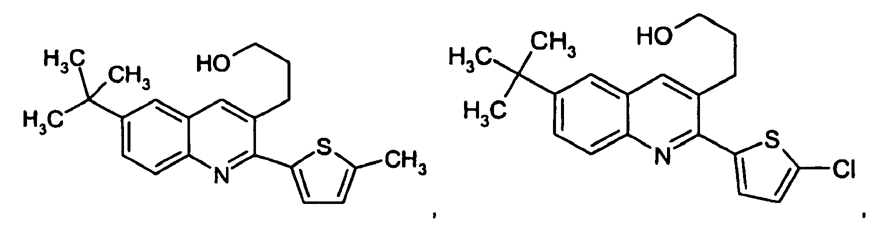

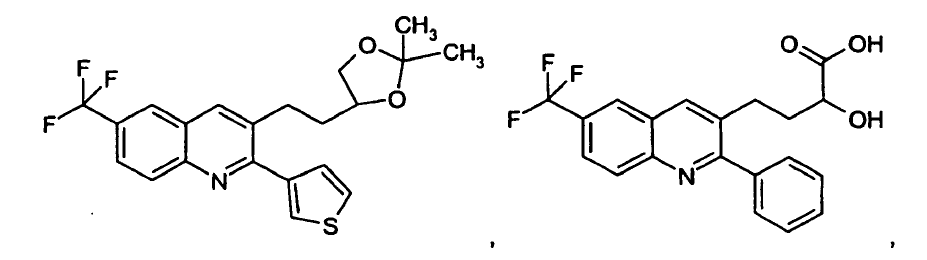

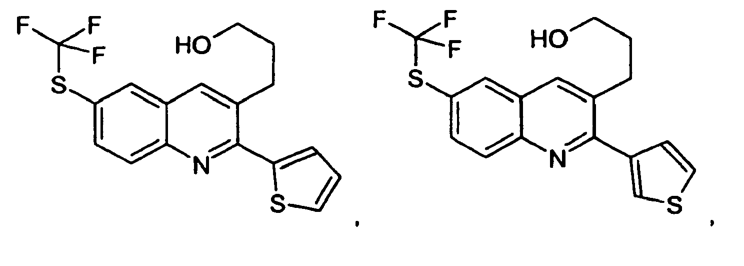

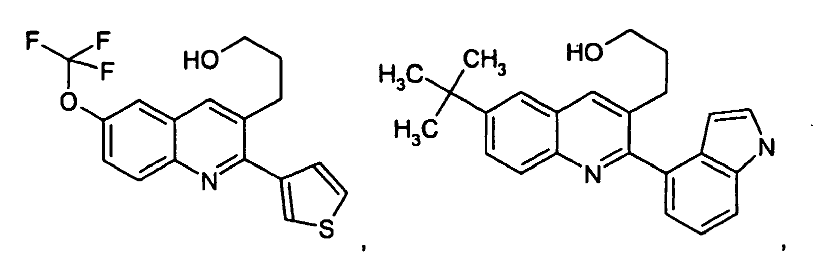

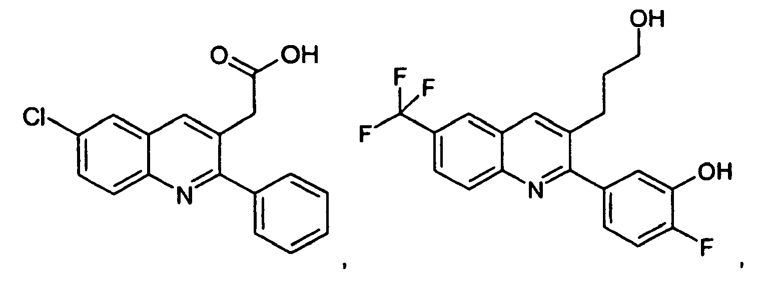

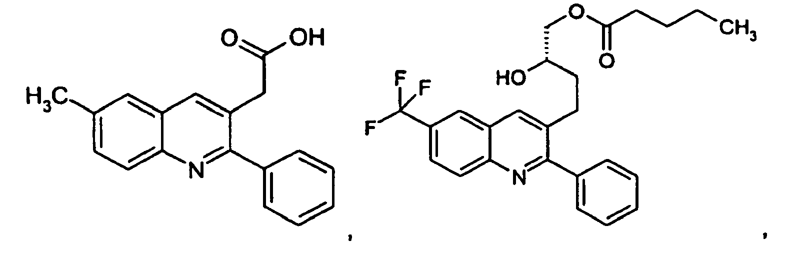

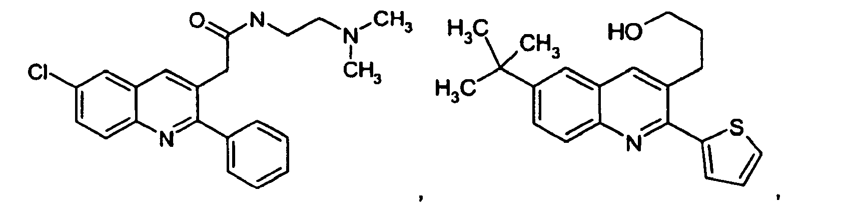

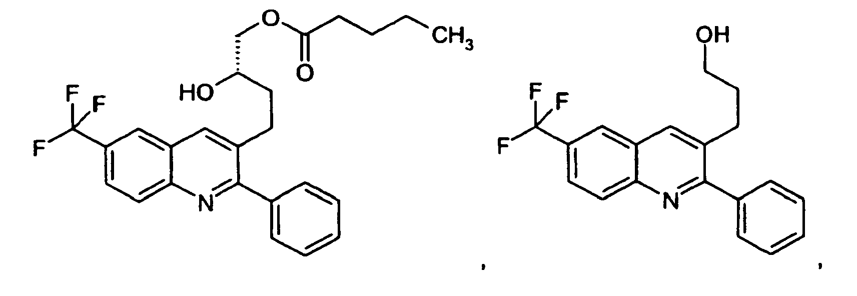

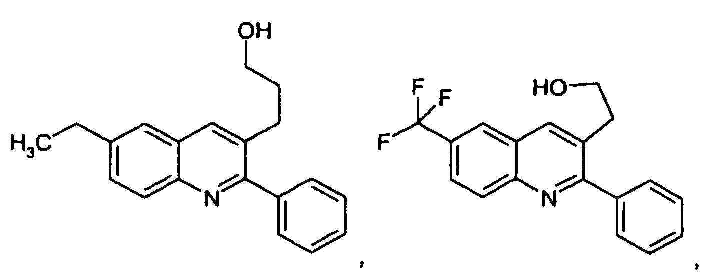

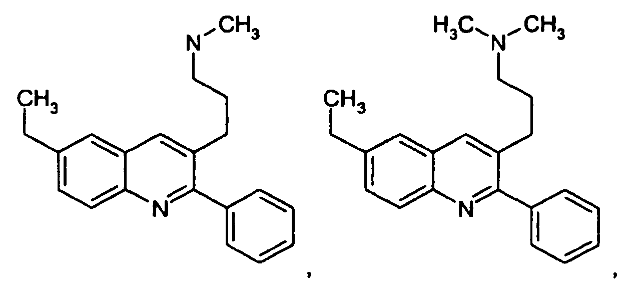

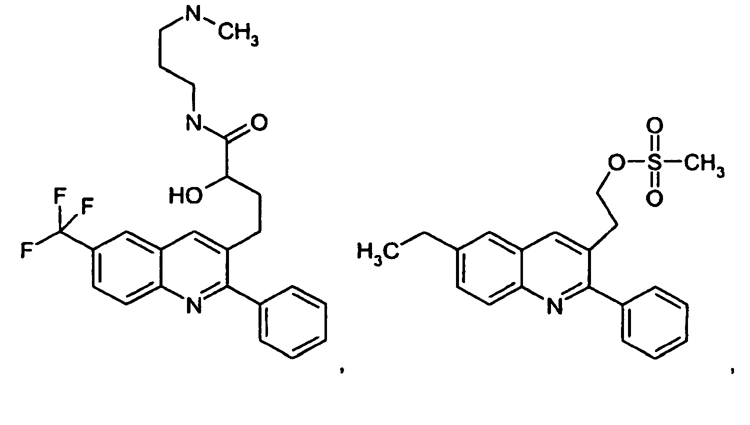

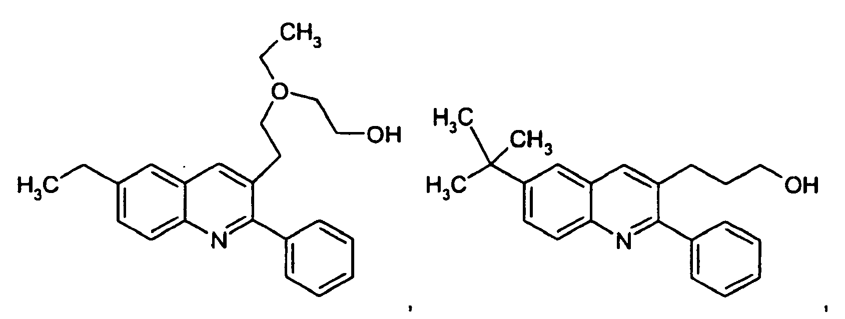

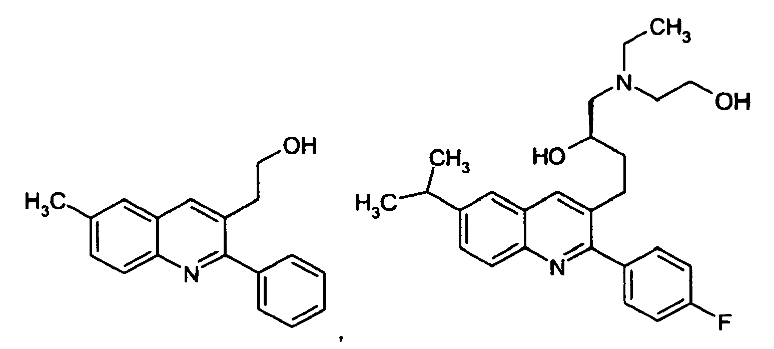

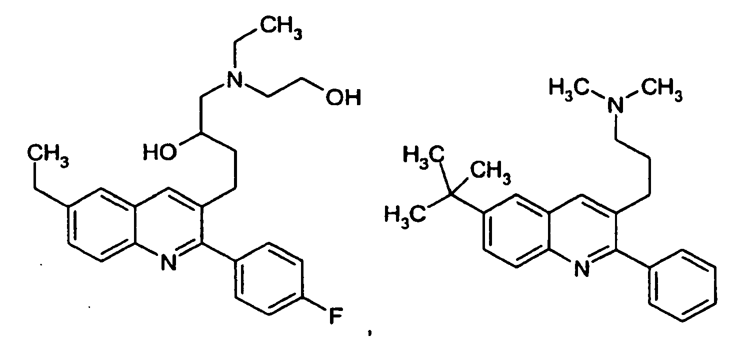

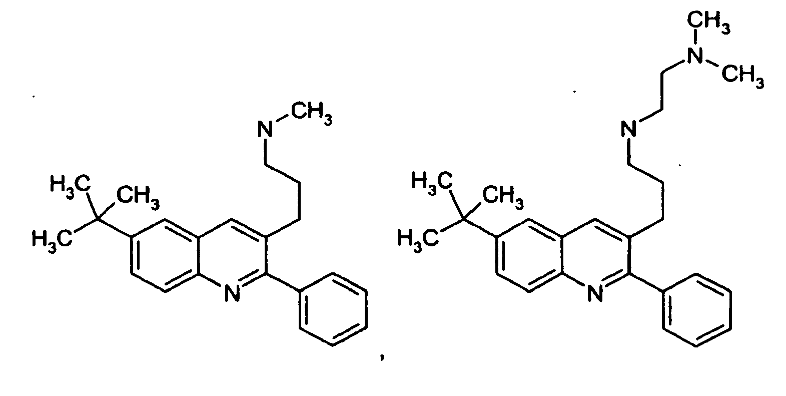

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre Solvate und Stereolsomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nicht-toxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatldylcholinen, gebildet werden.

Die Verbindungen der Formel I sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einlaufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung per se bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre Solvate und Stereoisomere, einschließlich deren Mischungen in allen Verhältnissen, und mindestens einen weiteren Arzneimittelwirkstoff.

Bevorzugt aber nicht ausschliesslich werden die Arzneimittel der Tabelle 1 mit den Verbindungen der Formel I kombiniert. Eine Kombination der Formel I und Arzneimitteln der Tabelle 1 kann auch mit Verbindungen der Formel V kombiniert werden.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | |
| | Ormiplatin | BBR-3464 (Hoffmann-La Roche) |
| | Iproplatin | |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | |
| | | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour-Ipsen) |
| | Irinotecan (CPT-11) | |
| | 7-Ethyl-10-hydroxycamptothecin | TAS-103 (Taiho) |
| | Topotecan | Elsamitrucin (Spectrum) |
| | Dexrazoxanet | J-107088 (Merck & Co) |
| | (TopoTarget) | BNP-1350 (BioNumerik) |
| | Pixantron (Novuspharrna) | CKD-602 (Chong Kun Dang) |
| | Rebeccamycin-Analogon | |
| | (Exelixis) | KW-2170 (Kyowa Hakko) |
| | BBR-3576 (Novuspharrna) | |
| | | |
| Antitumor-Antibiotika | Dactinomycin (Actinomycin | Amonafid |
| | D) | Azonafid |
| | Doxorubicin (Adriamycin) | Anthrapyrazol |
| | Deoxyrubicin | Oxantrazol |
| | Valrubicin | Losoxantron |
| | Daunorubicin | Bleomycinsulfat |
| | (Daunomycin) | (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem |
| | Cyanomorpholinodoxorubi cin | Pharmaceuticals) |
| | Mitoxantron (Novantron) | |
| | | |
| Antimitotische Mittel | Paclitaxel | SB 408075 |
| | Docetaxel | (GlaxoSmithKline) |
| | Colchicin | E7010 (Abbott) |
| | Vinblastin | PG-TXL (Cell |
| | Vincristin | Therapeutics) |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Wamer- | D 24851 (ASTA Medica) |
| | Lambert) | ER-86526 (Eisai) |
| | Cemadotin (BASF) | Combretastatin A4 (BMS) |
| | RPR 109881A (Aventis) | Isohomohalichondrin-B |
| | TXD 258 (Aventis) | (PharmaMar) |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | !DN-5109 (Indena) |
| | Vinflunin (Fabre) | AVLB (Prescient NeuroPharma) |
| | Auristatin PE (Teikoku Hormone) | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase-Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | Apaziquon (Spectrum Pharmaceuticals) |
| | Albumin + 32P (Isotope Solutions) | O6-Benzylguanin |
| | Thymectacin (NewBiotics) | (Paligent) |
| | Edotreotid (Novartis) | |
| | | |
| Farnesyltransfera se-Inhibitoren | Arglabin (NuOncology Labs) | Tipifamib (Johnson & Johnson) |
| | Ionafarnib (Schering-Plough) | Perillylalkohol (DOR BioPharma) |
| | BAY-43-9006 (Bayer) | |
| | | |
| Pumpen-Inhibitoren | CBT-1 (CBA Pharma) | Zosuquidar-Trihydrochlorid |
| | Tariquidar (Xenova) | (Eli Lilly) |
| | MS-209 (Schering AG) | Biricodar-Dicitrat (Vertex) |
| | | |
| Histonacetyltransferase-Inhibitoren | Tacedinalin (Pfizer) | Pivaloyloxymethylbutyrat |
| | SAHA (Aton Pharma) | (Titan) |
| | MS-275 (Schering AG) | Depsipeptid (Fujisawa) |
| | | |
| Metalloproteinase-Inhibitoren Ribonucleosidreduktase-Inhibitoren | Neovastat (Aeterna | CMT -3 (CollaGenex) |
| | Laboratories) | BMS-275291 (Celltech) |
| | Marimastat (British | Tezacitabin (Aventis) |
| | Biotech) | Didox (Molecules for Health) |
| | Galliummaltolat (Titan) | |
| | Triapin (Vion) | |
| | | |
| TNF-alpha-Agonisten/Antago nisten | Virulizin (Lorus Therapeutics) | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor-Agonisten | Fenretinid (Johnson & Johnson) | Alitretinoin (Ligand) |
| | LGD-1550 (Ligand) | |
| | | |
| Immunmodulatoren | Interferon | Dexosom-Therapie |
| | Oncophage (Antigenics) | (Anosys) |
| | GMK (Progenics) | Pentrix (Australian Cancer Technology) |
| | Adenokarzinom-Impfstoff | JSF-154 (Tragen) |
| | (Biomira) | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe | !3-Alethin (Dovetail) |
| | (CTL Immuno) | CLL-Thera (Vasogen) |
| | Melanom-Impfstoff (CTL Immuno) | |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol |
| | Toremofin | (EntreMed) |
| | Dexamethason | Arzoxifen (Eli Lilly) |
| | | |
| Photodynamische Mittel | Talaporfin (Light Sciences) | Pd-Bacteriopheophorbid |
| | Theralux | (Yeda) |
| | (Theratechnologies) | Lutetium-Texaphyrin |
| | Motexafin-Gadolinium | (Pharmacyclics) |
| | (Pharmacyclics) | Hypericin |
| | | |
| Tyrosinkinase-Inhibitoren | Imatinib (Novartis) | Kahalid F (PharmaMar) |
| | Leflunomid | CEP- 701 (Cephalon) |
| | (Sugen/Pharmacia) | CEP-751 (Cephalon) |
| | ZDI839 (AstraZeneca) | MLN518 (Millenium) |
| | Erlotinib (Oncogene Science) | PKC412 (Novartis) |
| | Canertjnib (Pfizer) | Phenoxodiol O |
| | Squalamin (Genaera) | Trastuzumab (Genentech) |
| | SU5416 (Pharmacia) | C225 (ImClone) |
| | SU6668 (Pharmacia) | rhu-Mab (Genentech) |
| | ZD4190 (AstraZeneca) | MDX-H210 (Medarex) |
| | ZD6474 (AstraZeneca) | 2C4 (Genentech) |
| | Vatalanib (Novartis) | MDX-447 (Medarex) |
| | PKI166 (Novartis) | ABX-EGF (Abgenix) |
| | GW2016 | IMC-1C11 (ImClone) |
| | (GlaxoSmithKline) | |
| | EKB-509 (Wyeth) | |
| | EKB-569 (Wyeth) | |
| | | |
| Verschiedene Mittel | SR-27897 (CCK-A-Inhibitor, Sanofi-Synthelabo) | BCX-1777 (PNP-Inhibitor, BioCryst) |
| | Tocladesin (cyclisches-AMP-Agonist, Ribapharm) | Ranpimase |
| | Alvocidib (CDK-Inhibitor, Aventis) | (Ribonuclease-Stimulans, Alfacell) |
| | CV-247 (COX-2-Inhibitor, Ivy Medical) | Galarubicin (RNA-Synthese-Inhibitor, Dong-A) |
| | P54 (COX-2-Inhibitor, Phytopharm) | Tirapazamin |
| | CapCell™ (CYP450-Stimulans, Bavarian Nordic) | (Reduktionsmittel, SRI International) |
| | GCS-IOO (gal3-Antagonist, GlycoGenesys) | N-Acetylcystein |
| | G17DT-Immunogen | (Reduktionsmittel, Zambon) |
| | (Gastrin-Inhibitor, Aphton) | R-Flurbiprofen (NF-kappaB-Inhibitor, Encore) |
| | Efaproxiral (Oxygenator, Allos Therapeutics) | 3CPA (NF-kappaB-Inhibitor, Active Biotech) |
| | PI-88 (Heparanase-Inhibitor, Progen) | Seocalcitol (Vitamin-D-Rezeptor-Agonist, Leo) |
| | Tesmilifen (Histamin-Antagonist, YM BioSciences) | 131-1-TM-601 (DNA-Antagonist, TransMolecular) |
| | Histamin (Histamin-H2-Rezeptor- Agonist, Maxim) | Eflomithin (ODC-Inhibitor, ILEX Oncology) |
| | Tiazofurin (IMPDH-Inhibitor, Ribapharm) | Minodronsäure |
| | Cilengitid (Integrin-Antagonist, Merck KGaA) | (Osteoclasten-Inhibitor, Yamanouchi) |
| | SR-31747 (IL-1-Antagonist, Sanofi-Synthelabo) | Indisulam (p53-Stimulans, Eisai) |
| | CCI-779 (mTOR-Kinase-Inhibitor, Wyeth) | Aplidin (PPT-Inhibitor, PharmaMar) |
| | Exisulind (PDE-V-Inhibitor, Cell Pathways) | Rituximab (CD20-Antikörper, Genentech) |
| | CP-461 (PDE-V-Inhibitor, Cell Pathways) | Gemtuzumab (CD33-Antikörper, Wyeth Ayerst) |
| | AG-2037 (GART-Inhibitor, Pfizer) | PG2 (Hämatopoese-Verstärker, Pharmagenesis) |
| | WX-UK1 (Plasminogenaktivator-Inhibitor, Wilex) | Immunol™ (Triclosan-Oralspülung, Endo) |
| | PBI-1402 (PMN-Stimulans, ProMetic LifeSciences) | Triacetyluridin (Uridin-Prodrug, Wellstat) |
| | Bortezomib (Proteasom-Inhibitor, Millennium) | SN-4071 (Sarkom-Mittel, Signature BioScience) |
| | SRL-172 (T-Zell-Stimulans, SR Pharma) | TransMID-107™ |
| | TLK-286 (Glutathion-S-Transferase-Inhibitor, Telik) | (Immunotoxin, KS Biomedix) |
| | PT-100 (Wachstumsfaktor-Agonist, Point Therapeutics) | PCK-3145 (Apoptose-Förderer, Procyon) |
| | Midostaurin (PKC-Inhibitor, Novartis) | Doranidazol (Apoptose-Förderer, Pola) |
| | Bryostatin-1 (PKC-Stimulans, GPC Biotech) | CHS-828 (cytotoxisches Mittel, Leo) |
| | CDA-II (Apoptose-Förderer, Everlife) | trans-Retinsäure |
| | SDX-101 (Apoptose-Förderer, Salmedix) | (Differentiator, NIH) |
| | Ceflatonin (Apoptose-Förderer, ChemGenex) | MX6 (Apoptose-Förderer, MAXIA) |
| | | Apomin (Apoptose-Förderer, ILEX Oncology) |
| | | Urocidin (Apoptose-Förderer, Bioniche) |
| | | Ro-31-7453 (Apoptose-Förderer, La Roche) |
| | | Brostallicin (Apoptose-Förderer, Pharmacia) |

Bevorzugt werden die Verbindungen der Formel I mit den mit bekannten Antikrebsmitteln kombiniert:
Zu diesen bekannten Antikrebsmitteln zählen die folgenden:
   Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-ProteaseHemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).
   "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
   "Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
   "Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylomithin, ILX23-7553, trans-N-(4'-Hydroxyphenyl)retinamid und N-4-Carboxyphenylretinamid.
   "Zytotoxika" bezieht sich auf Verbindungen, die in erster Linie durch direkte Einwirkung auf die Zellfunktion zum Zelltod führen oder die die Zellteilung hemmen oder diese stören, darunter Alkylierungsmittel, Tumornekrosefaktoren, interkaliemde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNP11100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-hepto-pyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo-(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

Insbesondere bevorzugt ist die Verwendung der erfindungsgemäßen Verbindung zur Behandlung und Prophylaxe von Tumorerkrankungen.

Der Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge.

Der Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die Erfindung umfasst auch ein Verfahren zur Behandlung eines Patienten, der ein Neoplasma, wie einen Krebs, hat, durch Verabreichung
a) einer oder mehrerer der Verbindungen der Formel I:
b) und einer oder mehrerer der Verbindungen der Formel V oder deren Säure-Additionssalze, insbesondere Hydrochloride: 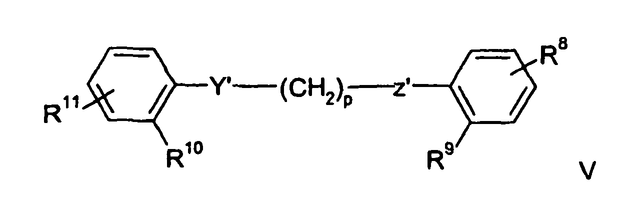 worin Y' und Z' jeweils unabhängig voneinander O oder N bedeuten, R⁶ und R⁷ jeweils unabhängig voneinander H, OH, Halogen, OC1-10-Alkyl, OCF₃, NO₂ oder NH₂ bedeuten, n eine ganze Zahl zwischen 2 und 6, jeweils einschließlich, bedeutet und R⁸ und R⁹ jeweils unabhängig voneinander vorzugsweise an der meta- oder para-Position stehen und aus der Gruppe: 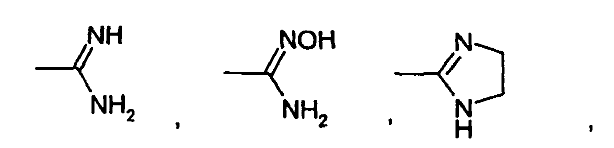 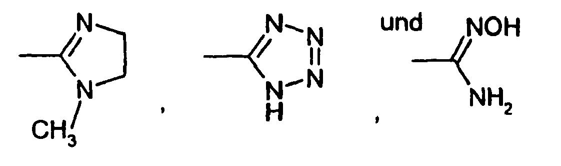
ausgewählt sind, wobei die erste und die zweite Verbindung gleichzeitig oder innerhalb von 14 Tagen voneinander in Mengen verabreicht werden, die ausreichen, um das Wachstum des Neoplasmas zu hemmen.

Die Kombination der Verbindungen der Formel I mit den Verbindungen der Formels V und anderer Pentamidin-Analoga führt zu einer synergistischen Wirkung bei der Hemmung von Neoplasien. Kombinationen enthaltend die Verbindungen der Formel V sind z.B. in WO 02058684 erwähnt.

Der Wirkungsmechanismus von Pentamidin oder seiner Derivate ist derzeit nicht eindeutig geklärt: Pentamidin oder seine Derivate hat offenbar pleiotrope Wirkungen, da es zu einer Abnahme von DNA-, RNA- und Protein-Synthese führt. Vor kurzem wurde beschrieben, dass Pentamidin ein leistungsfähiger Hemmstoff von PRL1-, -2- und 3-Phosphatasen (Pathak et al., 2002) und Tyrosinphosphatasen ist, und ihre Überexpression geht mit neoplastischen bösartigen Tumoren beim Menschen einher. Andererseits wurde beschrieben, dass Pentamidin ein Arzneimittel ist, das an die kleine DNA-Furche bindet (Puckowska et al., 2004) und das seine Wirkung über die Störung der Genexpression und/oder DNA-Synthese ausüben kann.

Die beigefügten Experimente zeigen, dass:
- sowohl Pentamidin als auch die Verbindungen der Formel I Zellen im G2/M-Zellzyklus aufhalten.
- die Kombination von Pentamidin und Verbindungen der Formel I additive bis synergistische Wirkungen auf die Zellproliferation haben.

Andere geeignete Pentamidin-Analoga umfassen Stilbamidin (G-1) und Hydroxystilbamidin (G-2) und ihre Indolanaloga (z.B. G-3): 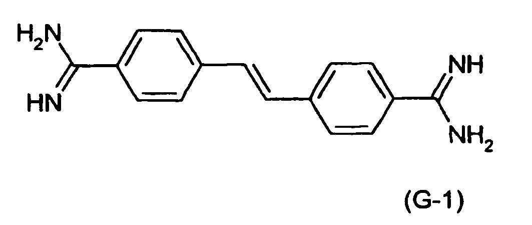 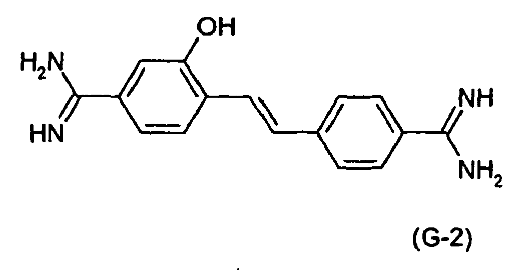 und

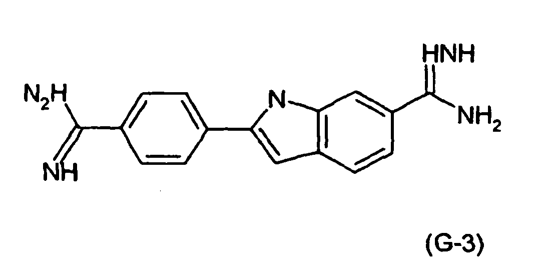

Jede Amidineinheit kann unabhängig voneinander durch eine der Einheiten ersetzt werden, die vorstehend für R⁸ und R¹¹ definiert sind. Wie im Fall der Benzimidazole und Pentamidine, eignen sich auch Salze von Stilbamidin, Hydroxystilbamidin und ihren Indolderivaten für das erfindungsgemäße Verfahren. Bevorzugte Salze umfassen zum Beispiel Dihydrochlorid- und Methansulfonatsalze.

Noch andere Analoga sind diejenigen, die unter eine Formel fallen, die in einem der U.S.-Patente Nr. 5,428,051, 5,521,189, 5,602,172, 5,643,935, 5,723,495, 5,843,980, 6,172,104 und 6,326,395 oder der U.S.-Patentanmeldung mit der Veröffentlichungsnr. US 2002/0019437 A1 bereitgestellt werden, die jeweils in ihrer Gesamtheit durch Bezugname aufgenommen sind. Beispielhafte Analoga umfassen 1,5-Bis-(4'-(N-hydroxyamidino)phenoxy)pentan, 1,3-Bis-(4'-(N-hydroxyamidino)-phenoxy)propan, 1,3-Bis-(2'-methoxy-4'-(N-hydroxyamidino)-phenoxy)-propan, 1,4-Bis-(4'-(N-hydroxyamidino)phenoxy)butan, 1,5-Bis-(4'-(N-hydroxyamidino)phenoxy)pentan, 1,4-Bis-(4'-(N-hydroxyamidino)-phenoxy)butan, 1,3-Bis-(4'-(4-hydroxyamidino)phenoxy)propan, 1,3-Bis-(2'-methoxy-4'-(N-hydroxyamidino)phenoxy)propan, 2,5-Bis-[4-amidinophenyl]furan, 2,5-Bis-[4-amidinophenyl]furan-bis-amidoxim, 2,5-Bis-[4-amidinophenyl]furan-bis-O-methylamidoxim, 2,5-Bis-[4-amidinophenyl]furan-bis-O-ethylarnidoxim, 2,8-Diamidinodibenzothiophen, 2,8-Bis-(N-isopropylamidino)carbazol, 2,8-Bis-(N-hydroxyamidino)carbazol, 2,8-Bis-(2-imidazolinyl)dibenzothiophen, 2,8-Bis-(2-imidazolinyl)-5,5-dioxodibenzothiophen, 3,7-Diamidinodibenzothiophen, 3,7-Bis-(N-isopropylamidino)dibenzothiophen, 3,7-Bis-(N-hydroxyamidino)-dibenzothiophen, 3,7-Diaminodibenzothiophen, 3,7-Dibromdibenzothiophen, 3,7-Dicyanodibenzothiophen, 2,8-Diamidinodibenzofuran, 2,8-Di-(2-imidazolinyl)dibenzofuran, 2,8-Di-(N-isopropylamidino)dibenzofuran, 2,8-Di-(N-hydroxylamidino)dibenzofuran, 3,7-Di-(2-imidazolinyl)dibenzofuran, 3,7-Di-(isopropylamidino)dibenzofuran, 3,7-Di-(A-hydroxylamidino)dibenzofuran, 2,8-Dicyanodibenzofuran, 4,4'-Dibrom-2,2'-dinitrobiphenyl, 2-Methoxy-2'-nitro-4,4'-dibrombiphenyl, 2-Methoxy-2'-amino-4,4'-dibrombiphenyl, 3,7-Dibromdibenzofuran, 3,7-Dicyanodibenzofuran, 2,5-Bis-(5-amidino-2-benzimidazolyl)pyrrol, 2,5-Bis-[5-(2-imidazolinyl)-2-benzimidazolyl]pyrrol, 2,6-Bis-[5-(2-imidazolinyl)-2-benzimidazolyl]pyridin, 1-Methyl-2,5-bis-(5-amidino-2-benzimidazolyl)pyrrol, 1-Methyl-2,5-bis-[5-(2-imidazolyl)-2-benzimidazolyl]pyrrol, 1-Methyl-2,5-bis-[5-(1,4,5,6-tetrahydro-2-pyrimidinyl)-2-benzimidazolyl]pyrrol, 2,6-Bis-(5-amidino-2-benzimidazoyl)pyridin, 2,6-Bis-[5-(1,4,5,6-tetrahydro-2-pyrimidinyl)-2-benzimidazolyl]pyridin, 2,5-Bis-(5-amidino-2-benzimidazolyl)furan, 2,5-Bis-[5-(2-imidazolinyl)-2-benzimidazolyl]furan, 2,5-Bis-(5-N-isopropylamidino-2-benzimidazolyl)furan, 2,5-Bis-(4-guanylphenyl)furan, 2,5-Bis(4-guanylphenyl)-3,4-dimethylfuran, 2,5-Di-p-[2-(3,4,5,6-tetrahydropyrimidyl)phenyllfuran, 2,5-Bis-[4-(2-imidazolinyl)phenyl]-furan, 2,5-[Bis-{4-(2-tetrahydropyrimidinyl)}phenyl]-p-(tolyloxy)furan, 2,5-[Bis-{4-(2-imidazolinyl)}-phenyl]-3-p-(tolyloxy)furan, 2,5-Bis-{4-[5-(N-2-aminoethylamido)benzimidazol-2-yl]phenyl}furan, 2,5-Bis-[4-(3a,4,5,6,7,7a-hexahydro-1H-benzimidazol-2-yl)phenyl]furan, 2,5-Bis-[4-(4,5,6,7-tetrahydro-1H-1,3-diazepin-2-yl)phenyl]furan, 2,5-Bis-(4-N,N-dimethylcarboxhydrazidphenyl)furan, 2,5-Bis-{4-[2-(N-2-hydroxyethyl)imidazolinyl]phenyl}furan, 2,5-Bis-[4-(N-isopropyl-amidino)phenyl]furan, 2,5-Bis-{4-[3-(dimethylaminopropyl)amidino]phenyl}-furan, 2,5-Bis-{4-[N-(3-aminopropyl)amidino]phenyl}furan, 2,5-Bis-[2-(imidzaolinyl)phenyl]-3,4-bis-(methoxymethyl)furan, 2,5-Bis-[4-N-(dimethylaminoethyl)guanyl]-phenylfuran, 2,5-Bis-{4-[(N-2-hydroxyethyl)guanyl]-phenyl}furan, 2,5-Bis-[4-N-(cyclopropylguanyl)phenyl]furan, 2,5-Bis-[4-(N,N-diethylaminopropyl)-guanyl]phenylfuran, 2,5-Bis-{4-[2-(N-ethylimidazolinyl)]-phenyl}furan, 2,5-Bis-{4-[N-(3-pentylguanyl)]}phenylfuran, 2,5-Bis-[4-(2-imidazolinyl)phenyl]-3-methoxyfuran, 2,5-Bis-[4-(N-isopropylamidino)phenyl]-3-methylfuran, Bis-[5-amidino-2-benzimidazolyl]methan, Bis-[5-(2-imidazolyl)-2-benzimidazolyl]methan, 1,2-Bis-[5-amidino-2-benzimidazolyl]ethan, 1,2-Bis-[5-(2-imidazolyl)-2-benzimidazolyl]ethan, 1,3-Bis-[5-amidino-2-benzimidazolyl]propan, 1,3-Bis-[5-(2-imidazolyl)-2-benzimidazolyl]propan, 1,4-Bis-[5-amidino-2-benzimidazolyl]propan, 1,4-Bis-[5-(2-imidazolyl)-2-benzimidazolyl]butan, 1,8-Bis-[5-amidino-2-benzimidazolyl]octan, trans-1,2-Bis-[5-amidino-2-benzimidazolyl]-ethen, 1,4-Bis-[5-(2-imidazolyl)-2-benzimidazolyl]-1-buten, 1,4-Bis-[5-(2-imidazolyl)-2-benzimidazolyl]-2-buten, 1,4-Bis-[5-(2-imidazolyl)-2-benzimidazolyl]-1-methylbutan, 1,4-Bis-[5-(2-imidazolyl)-2-benzimidazolyl]-2-ethylbutan, 1,4-Bis-[5-(2-imidazolyl)-2-benzimidazolyl]-1-methyl-1-buten, 1,4-Bis-[5-(2-imidazolyl)-2-benzimidazolyl]-2,3-diethyl-2-buten, 1,4-Bis-[5-(2-imidazolyl)-2-benzimidazolyl]-1,3-butadien, 1,4-Bis-[5-(2-imidazolyl)-2-benzimidazolyl]-2-methyl-1,3-butadien, Bis-[5-(2-pyrimidyl)-2-benzimidazolyl]methan, 1,2-Bis-[5-(2-pyrimidyl)-2-benzimidazolyl]ethan, 1,3-Bis-[5-amidino-2-benzimidazolyl]propan, 1,3-Bis-[5-(2-pyrimidyl)-2-benzimidazolyl]propan, 1,4-Bis-[5-(2-pyrimidyl)-2-benzimidazolyl]butan, 1,4-Bis-[5-(2-pyrimidyl)-2-benzimidazolyl]-1-buten, 1,4-Bis-[5-(2-pyrimidyl)-2-benzimidazolyl]-2-buten, 1,4-Bis-[5-(2-pyrimidyl)-2-benzimidazolyl]-1-methylbutan, 1,4-Bis-[5-(2-pyrimidyl)-2-benzimidazolyl]-2-ethylbutan, 1,4-Bis-[5-(2-pyrimidyl)-2-benzimidazolyl]-1-methyl-1-buten, 1,4-Bis-[5-(2-pyrimidyl)-2-benzimidazolyl]-2,3-diethyl-2-buten, 1,4-Bis-[5-(2-pyrimidyl)-2-benzimidazolyl]-1,3-butadien und 1,4-Bis-[5-(2-pyrimidyl)-2-benzimidazolyl]-2-methyl-1,3-butadien, 2,4-Bis-(4-guanylphenyl)pyrimidin, 2,4-Bis-(4-imidazolin-2-yl)pyrimidin, 2,4-Bis-[(tetrahydropyrimidinyl-2-yl)phenyl]pyrimidin, 2-(4-[N-i-Propylguanyl]phenyl)-4-(2-methoxy-4-[N-i-propylguanyl]phenyl)pyrimidin, 4-(N-Cyclopentylamidino)-1,2-phenylendiamin, 2,5-Bis-[2-(5-amidino)benzimidazoyl]furan, 2,5-Bis-[2-{5-(2-imidazolino)}benzimidazoyl]furan, 2,5-Bis-[2-(5-N-isopropylamidino)-benzimidazoyl]furan, 2,5-Bis-[2-(5-N-cyclopentylamidino)-benzimidazoyl]furan, 2,5-Bis[2-(5-amidino)benzimidazoyl]pyrrol, 2,5-Bis-[2-{5-(2-imidazolino)}benzimidazoyl]pyrrol, 2,5-Bis-[2-(5-N-isopropylamidino)-benzimidazoyl]pyrrol, 2,5-Bis-[2-(5-N-cyclopentylamidino)-benzimidazoyl]pyrrol, 1-Methyl-2,5-bis-[2-(5-amidino)benzimidazoyl]pyrrol, 2,5-Bis-[2-{5-(2-imidazolino)}benzimidazoyl]-1-methylpyrrol, 2,5-Bis-[2-(5-N-cyclopentylamidino)benzimidazoyl]-1-methylpyrrol, 2,5-Bis-[2-(5-N-isopropylamidino)benzimidazoyl]thiophen, 2,6-Bis-[2-{5-(2-imidazolino)}benzimidazoyl]pyridin, 2,6-Bis-[2-(5-amidino)benzimidazoyl]-pyridin, 4,4'-Bis-[2-(5-N-isopropylamidino)benzimidazoyl]-1,2-diphenylethan, 4,4'-Bis-[2-(5-N-cyclopentylamidino)benzimidazoyl]-2,5-diphenylfuran, 2,5-Bis-[2-(5-amidino)benzimidazoyl]benzo-[b]-furan, 2,5-Bis-[2-(5-N-cyclopentylamidino)benzimidazoyl]benzo-[b]-furan, 2,7-Bis-[2-(5-N-isopropylamidino)benzimidazoyl]fluor, 2,5-Bis-[4-(3-(N-morpholinopropyl)-carbamoyl)phenyl]furan, 2,5-Bis-[4-(2-N,N-dimethylaminoethylcarbamoyl)phenyl]furan, 2,5-Bis-[4-(3-N,N-dimethylaminopropylcarbamoyl)phenyl]furan, 2,5-Bis-[4-(3-N-methyl-3-N-phenylaminopropylcarbarmoyl)phenyl]furan, 2,5-Bis-[4-(3-N,N8,N11-trimethylaminopropylcarbamoyl)phenyl]furan, 2,5-Bis-[3-amidinophenyl]furan, 2,5-Bis-[3-(N-isopropylamidino)amidinophenyl]furan, 2,5-Bis-[3-[(N-(2-dimethylaminoethyl)amidino]phenylfuran, 2,5-Bis-[4-(N-2,2,2-trichlorethoxycarbonyl)amidinophenyl]furan, 2,5-Bis-[4-(N-thioethylcarbonyl)amidinophenyl]furan, 2,5-Bis-[4-(N-benzyloxycarbonyl)amidinophenyl]furan, 2,5-Bis[4-(N-phenoxycarbonyl)-amidinophenyl]furan, 2,5-Bis-[4-(N-(4-fluor)-phenoxycarbonyl)amidinophenyl]furan, 2,5-Bis-[4-(N-(4-methoxy)phenoxycarbonyl)amidinophenyl]furan, 2,5-Bis-[4-(1-acetoxyethoxycarbonyl)amidinophenyl]furan und 2,5-Bis-[4-(N-(3-fluor)phenoxycarbonyl)amidinophenyl]furan. Verfahren zur Herstellung einer der vorstehenden Verbindungen sind in den U.S.-Patenten Nr. 5,428,051, 5,521,189, 5,602,172, 5,643,935, 5,723,495, 5,843,980, 6,172,104 und 6,326,395 oder der US-Patentanmeldung mit der Veröffentlichungsnr. US 2002/0019437 A1 beschrieben.

Pentamidin-Metabolite eignen sich ebenfalls in der erfindungsgemäßen antiproliferativen Kombination. Pentamidin wird im Körper schnell zu mindestens sieben primären Metaboliten metabolisiert. Einige dieser Metabolite haben eine oder mehrere Wirkungen mit Pentamidin gemeinsam. Pentamidin-Metabolite weisen eine antiproliferative Wirkung auf, wenn sie mit einem Benzimidazol oder einem Analogon davon kombiniert werden.

Sieben Pentamidin-Analoga sind nachstehend gezeigt.

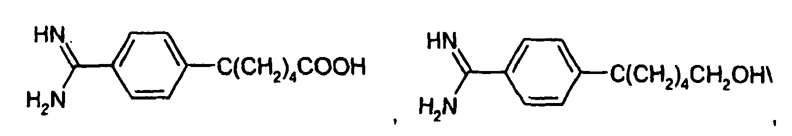

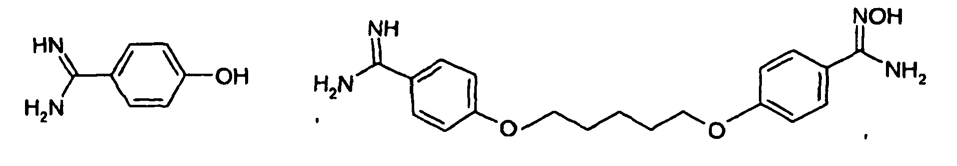

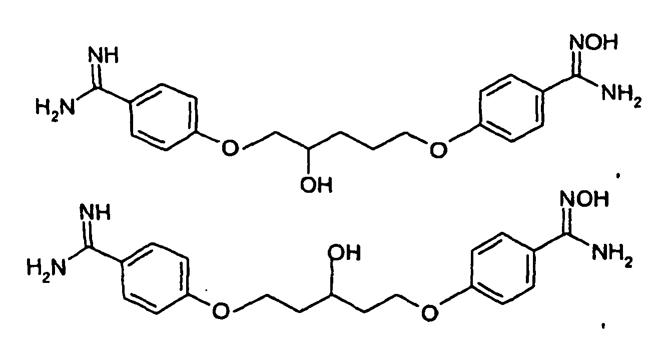

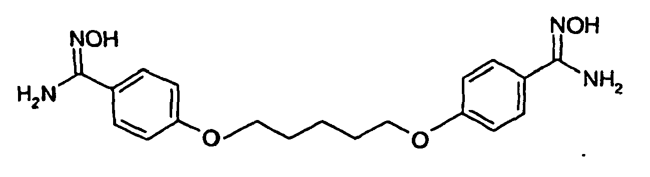

Die erfindungsgemäßen Kombinationen von Verbindungen der Formel I und Formel V oder deren Analoga und seiner Metaboliten eignen sich zur Behandlung von Neoplasmen. Eine Kombinationstherapie kann allein oder in Verbindung mit einer anderen Therapie (z.B. Operation, Bestrahlung, Chemotherapie, biologische Therapie) durchgeführt werden. Zusätzlich kann eine Person, deren Risiko, ein Neoplasma zu entwickeln, größer ist, (z.B. jemand, der genetisch prädisponiert ist, oder jemand, der zuvor ein Neoplasma hatte) eine prophylaktische Behandlung erhalten, um die Neoplasmabildung zu hemmen oder zu verzögern.

Die Kombination der kinesin-ATPase Eg5/KSP mit den Verbindungen der Formel V, Pentamidin, seine Analoga und /der seiner Metaboliten ist ebenfalls Gegenstand der Erfindung.

Die Dosierung und Häufigkeit der Verabreichung jeder Verbindung der Kombination kann unabhängig gesteuert werden. Zum Beispiel kann eine Verbindung dreimal täglich oral verabreicht werden, während die zweite Verbindung einmal pro Tag intramuskulär verabreicht werden kann. Die Verbindungen können auch zusammen formuliert werden, so dass eine Verabreichung beiden Verbindungen zuführt.

Die erfindungsgemäßen antiproliferativen Kombinationen können auch als Komponenten eines pharmazeutischen Pakets bereitgestellt werden. Die zwei Arzneimittel können zusammen oder getrennt und in einzelnen Dosierungsmengen formuliert werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetet oder Dichlormethan, trennt ab, trocknet die organische Phase über Natriumsulfat, dampft ein und reinigt duch Chromatographie an Kieselgel und/oder durch Kristallisation. Rf-Werte an Kieselgel; Laufmittel: Ethylacetat/Methanol 9:1.

| | |
|---|---|
| Massenspektrometrie (MS): | EI (Eletronenstoß-Ionisation) M⁺ |
| | FAB (Fast Atom Bombardment) (M+H)⁺ |
| | ESI (Electrospray lonization) (M+H)⁺ |

APCI-MS (atmospheric pressure chemical ionization - mass spectrometry) (M+H)⁺

### Beispiel 1

### Synthese von (4a,10,10a)-6-tert-Butyl-10-phenyl-2,3,4a,9,10,10a-hexahydro-1,4-dioxa-9-aza-phenanthren

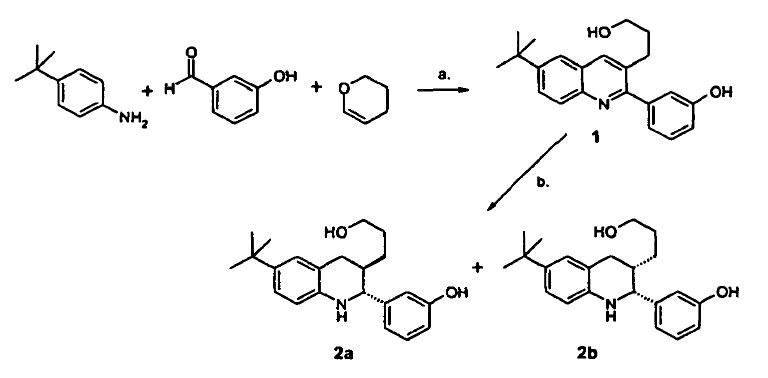

### Reaktionsbedingungen:

### a. Synthese von 3-[6-tert-Butyl-3-(3-hydroxy-propyl)-chinolin-2-yl]phenol 1

99.0 g (663 mmol) 4-tert.-Butylanilin wurde in 500 ml Acetonitril gelöst und unter Eiskühlung 51.6 mL (670 mmol) Trifluoressigsäure zugegeben. In einen zweiten Kolben wurden 46.4 g (670 mmol) 3,4-Dihydro-2H-pyran und 81.8 g (670 mmol) 3-Hydroxybenzaldehyd in 500 mL Acetonitril unter Eiskühlung vorgelegt. Zu dieser Reaktionslösung wurde das auf 5°C vorgekühlte Anilin*Trifluoressigsäuresalz rasch zugegeben und bei dieser Temperatur ca. 2h weiter gerührt. Das Reaktionsgemisch wurde eingedampft und der Rückstand in 2l Wasser suspendiert und mit Natronlauge auf pH 12 eingestellt. Die entstandene Emulsion wurde 2x mit 1l tert.-Butylmethylether extrahiert. Die Org. Phase über Na₂SO₄ getrocknet und über Nacht in bei 4°C kristallisiert. Ausgefallenes farbloses Produkt **1** wurde abfiltriert und getrocknet. Man erhielt 6.00 farblose Kristalle. Aus dem Filtrat wurde wie unter DE10360154.6 beschrieben 3-(9-tert-Butyl-3,4,4a,5,6,10b-hexahydro-2H-pyrano[3,2-c]chinolin-5-yl)-phenol isoliert, welches wie unter c. beschrieben auch in Verb. **1** überführt werden konnte.

### b. Synthese von cis- und trans-3-[6-tert-Butyl-3-(3-hydroxy-propyl)-1,2,3,4-tetrahydro-chinolin-2-yl]-phenol 3b und 3a

5.00 g (14.9 mmol) **1** wurde in absolutem Ethanol (60 mL) gelöst und auf Rückfluss erhitzt. Zu der siedenden Lösung wurde 6.00 g (0.23 mol, 17.5 eq) Natrium in kleinen Portionen über einen Zeitraum von 30-60 min zugegeben und nach beendeter Zugabe 2 h bei Rückfluss weitergerührt. Die abgekühlte Lösung wurde vorsichtig mit Eiswasser versetzt, NaHCO₃ zugefügt bis ein neutraler pH-Wert erreicht wurde und 2x mit Ethylacetat extrahiert. Die gesammelten organischen Phasen wurden getrocknet und zur Trockne eingedampft. Die Aufreinigung erfolgte säulenchromatographisch (Ethylacetat/Cyclohexan), wobei 2 Fraktionen gleicher Masse isoliert werden konnten. Es handelte sich um farblose Feststoffe, die als Verbindung trans-**2a** und cis-**2b** identifiziert werden konnten.

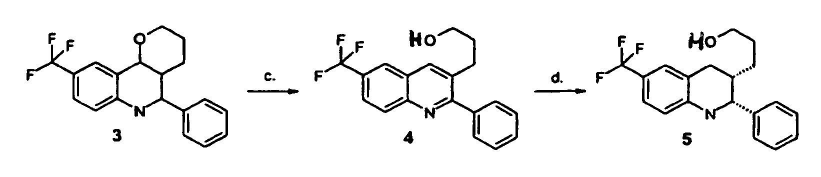

### c. Synthese von 3-(2-Phenyl-6-trifluoromethyl-chinolin-3-yl)-propan-1-ol 4

0.50 g (1.50 mmol) 5-Phenyl-9-trifluoromethyl-3,4,4a,5,6,10b-hexahydro-2H-pyrano[3,2-c]chinolin **3** wurde in THF (15 mL) gelöst, bei RT 1.65 g (3.00 mmol, 2 eq) Ammoniumcer(IV)nitrat zugefügt und über Nacht bei RT weiter gerührt. Nach beendeter Reaktion wurden 50 mL Ethylacetat zugefügt und 2x mit 50 mL Wasser extrahiert. Die org. Phase wurde getrocknet und zur Trockne eingedampft. Der Rückstand wurde aus Ethylacetat/Diethylether unkristallisiert. Man erhielt einen leicht gelben Feststoffs (Verbindung **4**).

### d. Synthese von cis-3-(2-Phenyl-6-trifluoromethyl-1,2,3,4-tetrahydrochinolin-3-yl)-propan-1-ol 5

0.44 g (1.33 mmol) **4** wurde in 15 mL Methanol aufgenommen, mit 1 g RaNi (wassemass) versetzt und über Nacht bei 60°C bei einem Wasserstoffdruck von 5 bar hydriert. Der Katalysator wurde abgetrennt, erneut 1 g des RaNi zugefügt und wiederum bei 60°C über Nacht bei 5 bar Wasserstoffdruck hydriert. Der Katalysator wurde abfiltriert, das Filtrat zur Trockne eingedampft und der Rückstand säulenchromatographisch aufgereinigt (MeOH/Ethylacetat). Man erhielt einen farblosen Feststoff, der als die racemische Verbindung **5** identifiziert wurde.

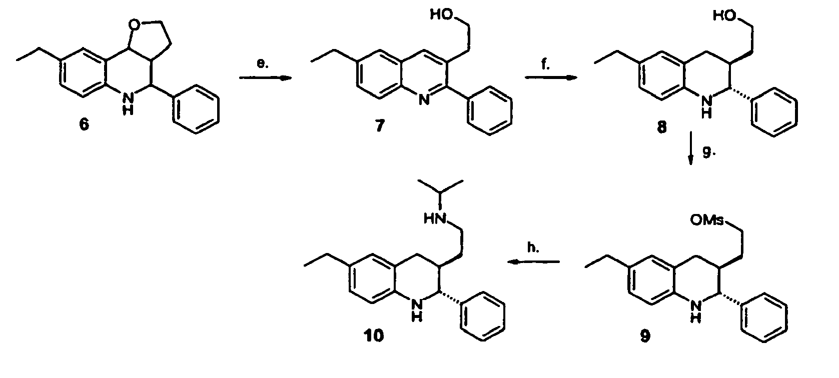

### e. Synthese von 2-(6-Ethyl-2-phenyl-chinolin-3-yl)-ethanol 7

Wie unter c. beschrieben wurden 1.00 g (3.58 mmol) **6** mit 3.92 g (7.16 mmol, 2 eq) Ammoniumcer(IV)nitrat in THF (30 mL) zu Verbindung **7** als gelblichen Feststoff umgesetzt.

### f. Synthese von trans- 2-(6-Ethyl-2-phenyl-1,2,3,4-tetrahydro-chinolin-3-yl)-ethanol 8

Wie unter b.) beschrieben wurden 0.80 g (2.88 mmol) **7** mit 0.66 g (28.8 mmol, 10 eq) Natrium in EtOH (10 mL) zu Verbindung trans-**8** als farblosen Feststoff und Verbindung cis-**8** ebenfalls als farblosen Feststoff umgesetzt.

### g. Synthese von trans- 2-(6-ethyl-2-phenyl-1,2,3,4-tetrahydro-chinolin-3-yl)-methansulfonsäureethylester 9

0.10 g (0.36 mmol) trans-8 wurde in DCM (2 mL) gelöst, dann zuerst 76 mg (0.75, 2.1 eq) Triethhylamin und 50 mg (0.43 mmol, 1.2 eq) Methansulfonylchlorid, gelöst in 1 mL DCM, bei RT zugefügt und weitere 30 min bei gleicher Temperatur gerührt. Die Mischung wurde zur Trockne eingedampft, in Ethylacetat aufgenommen und 2 x mit Wasser gewaschen. Die organische Phase wurde getrocknet, zur Trockne eingedampft und ohne weitere Aufreinigung weiter umgesetzt. Man erhielt ein farbloses Öl, welches über Nacht auskristallisierte (Verbindung **9).**

### h. Synthese von trans-[2-(6-Ethyl-2-phenyl-1,2,3,4-tetrahydro-chinolin-3-yl)-ethyl]-isopropylamin 10

0.10 g (0.28 mmol) **9** wurde in Isopropylamin (0.2 mL) gelöst und bei 100°C 18 h im Druckkolben erhitzt. Das überschüssige Amin wurde durch Vakuumevaporation entfernt und der Rückstand in Ethylacetat aufgenommen und mit Wasser extrahiert. Die organische Phase wurde getrocknet, zur Trockne eingedampft und der Rückstand mit einem Überschuss von HCl in iPrOH und mit Diethylether versetzt. Der kristalline Feststoff wurde abfiltriert und getrocknet. Man erhielt einen farblosen Feststoff (Verbindung **10** als Monohydrochlorid-Salz).
Teilweise müsste auf dieser Stufe eine säulenchromatische Aufreinigung gewählt werden, da Verbindung **8** als Nebenprodukt zurückgebildet wurde.

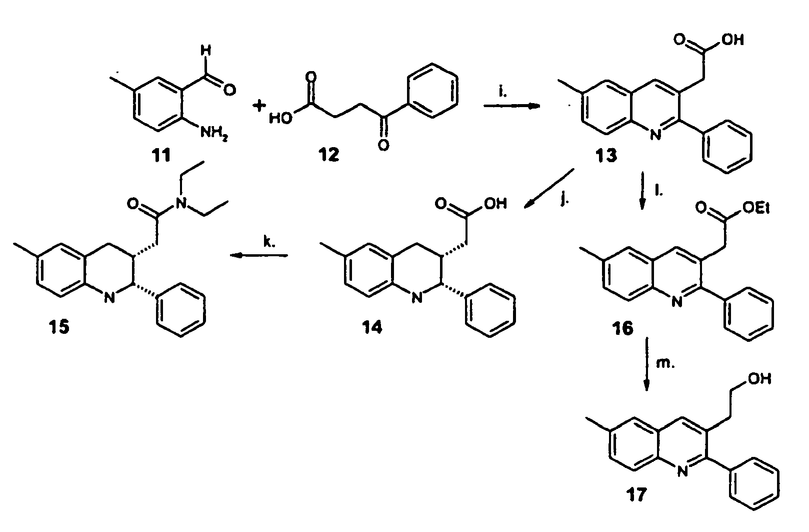

### i. Synthese von (6-Methyl-2-phenyl-chinolin-3-yl)-essigsäure 13

2.00 g (14.6 mmol) 2-Amino-5-methylbenzaldehyd **11** (erhalten durch Umsetzung von 2 g (14.6 mmol) 2-Amino-5-methyl-benzylalkohol mit 6.52 g (75.0 mmol) Mangandioxid in 50 mL DMF 3 h bei RT, anschließende Filtration über Celite und Evaporation des Lösungsmittels) und 2.60 g (14.6 mmol) 3-Benzoylpropionsäure **12** wurden 40 mL MeOH aufgenommen, bei RT 10 mL 2N NaOH-Lösung in Wasser zugefügt und 18 h unter Rückfluss erhitzt. Die Mischung wurde mit 15 mL Wasser verdünnt und Essigsäure zugefügt bis der pH-Wert in den sauren Bereich umschlug. Der sich ausgebildete Niederschlag wurde abfiltriert und getrocknet. Der erhaltene farblose Feststoff konnte als das gewünschte Produkt **13** mit hoher Reinheit identifiziert werden.

### j. Synthese von (6-Methyl-2-phenyl-1,2,3,4-tetrahydro-chinolin-3-yl)-essigsäure 15

1.00 g (3.61 mmol) **13** wurde in 80 mL Methanol aufgenommen, mit 1 g RaNi (wassemass) versetzt und über Nacht bei 50°C bei einem Wasserstoffdruck von 5 bar hydriert. Der Katalysator wurde abfiltriert, das Filtrat zur Trockne eingedampft und der Rückstand säulenchromatographisch aufgereinigt (Ethylacetat/Cyclohexan). Man erhielt einen gelblichen Feststoff, der als die racemische cis-Verbindung **14** identifiziert wurde.

### k. Synthese von N,N-Diethyl-2-(6-methyl-2-phenyl-1,2,3,4-tetrahydrochinolin-3-yl)-acetamid 15

100 mg (0.36 mmol) **14** und 50 mL (0.49 mmol, 1.4 eq) Diethylamin in 1 mL DMF wurden vorgelegt und unter Rühren bei RT 50 mg (0.37 mmol) 1-Hydroxybenzotriazol, 140 mg (0.73 mmol) N-(3-Dimethylaminopropyl)-N'-ethylcarbodiimidhydrochlorid sowie 80 mL (0.73 mmol) 4-Methylmorpholin zugefügt. Anschließend wurde das Reaktionsgemisch 2 h bei RT gerührt. Die Mischung wurde mit *tert*.-Butylmethylether versetzt und 2 x mit Wasser extrahiert. Die organische Phase wurde getrocknet, eingeengt und der Rückstand säulenchromatographisch aufgereinigt (Ethylacetat/Cyclohexan). Man erhielt einen farblosen Feststoff, welcher als Verbindung **15** identifiziert wurde.

### Alternative Synthese zu 2-(2-phenyl-chinolin-3-yl)ethanolen

### I. Synthese von (6-Methyl-2-phenyl-chinolin-3-yl)-essigsäureethylester 16

0.50 g (1.80 mmol) **13** wurde in 10 mL EtOH gelöst, ein Tropfen konz. Schwefelsäure zugegeben und 2 h unter Rückfluss erhitzt. Das Lösungsmittel wurde entfernt, der Rückstand in Ethylacetat aufgenommen und 2x mit Wasser gewaschen. Die organische Phase wurde getrocknet und eingeengt. Bei dem farblosen öligen Rückstand handelt es sich um die Verbindung **16**.

### m.Synthese von 2-(6-Methyl-2-phenyl-chinolin-3-yl)-ethanol 17

Zu einer Suspension von 0.12 g (3.27 mmol) Lithiumaluminiumhydrid (LiAlH₄, Pulver) in 10 mL THF wurde unter Rühren und Eiskühlung 0.50 g (1.64 mmol) **16**, gelöst in 5 mL THF, langsam zugetropft. Die Mischung wurde 3 h bei RT gerührt, durch Zusatz von gesättigter AmmoniumchloridLösung überschüssiges LiAlH₄ zerstört und 2x mit Ethylacetat extrahiert. Die vereinigten organischen Phasen wurden getrocknet, das Lösungsmittel entfernt und der Rückstand säulenchromatographisch aufgereinigt (Ethylacetat/Cyclohexan). Man erhielt einen farblosen Feststoff (Verbindung **17**).
Diese Verbindung kann wie unter f., g. und h. beschrieben weiter umgesetzt werden.

Alle weiteren erfindungsgemäßen Verbindungen lassen sich analog aus den entsprechenden Vorstufen erhalten.

### Beispiel A: Assay I

Die Bestimmung der Wirksamkeit der erfindungsgemäßen Verbindungen kann z. B. über die Eg5-ATPase Aktivität, die über eine enzymatische Regeneration des Produkts ADP zur ATP mittels Pyruvatkinase (PK) und anschließender Kopplung an eine NADH-abhängige Laktat-Dehydrogenase (LDH) Reaktion gemessen wird, erfolgen. Durch die Kopplung an die NADH-abhängige LDH kann die Reaktion über die Änderung der Extinktion bei 340 nm verfolgt werden. Die Regeneration des ATP gewhärleistet gleichzeitig, dass die Substratkonzentration konstant bleibt. Die Extinktionsänderung pro Zeiteinheit werden graphisch analysiert und eine lineare Regression im vusuell linearen Bereich der Reaktion durchgeführt.

### Beispiel B: Assay II

Die Kombination aus dem Antiprotozoikum Pentamidin und den Inhibitoren der Kinesin-ATPase Eg5/KSP führt zu verstärkter hemmender Wirkungen bei Zellproliferationstests mit der Kolon-Karzinom-Zelllinie HCT116. Eg5-Inhibitoren stören die ATPase-Aktivität und hemmen den Verlauf des Zellzyklus aufgrund eines Fehlers bei der Trennung der Spindelpole.

Die Bestimmung der Wirksamkeit der erfindungsgemäßen Verbindungen der Formel I in Kombination mit Verbindungen der Formel V und/oder Arzneimitteln der Tabelle I kann in Kombinations-Assays wie folgt gezeigt werden:
10³ bis 10⁴ Zellen einer definierten Zell-Linie (HCT116, Colo 205, MDA-MB 231, etc.) werden pro Vertiefung in einer 96-well Mikrotiterplatte ausgesät und über Nacht unter Standardbedingungen kultiviert. Für die Substanzen der zu testenden Kombination wurden 10-50 mM Stocklösungen in DMSO vorbereitet. Verdünnungsreihen (i.d.R. 3-fach Verdünnungsschritte) der einzelnen Substanzen wurden in Form einer Pipettierschemas (s. Schema unten), unter Konstanthaltung einer DMSO Endkonzentration von 0,5 % (v/v) miteinander kombiniert. Die Zellen wurden am nächsten Morgen mit den Substanzgemischen versetzt und für weitere 48 Stunden unter Kulturbedingungen inkubiert. Am Ende der Kultivierung erfolgte eine Kristallviolett-Färbung der Zellen. Nach Extraktion des Kristallviolett aus den fixierten Zellen wurde die Absorption bei 550 nm spektralphotometrisch gemessen. Sie kann als quantitatives Maß für die vorhandenen adhärenten Zellen herangezogen werden.

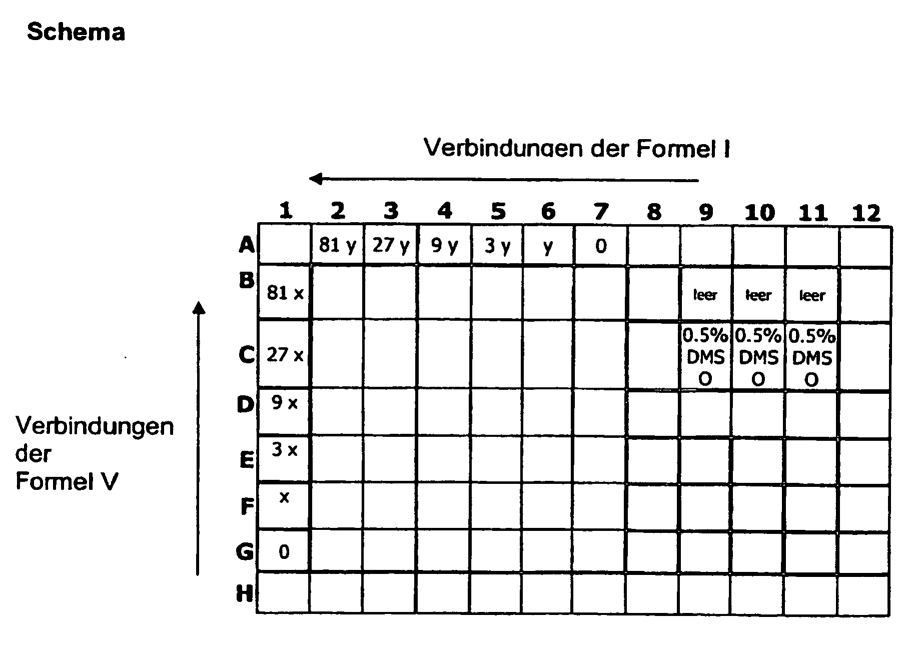

### Beispiel C: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel D: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel E: Lösung

Man bereitet eine Lösung aus 1 g eines Wirkstoffes der Formel I, 9,38 g NaH₂PO₄· 2 H₂O, 28,48 g Na₂HPO₄· 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel F: Salbe

Man mischt 500 mg eines Wirkstoffes der Formel I mit 99,5 g Vaseline unter aseptischen Bedingungen.

### Beispiel G: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel H: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel I: Kapseln

2 kg Wirkstoff der Formel I werden in üblicher Weise in Hartgelatinekapseln gefüllt, so daß jede Kapsel 20 mg des Wirkstoffs enthält.

### Beispiel J: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 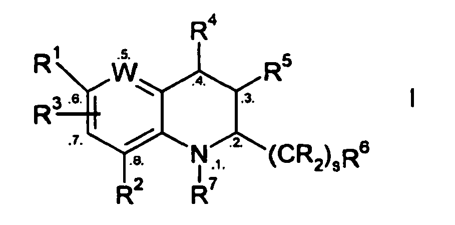 worin
W CH oder N,
R¹, R², R³ unabhängig voneinander H, A, Aryl, Heteroaryl, Hal, -(CY₂)ₙ-SA, -(CY₂)ₙ-SCF₃, -(CY₂)ₙ-SCN, -(CY₂)ₙ- CF₃, -(CY₂)ₙ-OCF₃, Cycloalkyl, -SCH₃, -SCN, -CF₃, -OCF₃, -OA, -(CY₂)ₙ-OH, -(CY₂)ₙ-CO₂R, -(CY₂)ₙ-CN, -(CY₂)ₙ-Hal, (CH₂)ₙR, -(CY₂)ₙ-NR₂, (CY₂)ₙ-OR, (CY₂)ₙ-OCOA, -SCF₃, (CY₂)ₙ-CONR₂, -(CY₂)ₙ-NHCOA, -(CY₂)ₙ-NHSO₂A, SF₅, Si(CH₃)₃, CO-(CY₂)ₙ-CH₃, -(CY₂)ₙ-N-Pyrolidon, (CH₂)ₙNRCOOR, NRCOOR, NCO, CH₂(CH₂)ₙCOOR, NHCOOR, CH₂(CH₂)ₙOH, NR(CH₂)ₙOH, CH₂NH₂, (CH₂)ₙNR₂, CH(OH)R₂, CH₂NHCOR, (CH₂)ₙAryl, CH₂(CH₂)ₙHeteroaryl, (CH₂)ₙR¹, NH(CH₂)ₙCOOR, CH₂(CH₂)ₙX(CH₂)ₙAryl, CH₂(CH₂)ₙX(CH₂)ₙHeterogryl, NH(CH₂)ₙCONR₂, XCONR(CH₂)ₙNR₂, N[(CH₂)ₙXCOOR]CO(CH₂)ₙAryl, N[(CH₂)ₙXR]CO(CH₂)ₙAryl, N[(CH₂)ₙXR]CO(CH₂)ₙXAryl, N[(CH₂)ₙXR]SO₂(CH₂)ₙAryl, N[(CH₂)ₙNRCOOR]CO(CH₂)ₙAryl, N[(CH₂)ₙNR₂]CO(CH₂)ₙAryl, N[(CH₂)ₙNR₂]CO(CH₂)ₙNRAryl, N[(CH₂)ₙNR₂]SO₂(CH₂)ₙAryl, N[(CH₂)ₙXR]CO(CH₂)ₙHeteroaryl, N[(CH₂)ₙXR]CO(CH₂)ₙXHeteroaryl, N[(CH₂)ₙXR]SO₂(CH₂)ₙHeteroaryl, N[(CH₂)ₙNRCOOR]CO(CH₂)ₙHeteroaryl, N[(CH₂)ₙNR₂]CO(CH₂)ₙHeteroaryl, N[(CH₂)ₙNR_{2]}CO(CH₂)ₙNRHeteroaryl wobei nicht benachbarte Gruppen CY₂ auch durch X ersetzt sein können, R¹ und R³ zusammen auch -N-C(CF₃)=N-, -N-CR=N-, -N-N=N-,
Y H, A, Hal, OR¹, N(R¹)₂, E-R¹
A Alkyl oder Cycloalkyl, worin eines oder mehrere H- Atome durch Hal und/oder eine oder mehrere nicht benachbarte CH₂ Gruppen durch X ersetzt sein können,
Hal F, Cl, Br oder l R H oder A, bei geminalen Resten R zusammen auch -(CH₂)₅-, -(CH₂)₄- oder -(CH₂)ₙ-X-(CH₂)ₙ, oder - (CHz)ₙ-Z-(CH₂)ₙ,
R⁴ H
R⁵ -(CY₂)ₙ-E-(CY₂)ₙ-XR¹, (CH₂)₂OH, (CH₂)₃OH, (CH₂) ₂NRR¹, (CH₂)₃NRR¹, (CH₂)₂CH(OH)CH₂OH, (CH₂)₂CH(OH)CH₂NRR¹, (CH₂)₂CH(OH)CH₂NR(CH₂)₂NRR¹, (CH₂)₂CH(OH)CH₂NR⁵(CH₂)₂NR, (CH₂)₂CH(OH)CH₂-E-R¹, Q oder (CH₂)₂CH(OH)CH₂NR(CH₂)₂OR,
E NR¹SO₂-, -SO₂NR¹-, -CONR¹-, -NR'CO-, -COO-, - OOC-, NR¹CONR¹-, -OCONR¹-, -NR¹COO-, - CSNR¹-, -NR¹CS-, -NR¹CSNR¹-, -SCONR¹-, - NR¹COS-, -OCSNR¹-, NR'CSO-, SCSNR'-, - NR'CSS oder eine Einfachbindung
X O, S oder NR¹,
Q (CH₂)ₚHal, CHO, COR^{a}, (CH₂)ₚR^{a} , (CH₂)ₚOCOR^{a}, (CH₂)ₚXR¹, (CH₂)ₚNCOR¹, (CH₂)ₚN(R¹)₂, (CH₂)ₚOR¹, (CH₂)ₚOCON(R¹)₂, (CH₂)ₚOCOOR¹, (CH₂)ₚNHCON(R¹)₂, (CH₂)ₚNHCOOR¹, (CH₂)ₚCN, (CH₂)ₚCOOR¹, (CH₂)ₚ-E-(CH₂)ₚR¹, (CH₂)ₚ-E- (CH₂)ₚR^{a}, wobei nicht benachbarte CH₂-Gruppen auch durch X ersetzt warden können.
R^{a} 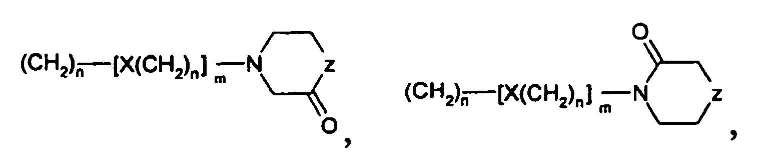 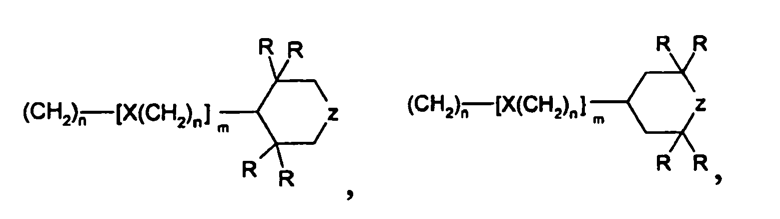 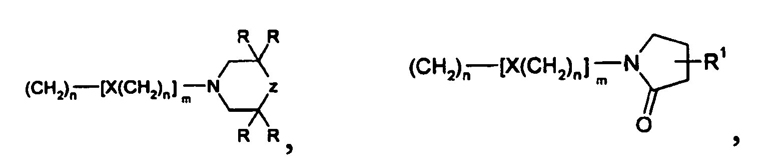 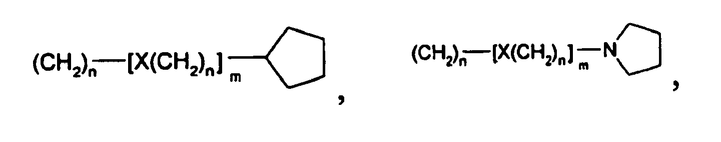 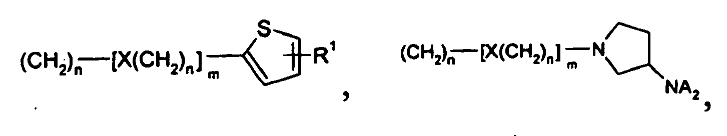 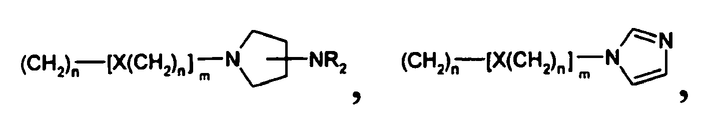 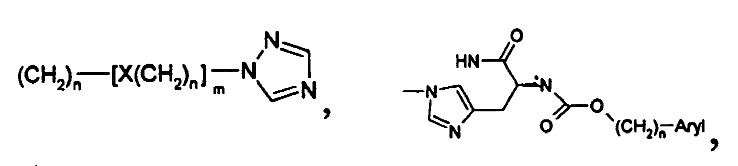 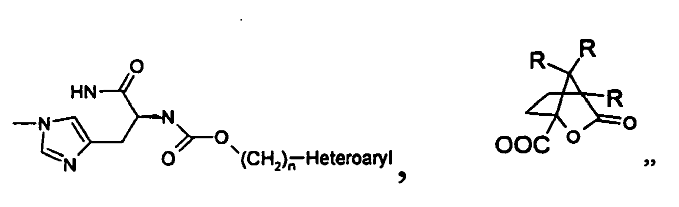 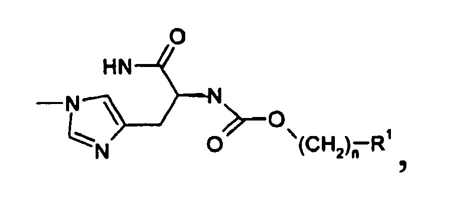 OR, NHR, NR₂, NR(CH₂)ₙAryl, NR(CH₂)ₙOR, COOR, N- Pyrrolidon-Rest, OCOR, NR(CH₂)ₙNR₂, N[(CH₂)ₙNR₂]CO(CH₂)ₙAryl, N[(CH₂)ₙNHCOOR]COAryl, R¹, N[CH₂(CH₂)ₙOR]₂, NR(CH₂)ₙNCOOR, X(CH₂)ₙX(CH₂)ₙXR, NR(CH₂)ₙX(CH₂)ₙOH, NR(CH₂)ₙO(CH₂)ₙOH, (CH₂)ₙCOOR, O(CO)NR(CH₂) ₙOR, O(CO)(CH₂)ₙNR₂, NR(CH₂)ₙNR₂, N[(CH₂)ₙNR₂]CO(CH₂)ₙAryl, N[(CH₂)ₙXR]CO(CH₂)ₙAryl, N[(CH₂)ₙXR]CO(CH₂)ₙHeteroaryl, N[(CH₂)ₙNR₂]CO(CH₂)ₙHeteroaryl, N[(CH₂)ₙNR₂]CO(CH₂)ₙR¹, N(R)(CH₂)ₙN(R)COOR, XCOO(CH₂)ₙNR₂, OSO₂A, OSO₂CF₃, OSO₂Ar, OCONR₂, OCH₂(CH₂)ₙNR₂
Z CH₂, X, CHCONH₂, CH(CH₂)ₙNR¹COOR¹, CHNR¹COOR¹, CHCON(R¹)₂, NCO, CH(CH₂)ₙCOOR¹, NCOOR¹, CH(CH₂)ₙOH, N(CH₂)ₙOH, CHNH₂, CH(CH₂)ₙN(R¹)₂, CH(CH₂)ₙN(R¹)₂, C(OH)R¹, CHNCOR¹, NCOR¹, N(CH₂)ₙAryl, N(CH₂)ₙHeteroaryl, CHR¹, NR¹, CH(CH₂)ₙAryl, CH(CH₂)ₙHeteroaryl, CH(CH₂)ₙR¹, N(CH₂)ₙCOOR¹, CH(CH₂)ₙX(CH₂)ₙR¹, CH(CH₂)ₙX(CH₂)ₙR^{a}, N(CH₂)ₙCON(R¹)₂, XCONR¹(CH₂)ₙN(R¹)₂, CO(CH₂)ₙR¹, CO(CH₂)ₙR¹, CO(CH₂)ₙXR¹, SO₂(CH₂)ₙR¹, O(CH₂)ₙN(R¹)₂, X(CH₂)ₙN(R¹)₂, NCO(CH₂)ₙN(R¹)₂, CHR^{a}, NR^{a},
R⁶ unsubstituiertes oder einfach oder mehrfach durch Aryl oder Heteroaryl, das durch Hal, NO₂, CN, A, OR, OCOR, COR, NR₂, CF₃, OCF₃, OCH(CF₃)₂ substituiert sein kann, Hal, NO₂, CN, OR, A, -(CY₂)ₙ-OR, -OCOR, -(CY₂)ₙ- CO₂R, -(CY₂)ₙ-CN, -NCOR, -COR oder -(CY₂)ₙ-NR₂ substituiertes Aryl oder Heteroaryl,
R⁷ (C=O)-R, (C=O)-NR₂, (C=O)-OR, H oder A worin R⁵ und R⁷ zusammen auch -(CH₂)ₙ- bedeuten kann,
m 0, 1 oder 2
n 0, 1, 2, 3, 4, 5, 6 oder 7,
und
p 0, 1, 2, 3, 4, oder 5, bevorzugt 2 oder 3
S 0, 1, 2, 3, 4, 5, 6 oder 7, bevorzugt 0
bedeuten,
sowie ihre Solvate, Tautomere, Salze und Stereoisomere, einschließlich deren Mischungen in allen Verhältnissen.

2. Verbindungen nach Anspruch 1, worin
R¹ A, CF₃, OCF₃, SA, SCN, CH₂CN, -OCOA, Hal, SCF₃, t- Butyl, -CH(CH₃)CH₂CH₃, Isopropyl, Ethyl oder Methyl
bedeutet.

3. Verbindungen nach Anspruch 1 oder 2, worin
R² H bedeutet
bedeutet.

4. Verbindungen nach einem oder mehreren der Ansprüche 1-3, worin
R³ H

5. Verbindungen nach einem oder mehreren der Ansprüche 1-4, worin
W CH bedeutet.

6. Verbindungen nach einem oder mehreren der Ansprüche 1-5, worin
R⁶ unsubstituiertes oder einfach oder mehrfach durch Hal, CN, NO₂, OH, CF₃, OCH(CF₃)₂, OCOCH₃ oder A substituiertes Phenyl, 2-, 3- oder 4-Pyridyl, Pyrimidyl, Furyl oder Thienyl
bedeutet.

7. Verbindungen nach einem oder mehreren der Ansprüche 1-6, worin R⁶ eine der folgenden Gruppen bedeutet:

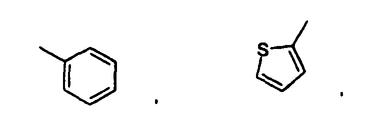

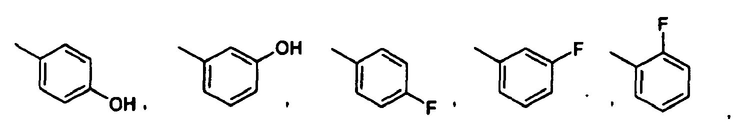

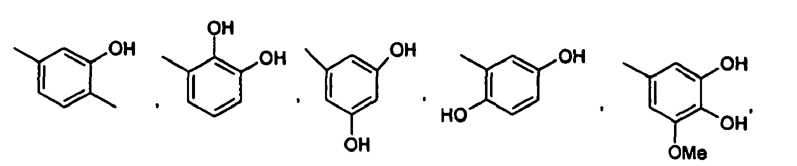

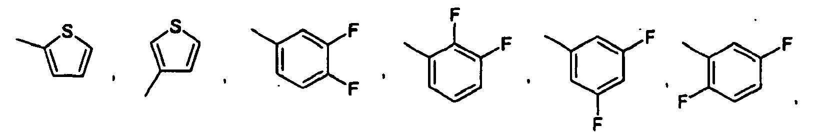

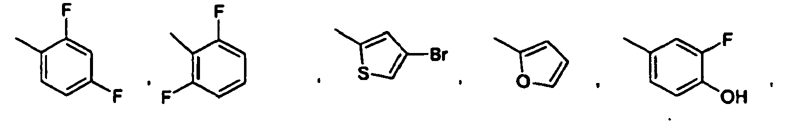

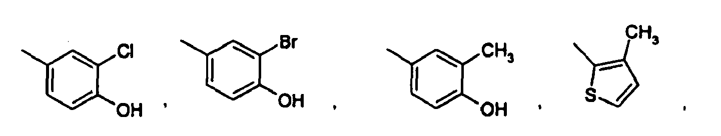

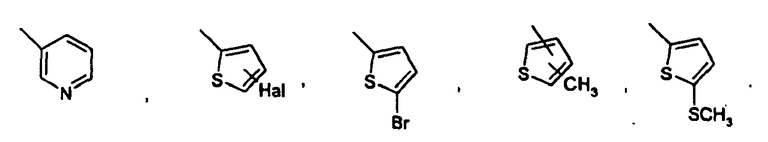

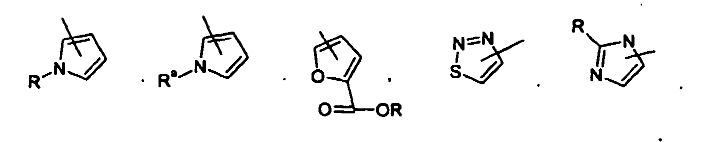

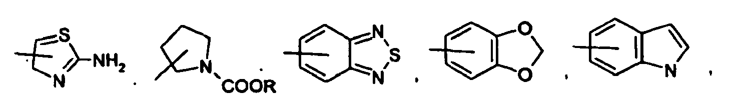

8. Verbindungen nach einem oder mehreren der Ansprüche 1-7, worin
R⁷ H bedeutet.

9. Verbindungen nach Anspruch 1 mit den Teilformeln IA bis IB: 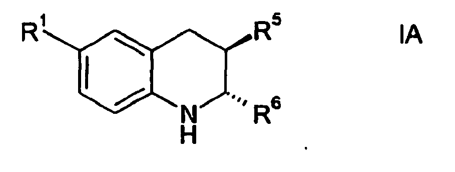 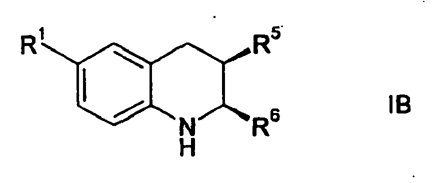 worin R¹, R⁵ und R⁶ die in Anspruch 1 angegebene Bedeutung aufweisen, sowie deren Racemat oder andere Gemische der Enantiomeren.

10. Verbindungen nach Anspruch 1 mit den Teilformeln I1 bis I27:

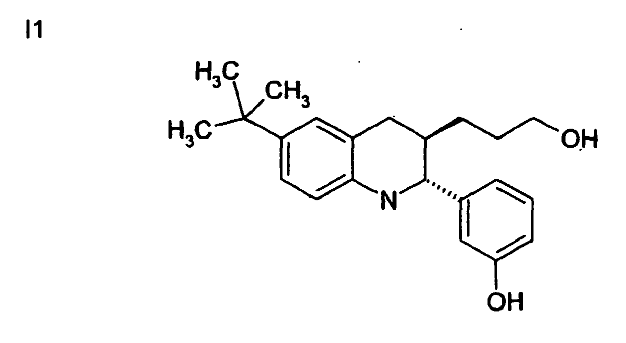

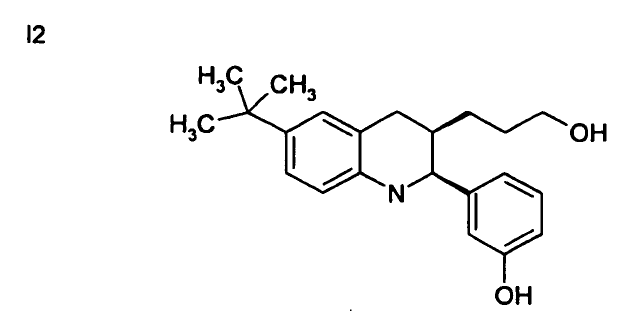

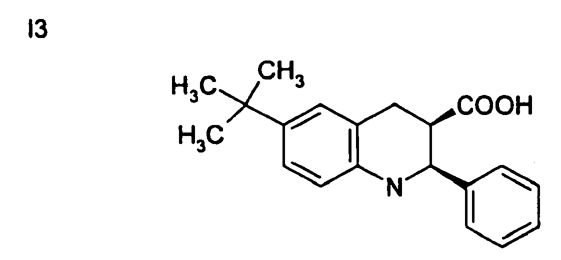

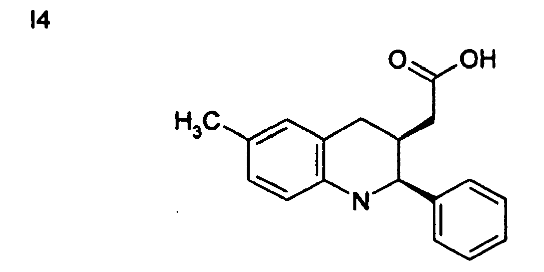

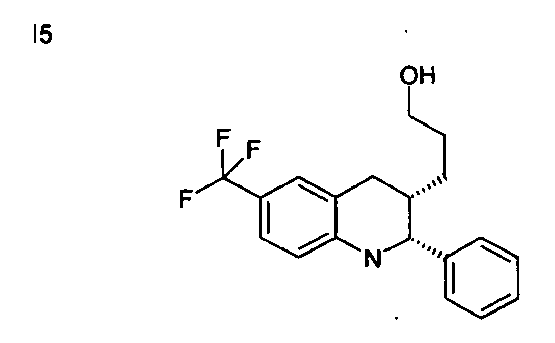

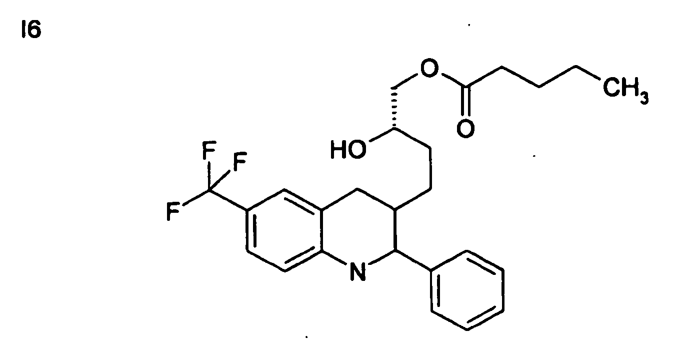

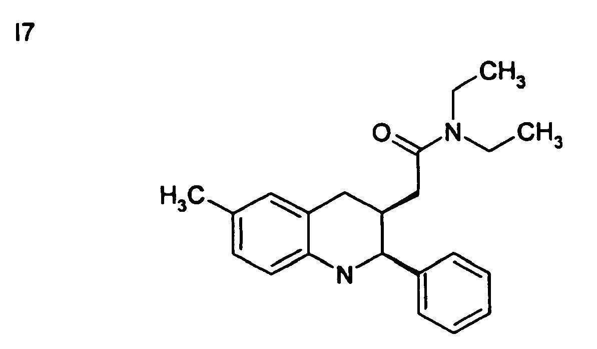

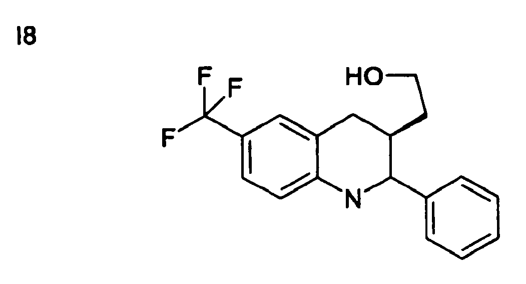

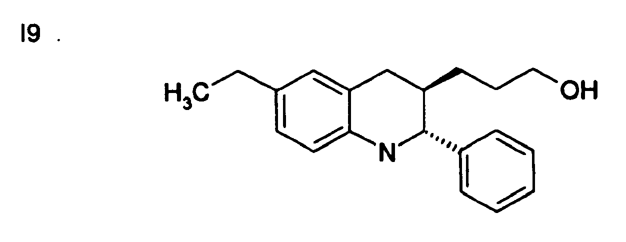

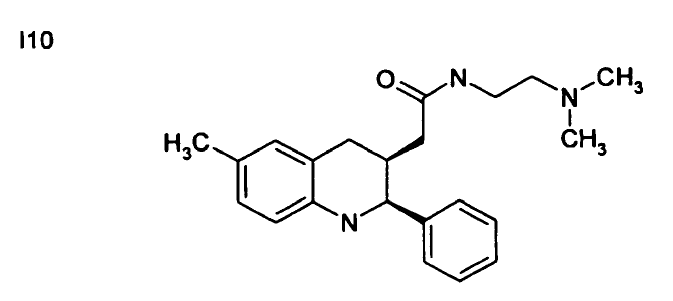

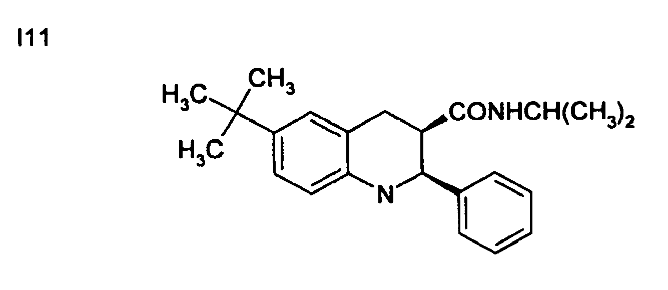

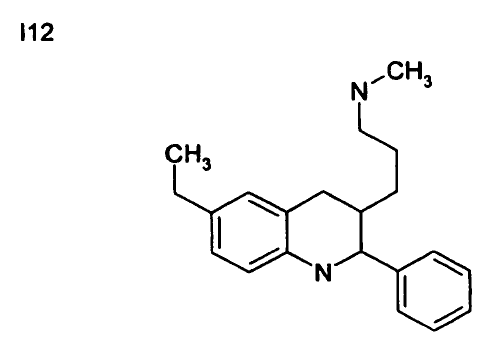

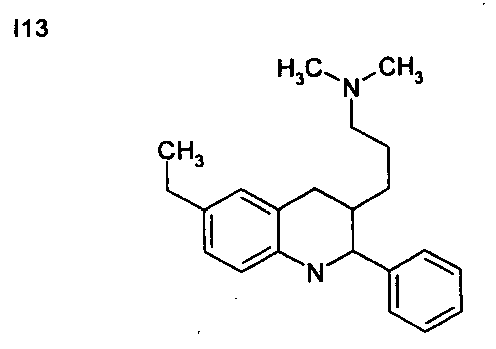

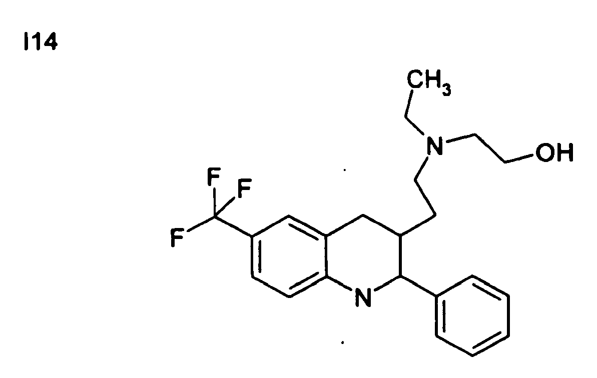

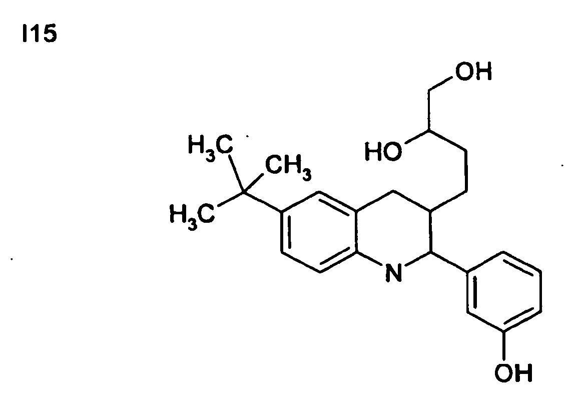

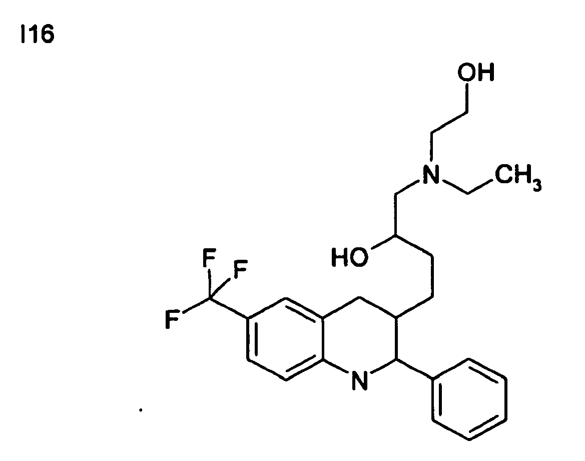

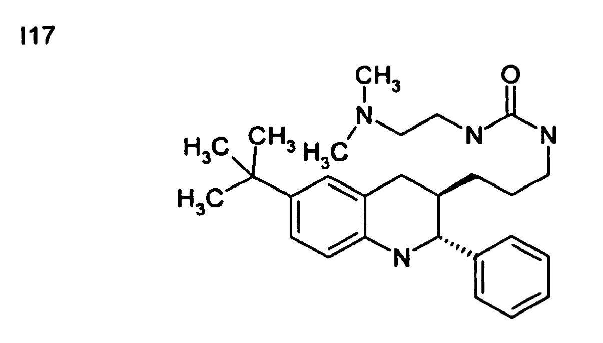

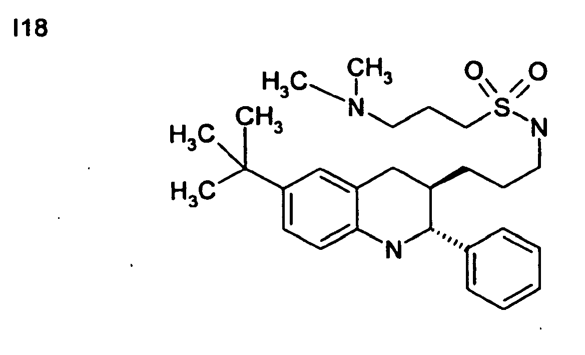

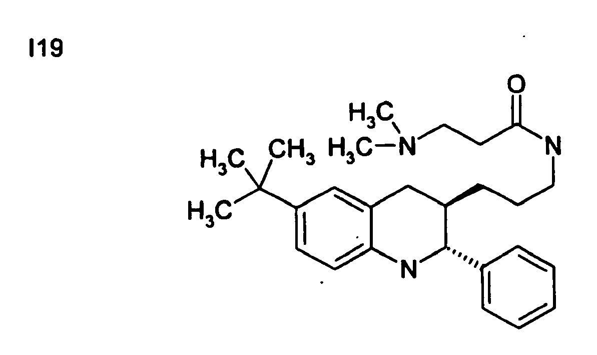

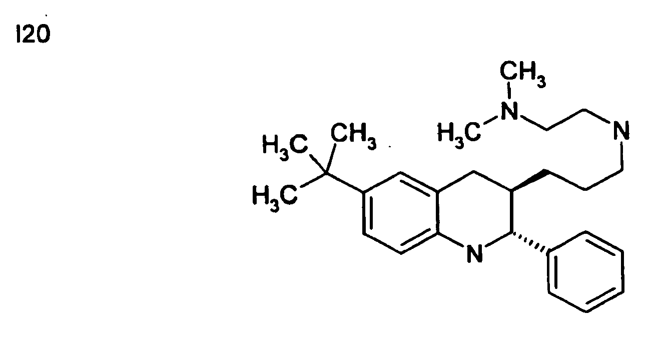

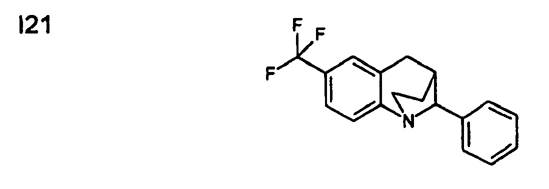

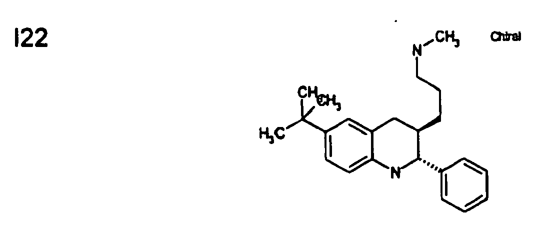

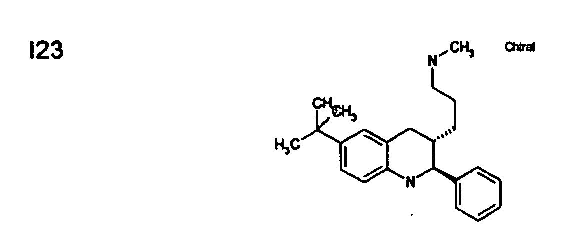

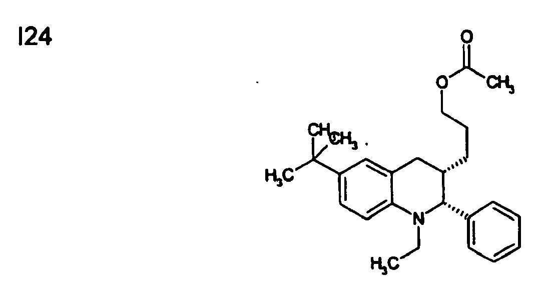

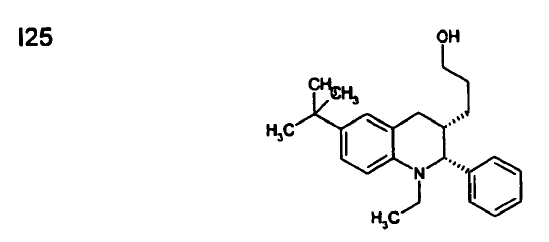

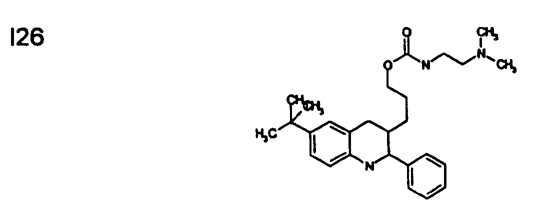

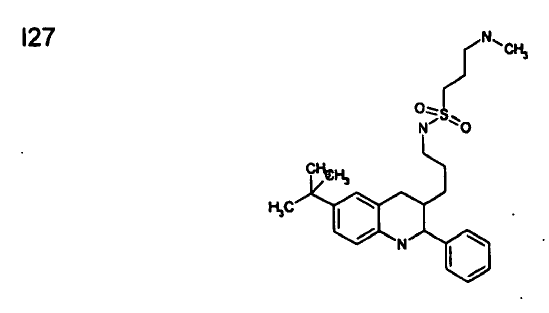

11. Verbindungen der Formel I nach den Ansprüchen 1-10, worin S O bedeutet.

12. Verfahren zur Herstellung von Verbindungen der Formel I nach den Ansprüchen 1-11 sowie ihrer Salze, Solvate, Tautomeren und Stereoisomeren, **dadurch gekennzeichnet, daß** man
eine Verbindung der Formel II 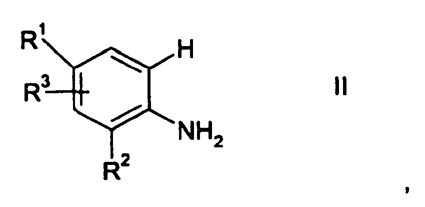 worin R¹, R² und R³ die in Anspruch 1 angegebenen Bedeutungen haben,
mit einer Verbindung der Formel III 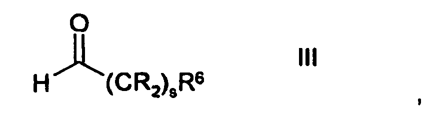 worin
R⁶ die in Anspruch 1 angegebene Bedeutung aufweist,
und
mit einer Verbindung der Formel IV, 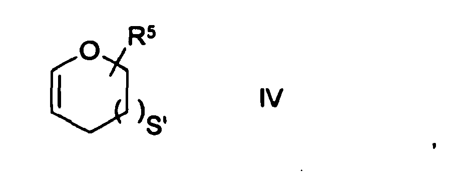 worin S' 0, 1 oder 2 bedeutet oder entsprechend substituierte Verbindungen und die erhaltenen Tetrahydrochinoline durch Anwendung von Säure in die entsprechenden Chinoline überführt, diese dann zu den Verbindungen der Formel I reduziert und gegebenenfalls den Rest R⁵ nach üblichen Methoden in die weiteren Reste R⁵ umwandelt,
und gegebenenfalls einen Rest R⁷ der H bedeutet in einen Rest R⁷ der eine andere Bedeutung als H aufweist, umwandelt
und/oder gegebenenfalls
eine Base oder Säure der Formel I in eines ihrer Salze umwandelt.

13. Verfahren nach Anspruch 12, **dadurch gekennzeichnet, dass** die Umsetzung in Gegenwart einer Protonensäure oder Lewis-Säure stattfindet.

14. Verfahren nach Anspruch 12 oder 13, **dadurch gekennzeichnet, dass** die Umsetzung in Gegenwart von Trifluoressgsäure, Hexafluorisopropanol, Bismut (III) chlorid, Ytterbium(III)triflat, Scandium (III) triflat oder Cerammonium (IV)nitrat stattfindet.

15. Arzneimittel, enthaltend mindestens eine Verbindung der Formel I nach Anspruch 1 bis 11 und/oder ihre Salze, Solvate, Tautomere und Stereoisomeren, einschließlich deren Mischungen in allen Verhältnissen, sowie gegebenenfalls Träger- und/oder Hilfsstoffe.

16. Mischung enthalten eine oder mehrere Verbindungen der Formel I nach den Ansprüchen 1-11 sowie Menge einer oder mehrerer Verbindungen der Formel V, 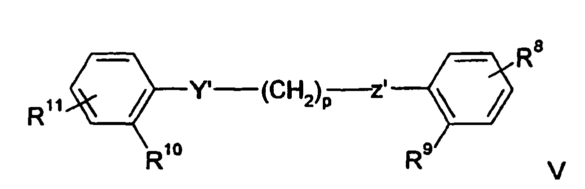 worin Y' und Z' jeweils unabhängig voneinander O oder N bedeuten, R⁹ und R¹⁰ jeweils unabhängig voneineander H, OH, Halogen, OC1-10-Alkyl,
OCF₃, NO₂ oder NH₂ bedeuten, n eine ganze Zahl zwischen 2 und 6, jeweils einschließlich, bedeutet und R⁸ und R¹¹ jeweils unabhängig voneinander an der meta- oder para-Position stehen und aus der Gruppe: 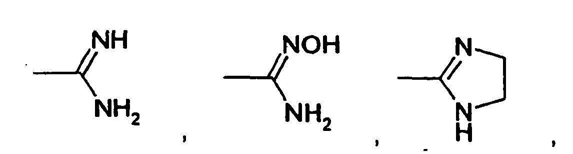 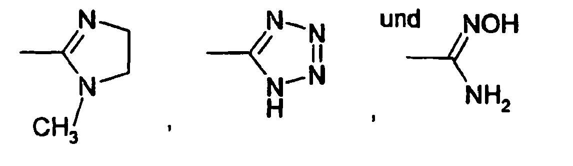 ausgewählt sind.

17. Mischung nach Anspruch 16, wobei als Verbindung der Formel V Pentamidin oder seine Salze verwendet werden.

18. Verwendung von Verbindungen nach Anspruch 1 bis 11 sowie ihrer Salze, Solvate, Tautomeren und Stereoisomeren, einschließlich deren Mischungen in allen Verhältnissen oder der Mischung nach Anspruch 16, zur Herstellung eines Arzneimittels zur Behandlung von Krankheiten, die durch die Hemmung, Regulierung und/oder Modulation der mitotischen Motor-Proteins Eg5 beeinflusst werden können.

19. Verwendung von Verbindung nach Anspruch 1 bis 11 oder der Mischung nach Anspruch 16, zur Herstellung eines Arzneimittels zur Behandlung und Prophylaxe von Krebskrankheiten.

20. Verwendung nach Anspruch 19, wobei die Krebskrankheiten mit einem Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopf und/oder der Lunge einhergehen.

21. Verwendung nach Anspruch 20, wobei der Tumor aus der Gruppe Monozytenleukämie, Lungenadenokarzinom, kleinzellige Lungenkarzinome, Bauchspeicheldrüsenkrebs, Glioblastome und Brustkarzinom und Kolokarzinom stammt.

22. Verwendung nach Anspruch 19, wobei die zu behandelnde Krebs-Krankheit ein Tumor des Blut- und Immunsystems ist.

23. Verwendung nach Anspruch 22, wobei der Tumor aus der Gruppe der akuten myelotischen Leukämie, der chronischen myelotischen Leukämie, akuten lymphatischen Leukämie und/oder chronischen lymphatischen Leukämie stammt.

24. Verwendung von Verbindungen der Formel I gemäß Anspruch 1 bis 11 und/oder ihrer physiologisch unbedenklichen Salze und Solvate zur Herstellung eines Arzneimittels zur Behandlung von Tumoren in Kombination mit einer therapeutisch wirksamen Menge einer oder mehrerer Verbindungen der Formel V, 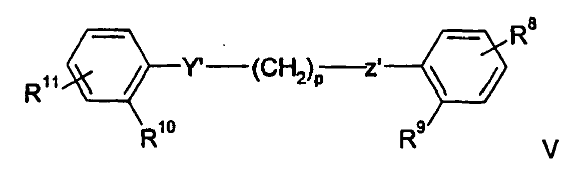 worin Y' und Z' jeweils unabhängig voneinander O oder N bedeuten, R⁹ und R¹⁰ jeweils unabhängig voneineander H, OH, Halogen, OC1-10-Alkyl,
OCF₃, NO₂ oder NH₂ bedeuten, n eine ganze Zahl zwischen 2 und 6, jeweils einschließlich, bedeutet und R⁸ und R¹¹ jeweils unabhängig voneinander an der meta- oder para-Position stehen und aus der Gruppe: 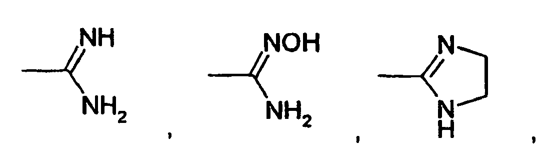 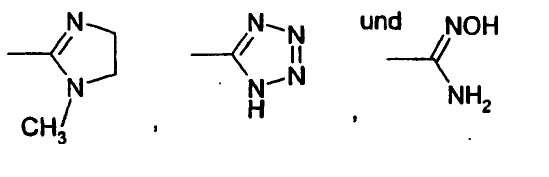 ausgewählt sind, wobei
die Verbindungen der Formel I und die Verbindungen der Formel V gleichzeitig oder innerhalb von 14 Tagen voneinander in Mengen verabreicht werden, die ausreichen, um das Wachstum eines Tumors oder von anderen hyperproliferativen Zellen zu hemmen.

25. Verwendung nach Anspruch 24, wobei als Verbindung der Formel V Pentamidin oder seine Salze verwendet werden.

26. Zwischen verbindungen der Formel 1A 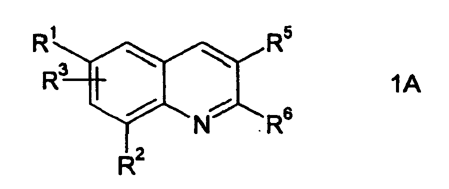 worin R¹, R², R³, R⁵ und R⁶ die in Anspruch 1 angegebene Bedeutung aufweisen.

27. Verwendung der Verbindungen der Formel 1A nach Anspruch 26 zur Herstellung der Verbindungen der Formel I, indem durch Anwendung von Wasserstoff in Gegenwart von Metall-Katalysatoren oder Natrium in Gegenwart von Ethanol eine Reduktion erfolgt.

## Claims

1. Compounds of the formula I 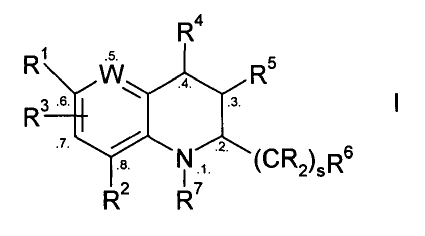 in which
W denotes CH or N,
R¹, R², R³, independently of one another, denote H, A, aryl,
heteroaryl, Hal, -(CY₂)ₙ-SA, -(CY₂)ₙ-SCF₃, -(CY₂)ₙ- SCN, -(CY₂)ₙ-CF₃, -(CY₂)ₙ-OCF₃, cycloalkyl, -SCH₃, -SCN, -CF₃, -OCF₃, -OA, -(CY₂)ₙ-OH, -(CY₂)ₙ- CO₂R, -(CY₂)ₙ-CN, -(CY₂)ₙ-Hal, (CH₂)ₙR, -(CY₂)ₙ-NR₂, (CY₂)ₙ-OR, (CY₂)ₙ-OCOA, -SCF₃, (CY₂)ₙ-CONR₂, -(CY₂)ₙ-NHCOA, -(CY₂)ₙ-NHSO₂A, SF₅, Si(CH₃)₃, CO-(CY₂)ₙ-CH₃, -(CY₂)ₙ-N-pyrrolidone, (CH₂)ₙNRCOOR, NRCOOR, NCO, CH₂(CH₂)ₙCOOR, NHCOOR, CH₂(CH₂)ₙOH, NR(CH₂)ₙOH, CH₂NH₂, (CH₂)ₙNR₂, CH(OH)R₂, CH₂NHCOR, (CH₂)ₙ-aryl, CH₂(CH₂)ₙ-heteroaryl, (CH₂)ₙR¹, NH(CH₂)ₙCOOR, CH₂(CH₂)ₙX(CH₂)ₙ-aryl, CH₂(CH₂)ₙX(CH₂)ₙ-heteroaryl, NH(CH₂)ₙCONR₂, XCONR(CH₂)ₙNR₂, N[(CH₂)ₙXCOOR]CO(CH₂)ₙ- aryl, N[(CH₂)ₙXR]CO(CH₂)ₙ-aryl, N[(CH₂)ₙXR]CO(CH₂)ₙX-aryl, N[(CH₂)ₙXR]SO₂(CH₂)ₙ-aryl, N[(CH₂)ₙNRCOOR]CO(CH₂)ₙ-aryl, N[(CH₂)ₙNR₂]CO(CH₂)ₙ-aryl, N[(CH₂)ₙNR₂]CO(CH₂)ₙNR-aryl, N[(CH₂)ₙNR₂]SO₂(CH₂)ₙ-aryl, N[(CH₂)ₙXR]CO(CH₂)ₙ-heteroaryl, N[(CH₂)ₙXR]CO(CH₂)ₙX-heteroaryl, N[(CH₂)ₙXR]SO₂(CH₂)ₙ-heteroaryl, N[(CH₂)ₙNRCOOR]CO(CH₂)ₙ-heteroaryl, N[(CH₂)ₙNR₂]CO(CH₂)ₙ-heteroaryl, N[(CH₂)ₙNR₂]CO(CH₂)ₙNR-heteroaryl, where non-adjacent CY₂ groups may also be replaced by X, R¹ and R³ together also denote -N-C(CF₃)=N-, -N-CR=N-, -N-N=N-,
Y denotes H, A, Hal, OR¹, N(R¹)₂, E-R¹
A denotes alkyl or cycloalkyl, in which one or more H atoms may be replaced by Hal and/or one or more non-adjacent CH₂ groups may be replaced by X,
Hal denotes F, Cl, Br or I
R denotes H or A, and geminal radicals R together also denote -(CH₂)₅-, -(CH₂)₄- or -(CH₂)ₙ-X-(CH₂)ₙ, or -(CH₂)ₙ-Z-(CH₂)ₙ,
R⁴ denotes H
R⁵ denotes -(CY₂)ₙ-E-(CY₂)ₙ-XR¹, (CH₂)₂OH, (CH₂)₃OH, (CH₂)₂NRR¹, (CH₂)₃NRR¹, (CH₂)₂CH(OH)CH₂OH, (CH₂)₂CH(OH)CH₂NRR¹, (CH₂)₂CH(OH)CH₂NR(CH₂)₂NRR¹, (CH₂)₂CH(OH)CH₂NR⁵(CH₂)₂NR, (CH₂)₂CH(OH)CH₂-E-R¹, Q or (CH₂)₂CH(OH)CH₂NR(CH₂)₂OR,
E denotes -NR¹SO₂-, -SO₂NR¹-, -CONR¹-, -NR¹CO-, -COO-, -OOC-, NR¹CONR¹-, -OCONR¹-, -NR¹COO-, -CSNR¹-, -NR¹CS-, -NR¹CSNR¹-, -SCONR¹-, -NR¹COS-, -OCSNR¹-, NR¹CSO-, -SCSNR¹-, -NR¹CSS or a single bond
X denotes O, S or NR¹,
Q denotes (CH₂)ₚHal, CHO, COR^{a}, (CH₂)ₚR^{a}, (CH₂)ₚOCOR^{a}, (CH₂)ₚXR¹, (CH₂)ₚNCOR¹, (CH₂)ₚN(R¹)₂, (CH₂)ₚOR¹, (CH₂)ₚOCON(R¹)₂, (CH₂)ₚOCOOR¹, (CH₂)ₚNHCON(R¹)₂, (CH₂)ₚNHCOOR¹, (CH₂)ₚCN, (CH₂)ₚCOOR¹, (CH₂)ₚ-E-(CH₂)ₚR¹, (CH₂)ₚ-E-(CH₂)ₚR^{a}, where non-adjacent CH₂ groups may also be replaced by X,
R^{a} denotes 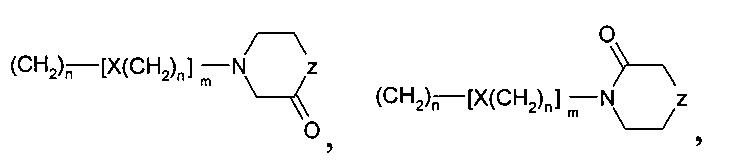 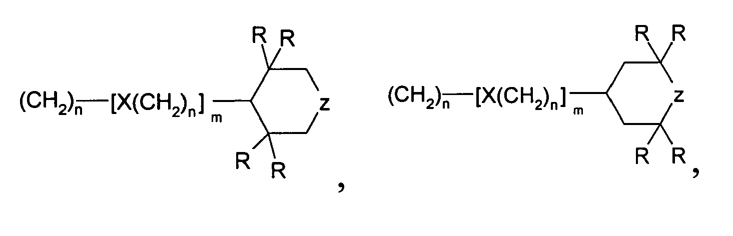 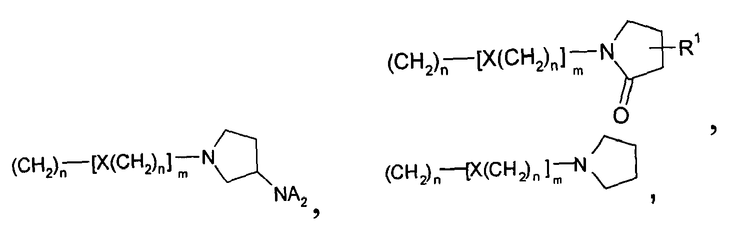 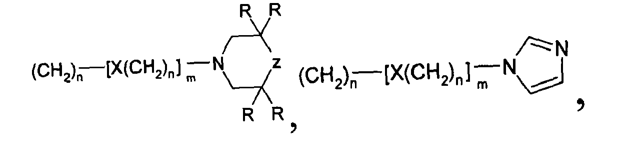 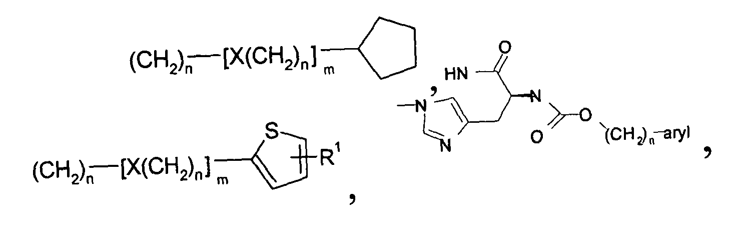 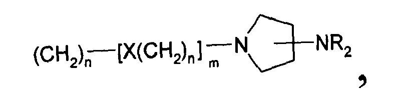 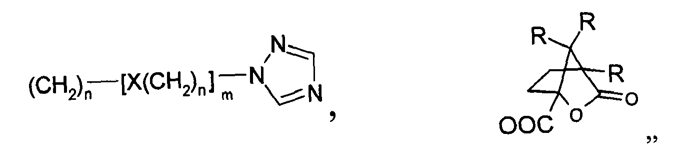 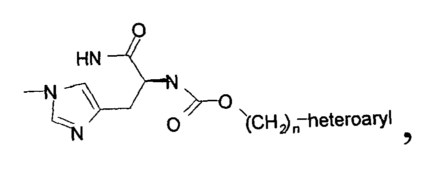 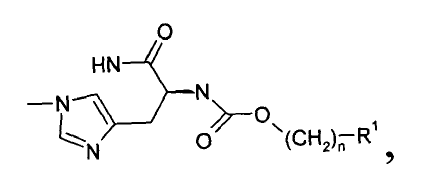 OR, NHR, NR₂, NR(CH₂)ₙ-aryl, NR(CH₂)ₙOR, COOR, N-pyrrolidone radical, OCOR, NR(CH₂)ₙNR₂, N[(CH₂)ₙNR₂]CO(CH₂)ₙ-aryl, N[(CH₂)ₙNHCOOR]CO-aryl, R¹, N[CH₂(CH₂)ₙOR]₂, NR(CH₂)ₙNCOOR, X(CH₂)ₙX(CH₂)ₙXR, NR(CH₂)ₙX(CH₂)ₙOH, NR(CH₂)ₙO(CH₂)ₙOH, (CH₂)ₙCOOR, O(CO)NR(CH₂)ₙOR, O(CO)(CH₂)ₙNR₂, NR(CH₂)ₙNR₂, N[(CH₂)ₙNR₂]CO(CH₂)ₙ-aryl, N[(CH₂)ₙXR]CO(CH₂)ₙ-aryl, N[(CH₂)ₙXR]CO(CH₂)ₙ-heteroaryl, N[(CH₂)ₙNR₂]CO(CH₂)ₙ-heteroaryl, N[(CH₂)ₙNR₂]CO(CH₂)ₙR¹, N(R)(CH₂)ₙN(R)COOR, XCOO(CH₂)ₙNR₂, OSO₂A, OSO₂CF₃, OSO₂Ar, OCONR₂, OCH₂(CH₂)ₙNR₂
Z denotes CH₂, X, CHCONH₂, CH(CH₂)ₙNR¹COOR¹, CHNR¹COOR¹, CHCON(R¹)₂, NCO, CH(CH₂)ₙCOOR¹, NCOOR¹, CH(CH₂)ₙOH, N(CH₂)ₙOH, CHNH₂, CH(CH₂)ₙN(R¹)₂, CH(CH₂)ₙN(R¹)₂, C(OH)R¹, CHNCOR¹, NCOR¹, N(CH₂)ₙ-aryl, N(CH₂)ₙ-heteroaryl, CHR¹, NR¹, CH(CH₂)ₙ-aryl, CH(CH₂)ₙ-heteroaryl, CH(CH₂)ₙR¹, N(CH₂)nCOOR¹, CH(CH₂)ₙX(CH₂)ₙR¹, CH(CH₂)ₙX(CH₂)ₙR^{a}, N(CH₂)ₙCON(R¹)₂, XCONR¹(CH₂)ₙN(R¹)₂, CO(CH₂)ₙR¹, CO(CH₂)ₙR¹, CO(CH₂)ₙXR¹, SO₂(CH₂)ₙR¹, O(CH₂)ₙN(R¹)₂, X(CH₂)ₙN(R¹)₂, NCO(CH₂)ₙN(R¹)₂, CHR^{a}, NR^{a},
R⁶ denotes aryl or heteroaryl, each of which is unsubstituted or mono- or polysubstituted by aryl or heteroaryl, which may be substituted by Hal, NO₂, CN, A, OR, OCOR, COR, NR₂, CF₃, OCF₃, OCH(CF₃)₂, or by Hal, NO₂, CN, OR, A, -(CY₂)ₙ-OR, -OCOR, -(CY₂)ₙ-CO₂R, -(CY₂)ₙ-CN, -NCOR, -COR or -(CY₂)ₙ-NR₂,
R⁷ denotes (C=O)-R, (C=O)-NR₂, (C=O)-OR, H or A, in which R⁵ and R⁷ together may also denote -(CH₂)ₙ-,
m denotes 0, 1 or 2
n denotes 0, 1, 2, 3, 4, 5, 6 or 7,
and
p denotes 0, 1, 2, 3, 4, or 5, preferably 2 or 3
s denotes 0, 1, 2, 3, 4, 5, 6 or 7, preferably 0,
and solvates, tautomers, salts and stereoisomers thereof, including mixtures thereof in all ratios.

2. Compounds according to Claim 1 in which
R¹ denotes A, CF₃, OCF₃, SA, SCN, CH₂CN, -OCOA, Hal, SCF₃, t-butyl, -CH(CH₃)CH₂CH₃, isopropyl, ethyl or methyl.

3. Compounds according to Claim 1 or 2 in which
R² denotes H.

4. Compounds according to one or more of Claims 1-3 in which
R³ denotes H.

5. Compounds according to one or more of Claims 1-4 in which
W denotes CH.

6. Compounds according to one or more of Claims 1-5 in which
R⁶ denotes phenyl, 2-, 3- or 4-pyridyl, pyrimidyl, furyl or thienyl, each of which is unsubstituted or mono- or poly- substituted by Hal, CN, NO₂, OH, CF₃, OCH(CF₃)₂, OCOCH₃ or A.

7. Compounds according to one or more of Claims 1-6 in which R⁶ denotes one of the following groups:

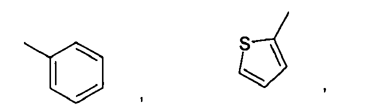

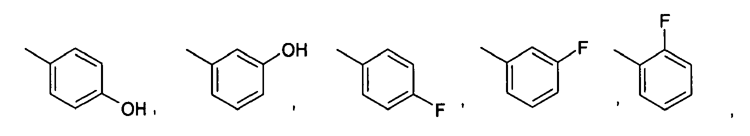

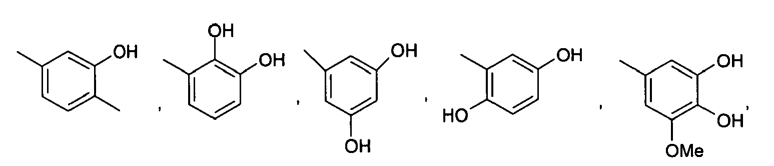

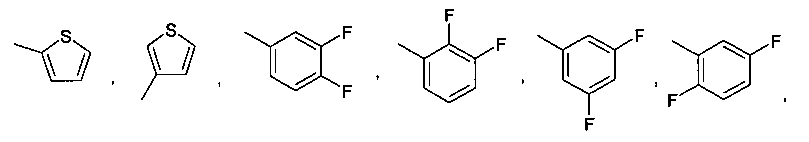

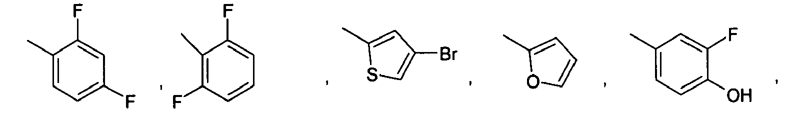

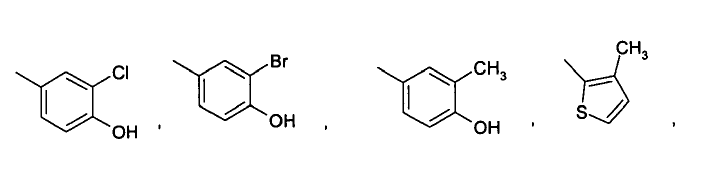

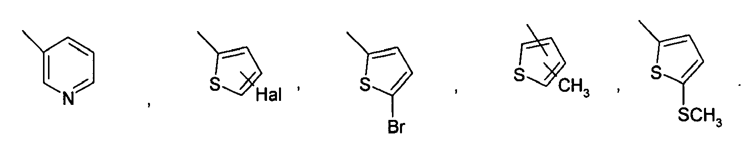

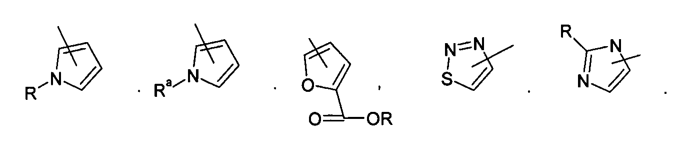

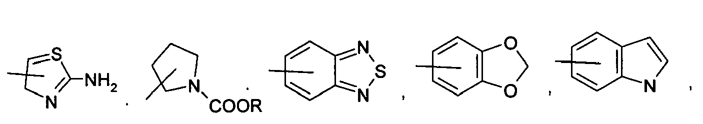

8. Compounds according to one or more of Claims 1-7 in which
R⁷ denotes H.

9. Compounds according to Claim 1 having the sub-formulae IA to IB: 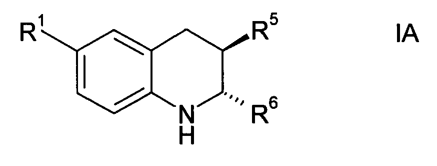 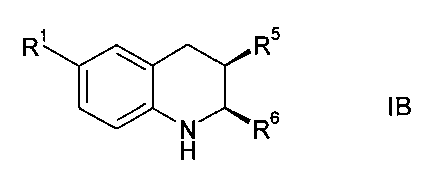 in which R¹, R⁵ and R⁶ have the meaning indicated in Claim 1, and the racemate thereof or other mixtures of the enantiomers.

10. Compounds according to Claim 1 having the sub-formulae I1 to I27:

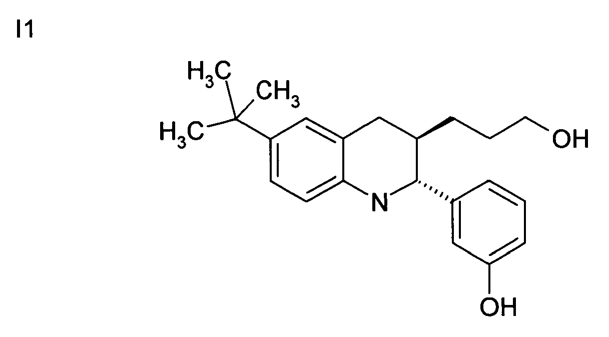

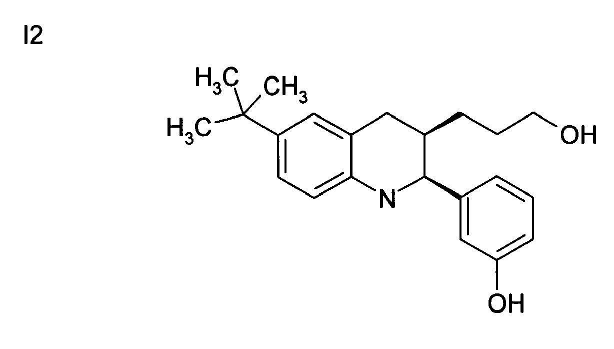

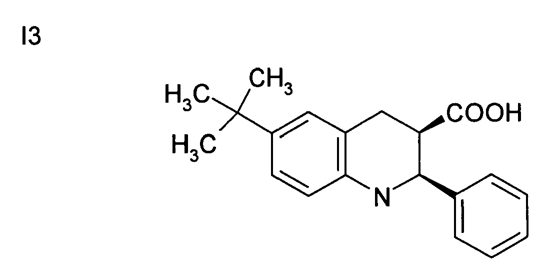

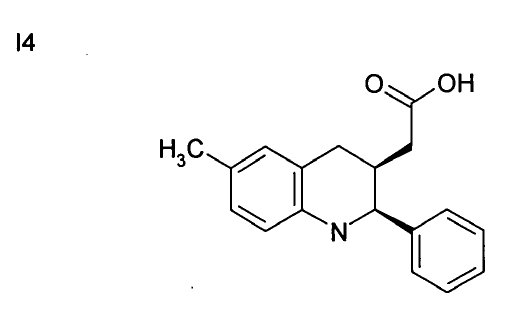

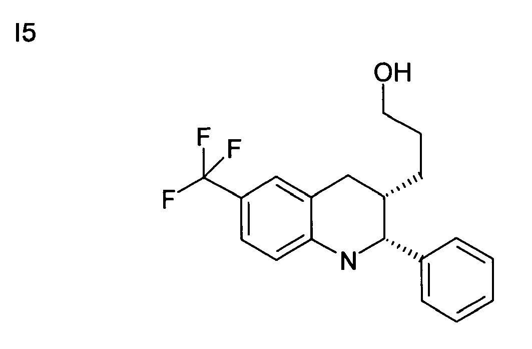

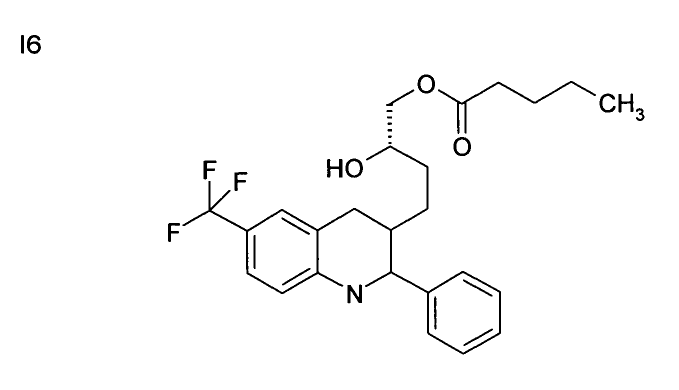

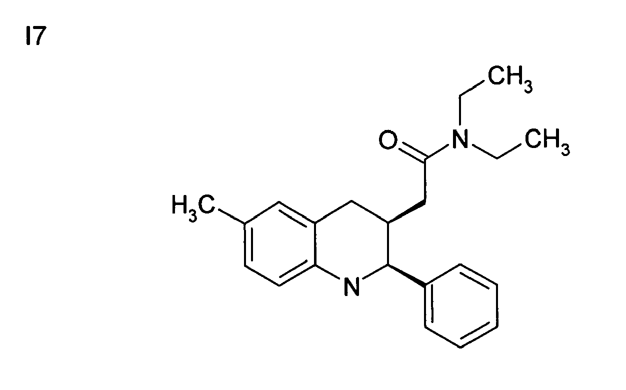

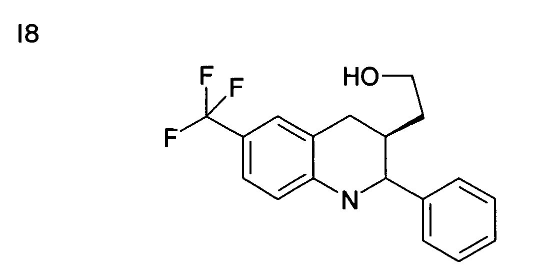

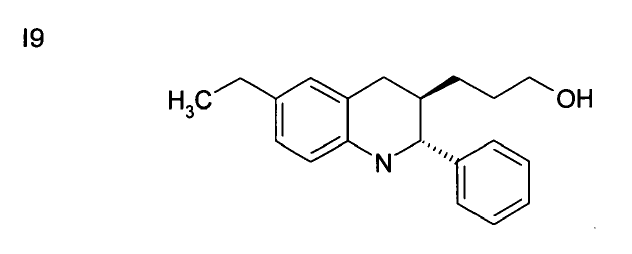

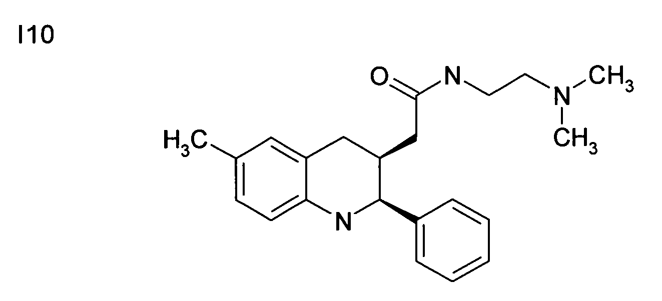

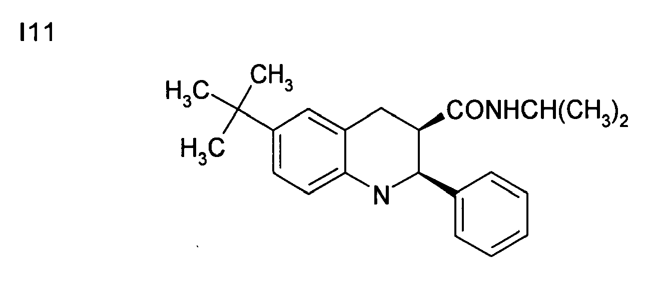

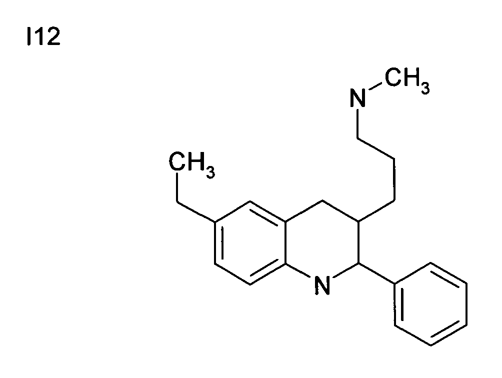

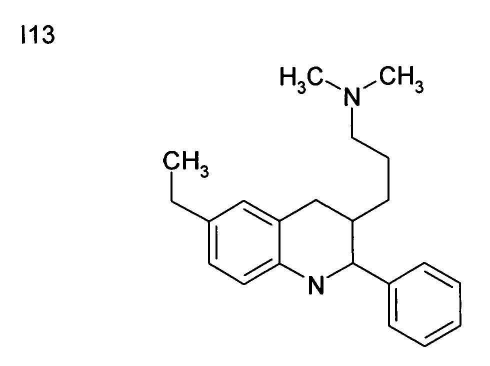

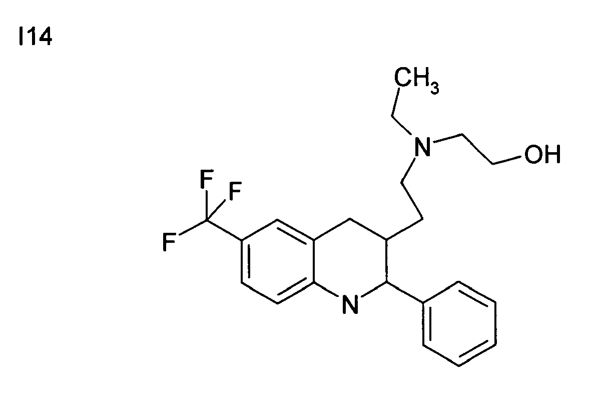

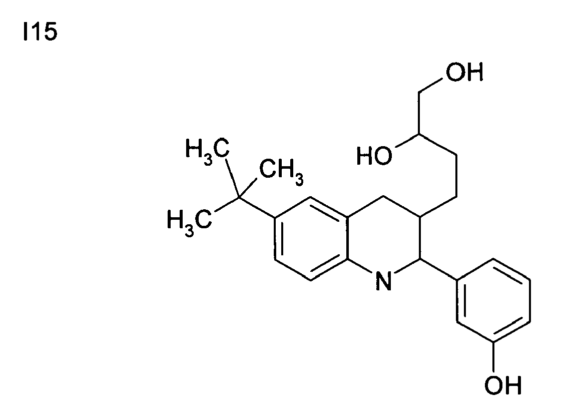

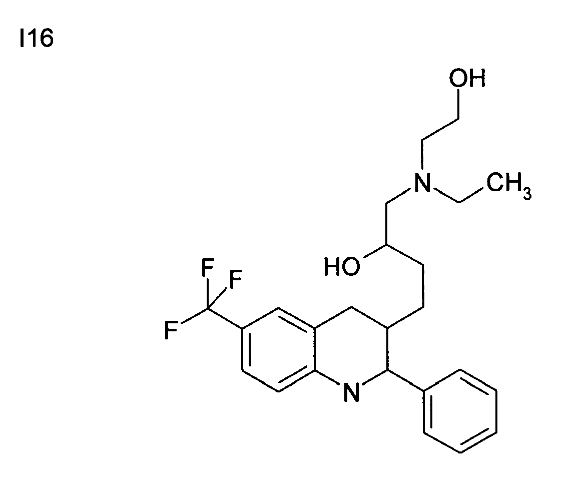

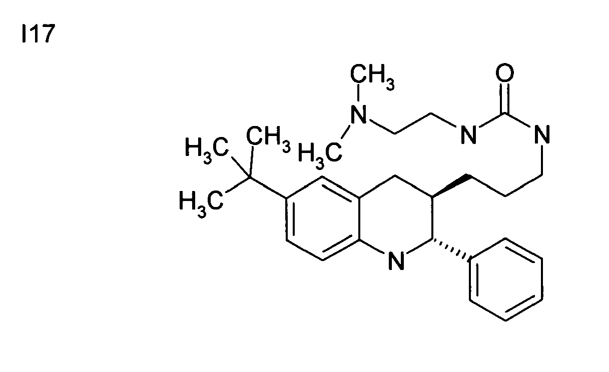

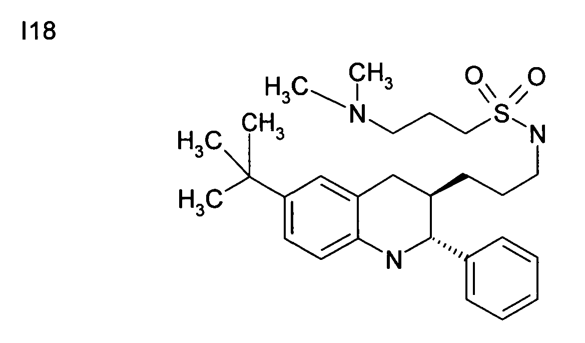

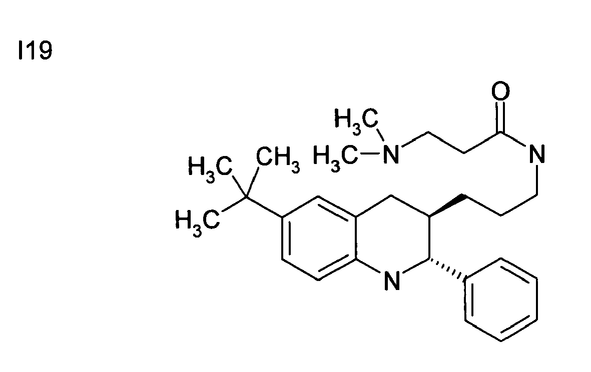

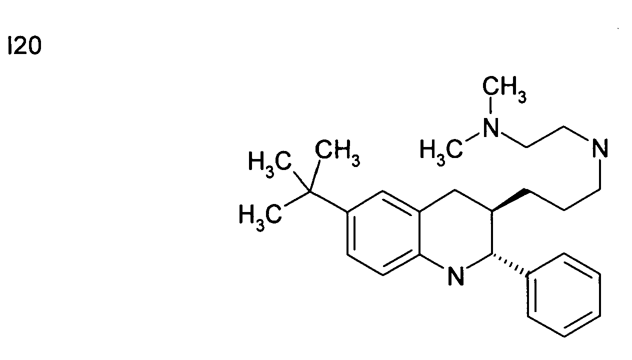

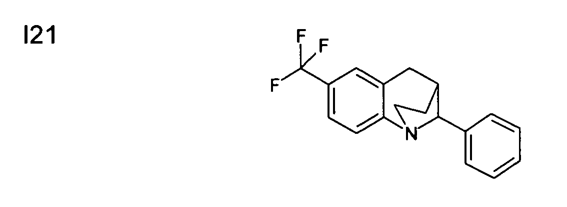

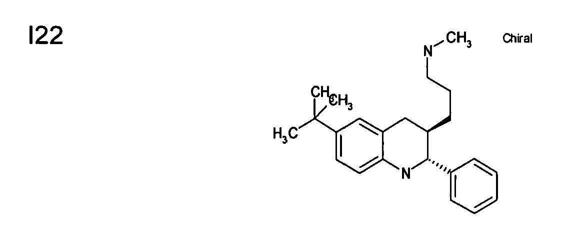

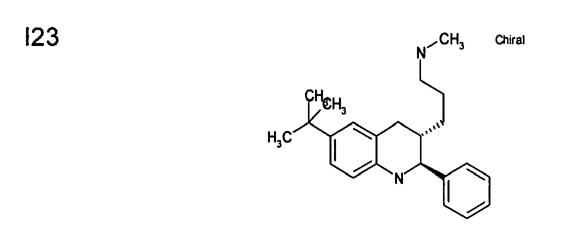

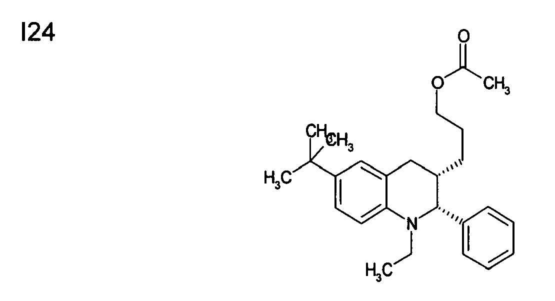

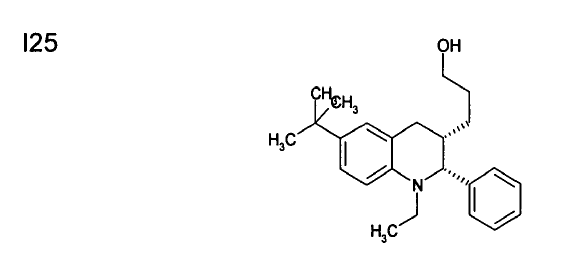

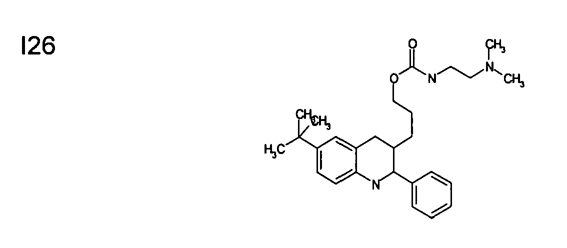

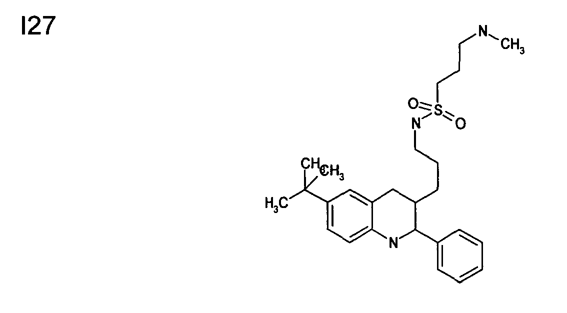

11. Compounds of the formula I according to Claims 1-10 in which S denotes O.

12. Process for the preparation of compounds of the formula I according to Claims 1-11 and salts, solvates, tautomers and stereoisomers thereof, **characterised in that**
a compound of the formula II 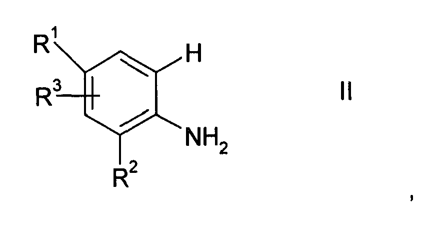 in which R¹, R² and R³ have the meanings indicated in Claim 1,
is reacted with a compound of the formula III 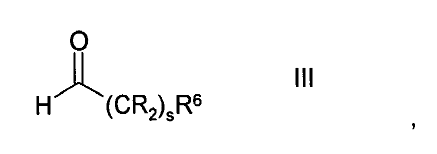 in which
R⁶ has the meaning indicated in Claim 1,
and
with a compound of the formula IV 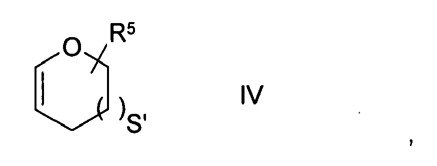 in which S' denotes 0, 1 or 2, or correspondingly substituted compounds, and the resultant tetrahydroquinolines are converted into the corresponding quinolines using acid, these are then reduced to the compounds of the formula I, and the radical R⁵ is optionally converted into the further radicals R⁵ by conventional methods,
and, if desired, a radical R⁷ which denotes H is converted into a radical R⁷ which has a meaning other than H,
and/or, if desired,
a base or acid of the formula I is converted into one of its salts.

13. Process according to Claim 12, **characterised in that** the reaction is carried out in the presence of a protonic acid or Lewis acid.

14. Process according to Claim 12 or 13, **characterised in that** the reaction is carried out in the presence of trifluoroacetic acid, hexafluoroisopropanol, bismuth(III) chloride, ytterbium(III) triflate, scandium(III) triflate or cerium(IV) ammonium nitrate.

15. Medicament comprising at least one compound of the formula I according to Claims 1 to 11 and/or salts, solvates, tautomers and stereoisomers thereof, including mixtures thereof in all ratios, and optionally excipients and/or adjuvants.

16. Mixture comprising one or more compounds of the formula I according to Claims 1-11 and an amount of one or more compounds of the formula V 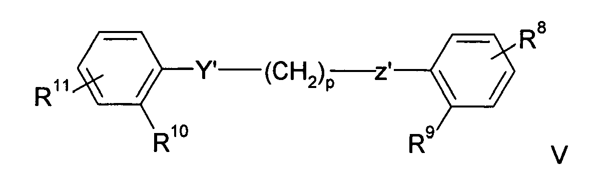 in which
Y' and Z' each, independently of one another, denote O or N, R⁹ and R¹⁰ each, independently of one another, denote H, OH, halogen, OC₁₋₁₀-alkyl, OCF₃, NO₂ or NH₂, n denotes an integer between 2 and 6, each inclusive, and R⁸ and R¹¹ are each, independently of one another, in the meta- or para-position and are selected from the group:

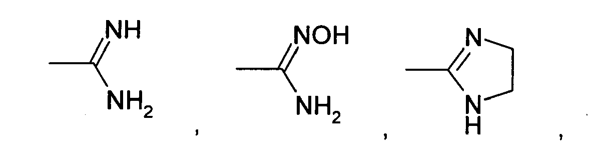

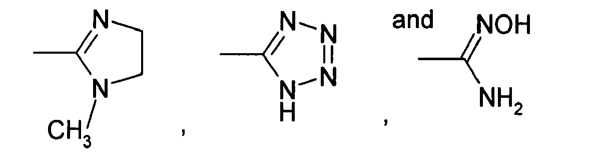

17. Mixture according to Claim 16, where the compound of the formula V used is pentamidine or salts thereof.

18. Use of compounds according to Claims 1 to 11 and salts, solvates, tautomers and stereoisomers thereof, including mixtures thereof in all ratios, or the mixture according to Claim 16 for the preparation of a medicament for the treatment of diseases which can be influenced by the inhibition, regulation and/or modulation of the mitotic motor protein Eg5.

19. Use of compounds according to Claims 1 to 11 or the mixture according to Claim 16 for the preparation of a medicament for the treatment and prophylaxis of cancer diseases.

20. Use according to Claim 19, where the cancer diseases are accompanied by a tumour from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx and/or of the lung.

21. Use according to Claim 20, where the tumour originates from the group monocytic leukaemia, lung adenocarcinoma, small-cell lung carcinomas, pancreatic cancer, glioblastomas and breast carcinoma and colon carcinoma.

22. Use according to Claim 19, where the cancer disease to be treated is a tumour of the blood and immune system.

23. Use according to Claim 22, where the tumour originates from the group of acute myeloid leukaemia, chronic myeloid leukaemia, acute lymphatic leukaemia and/or chronic lymphatic leukaemia.

24. Use of compounds of the formula I according to Claims 1 to 11 and/or physiologically acceptable salts and solvates thereof for the preparation of a medicament for the treatment of tumours in combination with a therapeutically effective amount of one or more compounds of the formula V 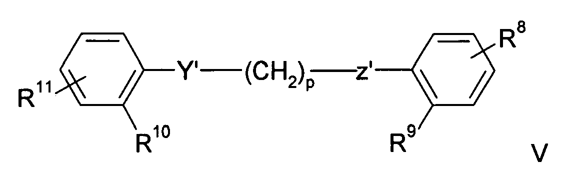 in which
Y' and Z' each, independently of one another, denote O or N, R⁹ and R¹⁰ each, independently of one another, denote H, OH, halogen, OC₁₋₁₀-alkyl, OCF₃, NO₂ or NH₂, n denotes an integer between 2 and 6, each inclusive, and R⁸ and R¹¹ are each, independently of one another, in the meta- or para-position and are selected from the group: 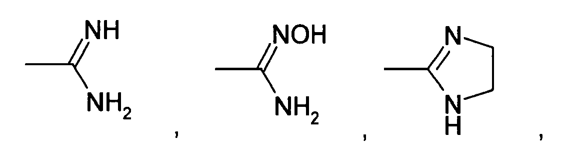 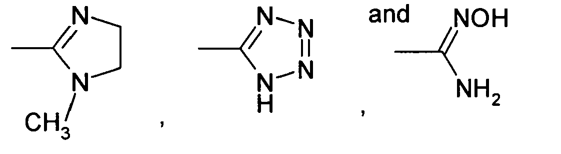 where
the compounds of the formula I and the compounds of the formula V are administered simultaneously or within 14 days of one another in amounts which are sufficient to inhibit the growth of a tumour or of other hyperproliferative cells.

25. Use according to Claim 24, where the compound of the formula V used is pentamidine or salts thereof.

26. Intermediate compounds of the formula 1A 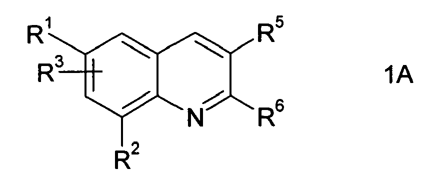 in which R¹, R², R³, R⁵ and R⁶ have the meaning indicated in Claim 1.

27. Use of the compounds of the formula 1A according to Claim 26 for the preparation of the compounds of the formula I by carrying out a reduction using hydrogen in the presence of metal catalysts or sodium in the presence of ethanol.

## Revendications

1. Composés de formule I 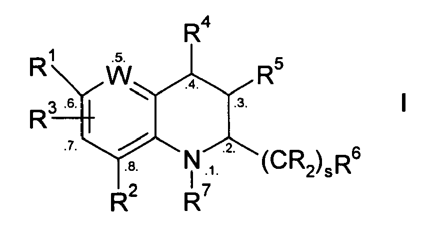 dans laquelle
W désigne CH ou N,
R¹, R², R³, indépendamment les uns des autres, désignent H, A, aryle, hétéroaryle, Hal, -(CY₂)ₙ-SA, -(CY₂)ₙ-SCF₃, -(CY₂)ₙ-SCN, -(CY₂)ₙ-CF₃, -(CY₂)ₙ-OCF₃, cycloalkyle, -SCH₃, -SCN, -CF₃, -OCF₃, -OA, -(CY₂)ₙ-OH, -(CY₂)ₙ-CO₂R, -(CY₂)ₙ-CN, -(CY₂)ₙ-Hal, (CH₂)ₙR, -(CY₂)ₙ-NR₂, (CY₂)ₙ-OR, (CY₂)ₙ-OCOA, -SCF₃, (CY₂)ₙ-CONR₂, -(CY₂)ₙ-NHCOA, -(CY₂)ₙ- NHSO₂A, SF₅, Si(CH₃)₃, CO-(CY₂)ₙ-CH₃, -(CY₂)ₙ-N- pyrrolidone, (CH₂)ₙNRCOOR, NRCOOR, NCO, CH₂(CH₂)ₙCOOR, NHCOOR, CH₂(CH₂)ₙOH, NR(CH₂)ₙOH, CH₂NH₂, (CH₂)ₙNR₂, CH(OH)R₂, CH₂NHCOR, (CH₂)ₙ-aryle, CH₂(CH₂)ₙ-hétéroaryle, (CH₂)ₙR¹, NH(CH₂)ₙCOOR, CH₂(CH₂)ₙX(CH₂)ₙ- aryle, CH₂(CH₂)ₙX(CH₂)ₙ-hétéroaryle, NH(CH₂)ₙCONR₂, XCONR(CH₂)ₙNR₂, N[(CH₂)ₙXCOOR]CO(CH₂)ₙ-aryle, N[(CH₂)ₙXR]CO(CH₂)ₙ-aryle, N[(CH₂)ₙXR]CO(CH₂)ₙX-aryle, N[(CH₂)ₙXR]SO₂(CH₂)ₙ-aryle, N[(CH₂)ₙNRCOOR]CO(CH₂)ₙ-aryle, N[(CH₂)ₙNR₂]CO(CH₂)ₙ-aryle, N[(CH₂)ₙNR₂]CO(CH₂)ₙNR-aryle, N[(CH₂)ₙNR₂]SO₂(CH₂)ₙ-aryle, N[(CH₂)ₙXR]CO(CH₂)ₙ-hétéroaryle, N[(CH₂)ₙXR]CO(CH₂)ₙX-hétéroaryle, N[(CH₂)ₙXR]SO₂(CH₂)ₙ-hétéroaryle, N[(CH₂)ₙNRCOOR]CO(CH₂)ₙ-hétéroaryle, N[(CH₂)ₙNR₂]CO(CH₂)ₙ-hétéroaryle, N[(CH₂)ₙNR₂]CO(CH₂)ₙNR-hétéroaryle, où des groupements CY₂ non adjacents peuvent aussi être remplacés par X, R¹ et R³ désignent aussi ensemble -N-C(CF₃)=N-, -N-CR=N-, -N-N=N-,
Y désigne H, A, Hal, OR¹, N(R¹)₂, E-R¹
A désigne alkyle ou cycloalkyle, dans lesquels un ou plusieurs atomes de H peuvent être remplacés par Hal et/ou un ou plusieurs groupements CH₂ non adjacents peuvent être remplacés par X,
Hal désigne F, CI, Br ou I
R désigne H ou A, et les radicaux géminés R désignent aussi ensemble -(CH₂)₅-, -(CH₂)₄- ou -(CH₂)ₙ-X-(CH₂)ₙ, ou -(CH₂)ₙ-Z-(CH₂)ₙ,
R⁴ désigne H
R⁵ désigne -(CY₂)ₙ-E-(CY₂)ₙ-XR¹, (CH₂)₂OH, (CH₂)₃OH, (CH₂)₂NRR¹, (CH₂)₃NRR¹, (CH₂)₂CH(OH)CH₂OH, (CH₂)₂CH(OH)CH₂NRR¹, (CH₂)₂CH(OH)CH₂NR(CH₂)₂NRR¹, (CH₂)₂CH(OH)CH₂NR⁵(CH₂)₂NR, (CH₂)₂CH(OH)CH₂-E-R¹, Q ou (CH₂)₂CH(OH)CH₂NR(CH₂)₂OR;
E désigne -NR¹SO₂-, -SO₂NR¹-, -CONR¹-, -NR¹CO-, -COO-, -OOC-, NR¹CONR¹-, -OCONR¹-, -NR¹COO-, -CSNR¹-, -NR¹CS-, -NR¹CSNR¹-, -SCONR¹-, -NR¹COS-, -OCSNR¹-, NR¹CSO-, -SCSNR¹-, -NR¹CSS ou une liaison simple
X désigne O, S ou NR¹,
Q désigne (CH₂)ₚHal, CHO, COR^{a}, (CH₂)ₚR^{a}, (CH₂)ₚOCOR^{a}, (CH₂)ₚXR¹, (CH₂)ₚNCOR¹, (CH₂)ₚN(R¹)₂, (CH₂)ₚOR¹, (CH₂)ₚOCON(R¹)₂, (CH₂)ₚOCOOR¹, (CH₂)ₚNHCON(R¹)₂, (CH₂)ₚNHCOOR¹, (CH₂)ₚCN, (CH₂)ₚCOOR¹, (CH₂)ₚ-E-(CH₂)ₚR¹, (CH₂)ₚ-E-(CH₂)ₚR^{a}, où les groupements CH₂ non adjacents peuvent aussi être remplacés par X,
R^{a} désigne 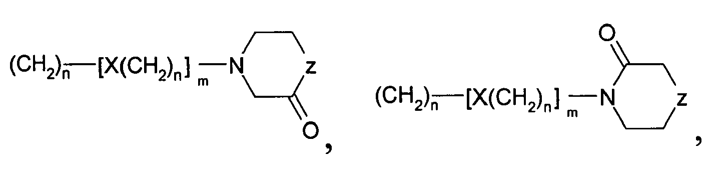 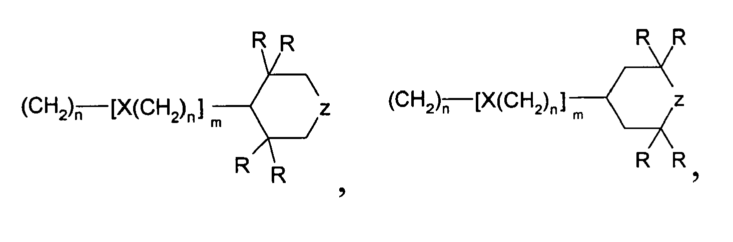 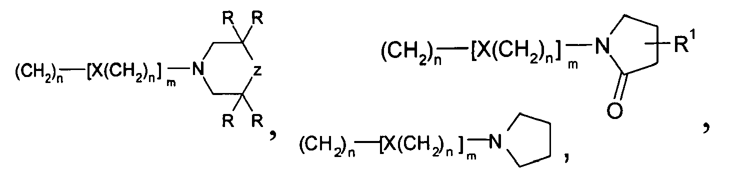 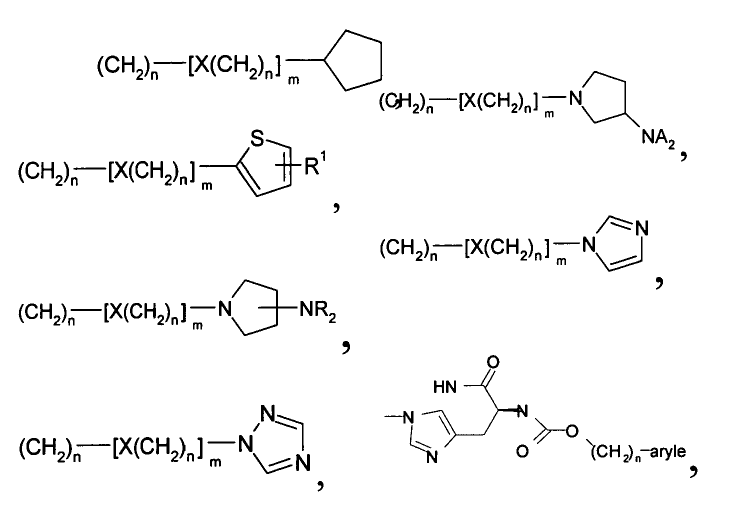 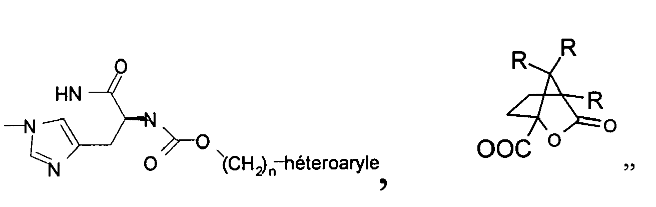 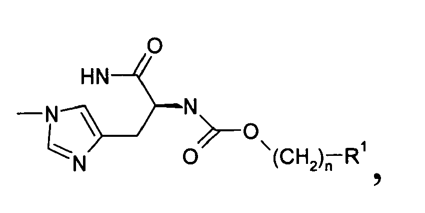 OR, NHR, NR₂, NR(CH₂)ₙ-aryle, NR(CH₂)ₙOR, COOR, un radical N-pyrrolidone, OCOR, NR(CH₂)ₙNR₂, N[(CH₂)ₙNR₂]CO(CH₂)ₙ-aryle, N[(CH₂)ₙNHCOOR]CO- aryle, R¹, N[CH₂(CH₂)ₙOR]₂, NR(CH₂)ₙNCOOR, X(CH₂)ₙX(CH₂)ₙXR, NR(CH₂)ₙX(CH₂)ₙOH, NR(CH₂)ₙO(CH₂)ₙOH, (CH₂)ₙCOOR, O(CO)NR(CH₂)ₙOR, O(CO)(CH₂)ₙNR₂, NR(CH₂)ₙNR₂, N[(CH₂)ₙNR₂]CO(CH₂)ₙ-aryle, N[(CH₂)ₙXR]CO(CH₂)ₙ- aryle, N[(CH₂)ₙXR]CO(CH₂)ₙ-hétéroaryle, N[(CH₂)ₙNR₂]CO(CH₂)ₙ-hétéroaryle, N[(CH₂)ₙNR₂]CO(CH₂)ₙR¹, N(R)(CH₂)ₙN(R)COOR, XCOO(CH₂)ₙNR₂, OSO₂A, OSO₂CF₃, OSO₂Ar, OCONR₂, OCH₂(CH₂)ₙNR₂
Z désigne CH₂, X, CHCONH₂, CH(CH₂)ₙNR¹COOR¹, CHNR¹COOR¹, CHCON(R¹)₂, NCO, CH(CH₂)ₙCOOR¹, NCOOR¹, CH(CH₂)ₙOH, N(CH₂)ₙOH, CHNH₂, CH(CH₂)ₙN(R¹)₂, CH(CH₂)ₙN(R¹)₂, C(OH)R¹, CHNCOR¹, NCOR¹, N(CH₂)ₙ-aryle, N(CH₂)ₙ-hétéroaryle, CHR¹, NR¹, CH(CH₂)ₙ-aryle, CH(CH₂)ₙ-hétéroaryle, CH(CH₂)ₙR¹, N(CH₂)ₙCOOR¹, CH(CH₂)ₙX(CH₂)ₙR¹, CH(CH₂)ₙX(CH₂)ₙR^{a}, N(CH₂)ₙCON(R¹)₂, XCONR¹(CH₂)ₙN(R¹)₂, CO(CH₂)ₙR¹, CO(CH₂)ₙR¹, CO(CH₂)ₙXR¹, SO₂(CH₂)ₙR¹, O(CH₂)ₙN(R¹)₂, X(CH₂)ₙN(R¹)₂, NCO(CH₂)ₙN(R¹)₂, CHR^{a}, NR^{a},
R⁶ désigne aryle ou hétéroaryle, chacun d'entre eux étant non substitué ou mono- ou polysubstitué par aryle ou hétéroaryle, pouvant être substitué par Hal, NO₂, CN, A, OR, OCOR, COR, NR₂, CF₃, OCF₃, OCH(CF₃)₂, ou par Hal, NO₂, CN, OR, A, -(CY₂)ₙ-OR, -OCOR, -(CY₂)ₙ-CO₂R, -(CY₂)ₙ-CN, -NCOR, -COR ou -(CY₂)ₙ-NR₂,
R⁷ désigne (C=O)-R, (C=O)-NR₂, (C=O)-OR, H ou A, dans lesquels R⁵ et R⁷ peuvent aussi désigner ensemble -(CH₂)ₙ-,
m désigne 0, 1 ou 2
n désigne 0, 1, 2, 3, 4, 5, 6 ou 7,
et
p désigne 0, 1, 2, 3, 4, ou 5, préférablement 2 ou 3
s désigne 0, 1, 2, 3, 4, 5, 6 ou 7, préférablement 0,
et des solvats, tautomères, sels et stéréoisomères de ceux-ci, y compris des mélanges de ceux-ci en toutes proportions.

2. Composés selon la revendication 1, dans lesquels
R¹ désigne A, CF₃, OCF₃, SA, SCN, CH₂CN, -OCOA, Hal, SCF₃, t-butyle, -CH(CH₃)CH₂CH₃, isopropyle, éthyle ou méthyle.

3. Composés selon la revendication 1 ou 2, dans lesquels
R² désigne H.

4. Composés selon l'une ou plusieurs des revendications 1-3, dans lesquels
R³ désigne H.

5. Composés selon l'une ou plusieurs des revendications 1-4, dans lesquels
W désigne CH.

6. Composés selon l'une ou plusieurs des revendications 1-5, dans lesquels
R⁶ désigne phényle, 2-, 3- ou 4-pyridyle, pyrimidyle, furyle ou thiényle, chacun d'entre eux étant non substitué ou mono- ou polysubstitué par Hal, CN, NO₂, OH, CF₃, OCH(CF₃)₂, OCOCH₃ ou A.

7. Composés selon l'une ou plusieurs des revendications 1-6, dans lesquels
R⁶ désigne l'un des groupements suivants:

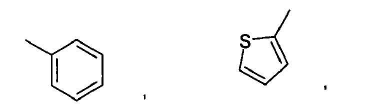

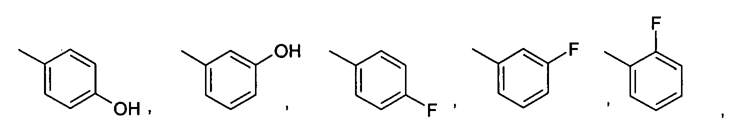

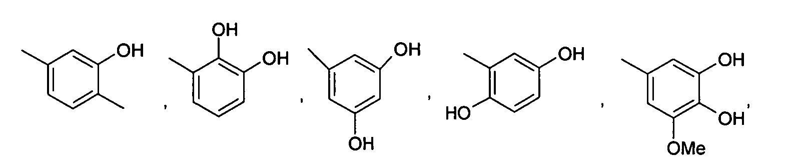

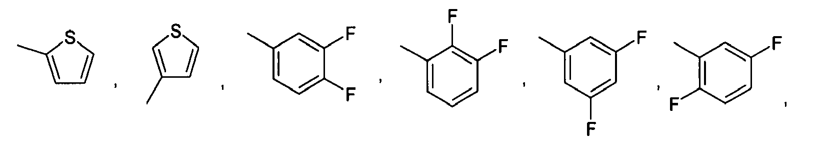

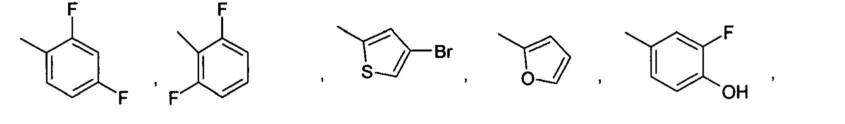

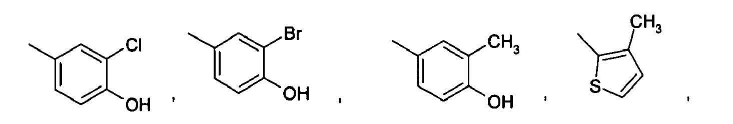

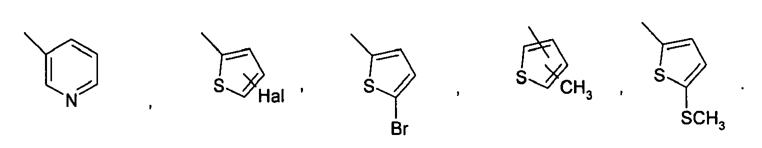

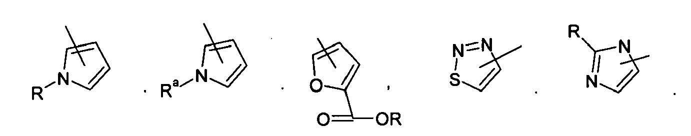

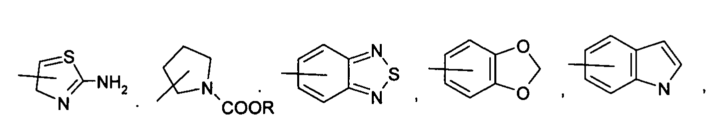

8. Composés selon l'une ou plusieurs des revendications 1-7, dans lesquels
R⁷ désigne H.

9. Composés selon la revendication 1, répondant aux sous-formules IA à IB: 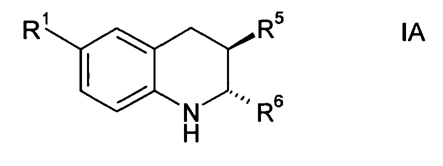 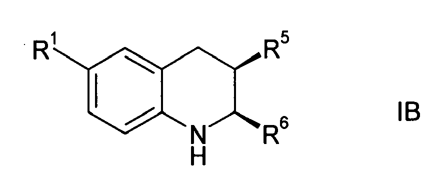 dans lesquelles R¹, R⁵ et R⁶ ont la signification indiquée dans la revendication 1, et le racémate de ceux-ci ou d'autres mélanges des énantiomères.

10. Composés selon la revendication 1, répondant aux sous-formules I1 à I27:

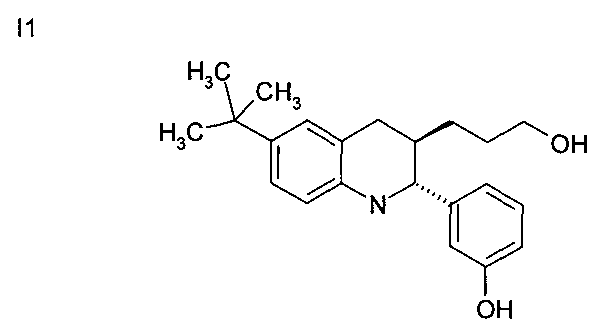

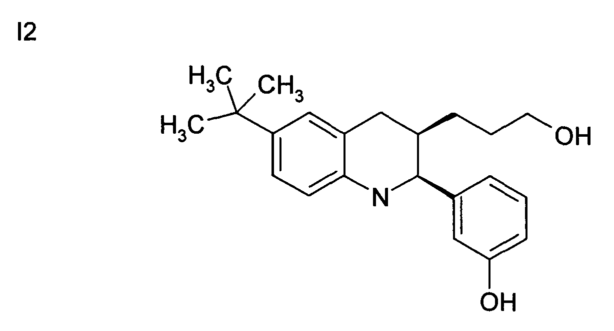

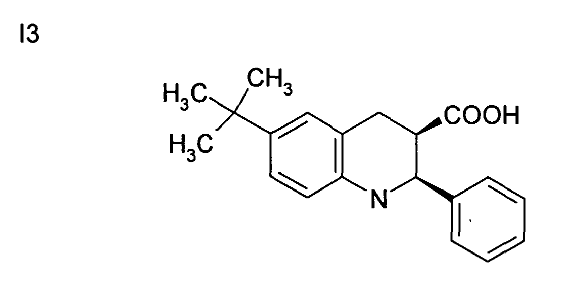

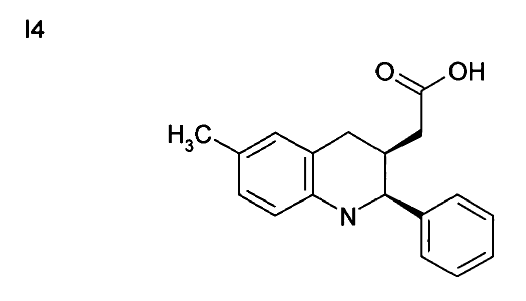

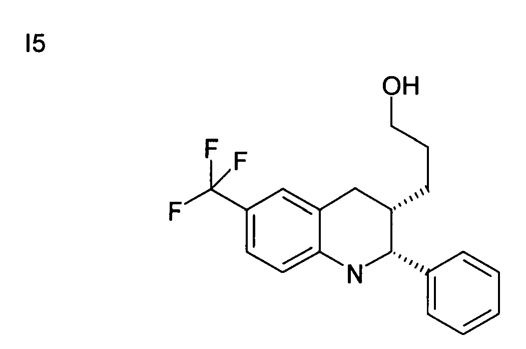

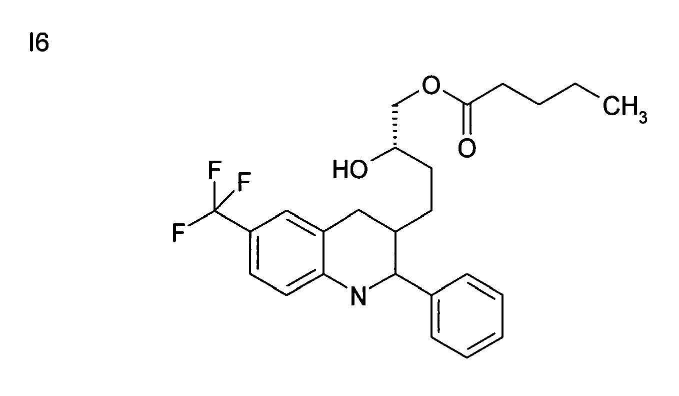

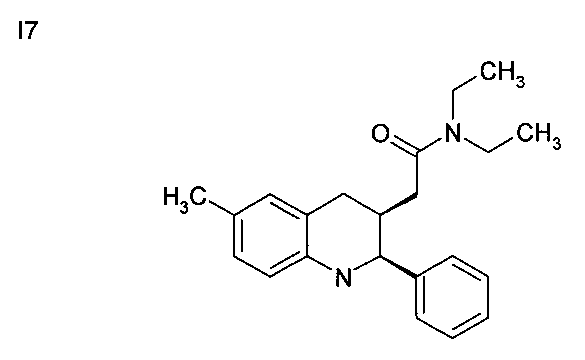

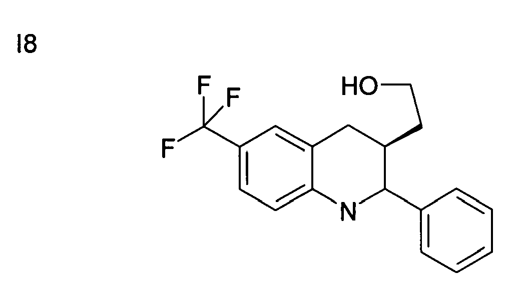

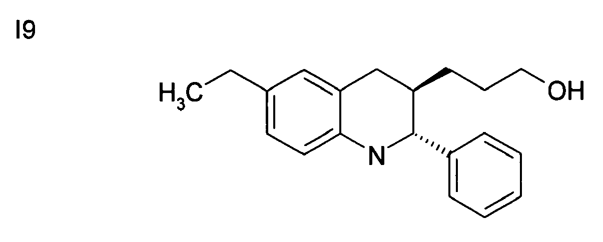

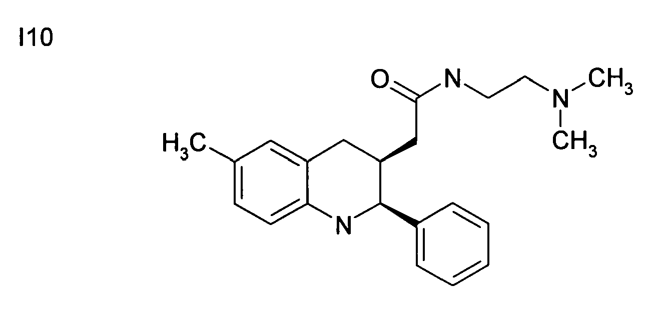

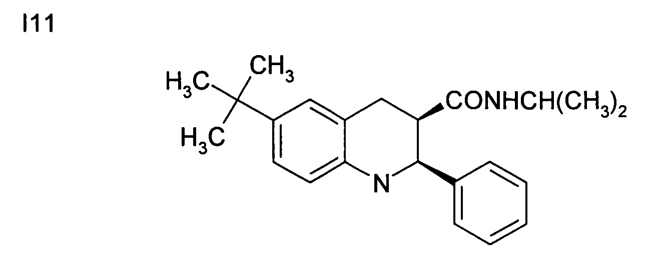

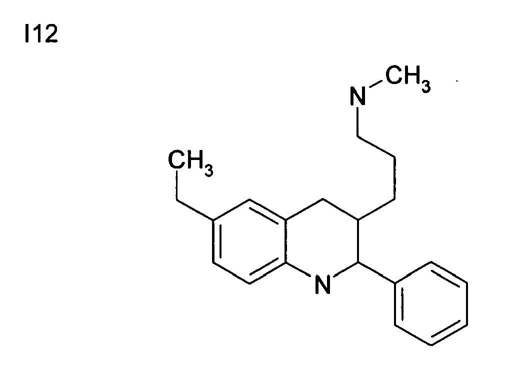

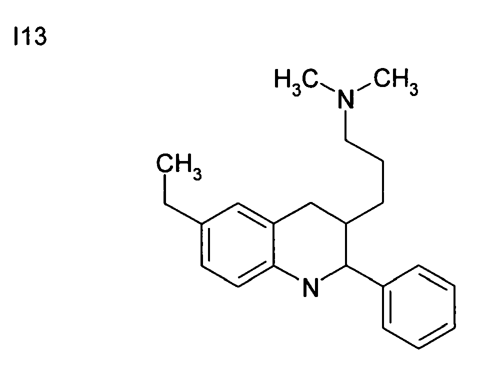

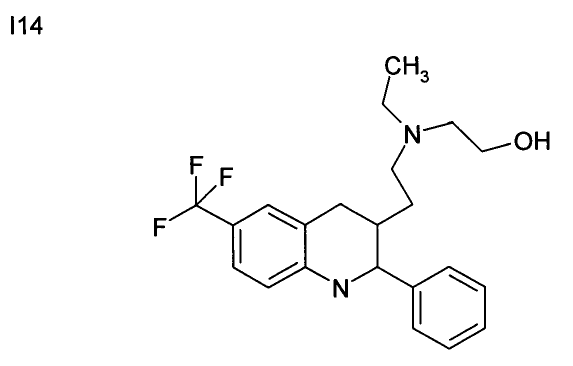

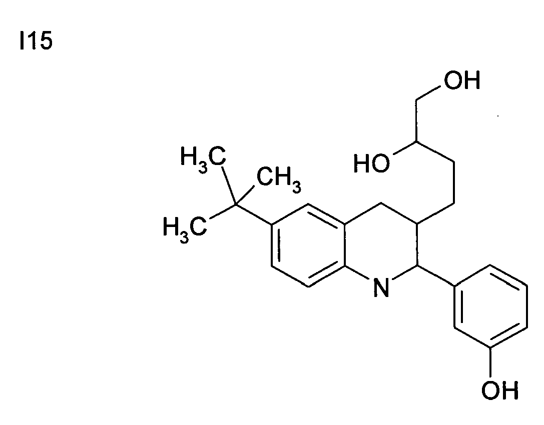

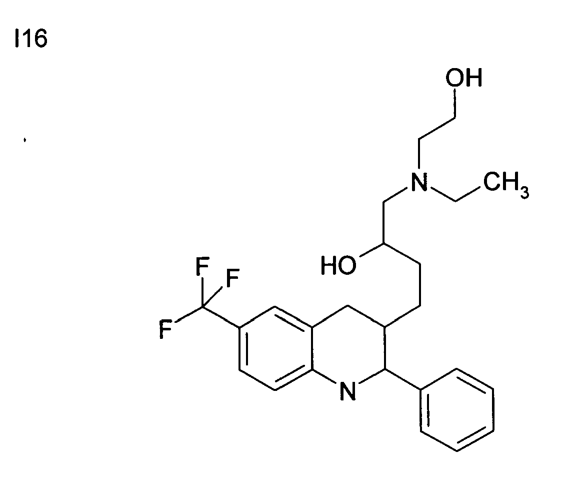

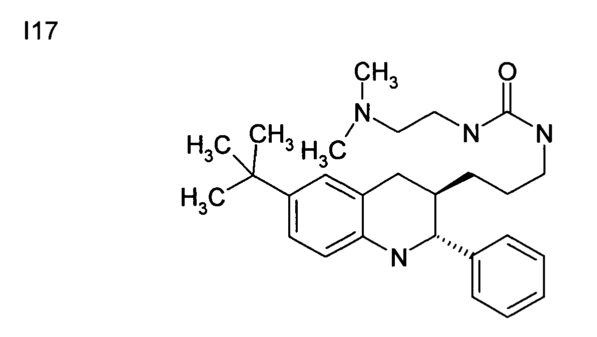

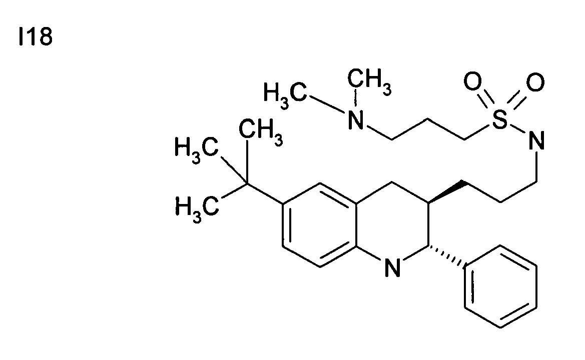

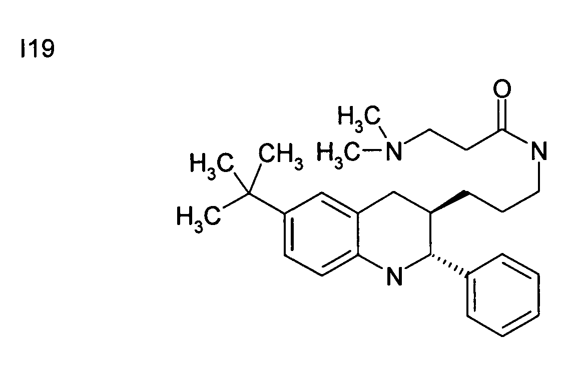

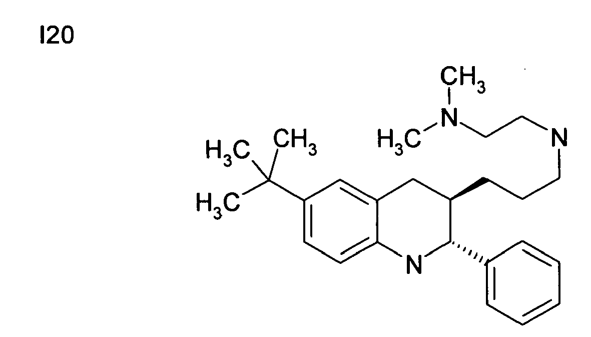

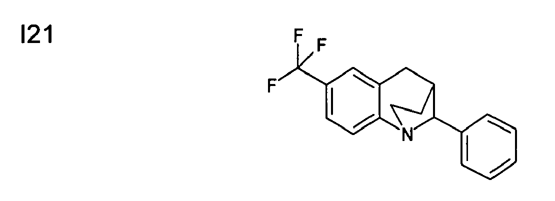

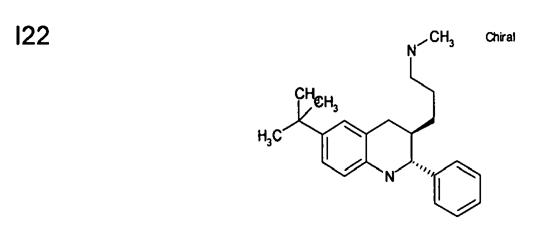

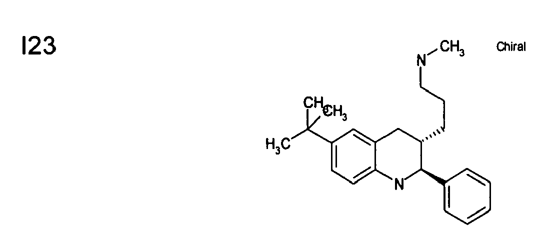

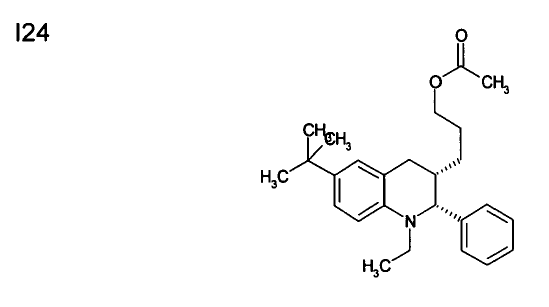

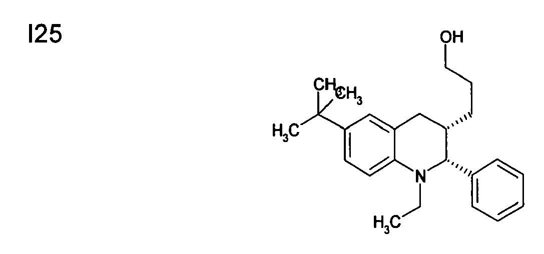

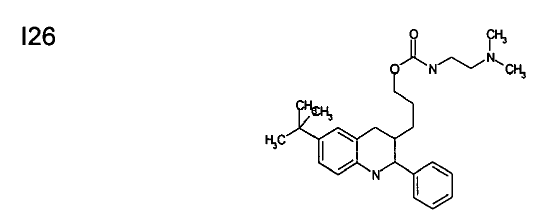

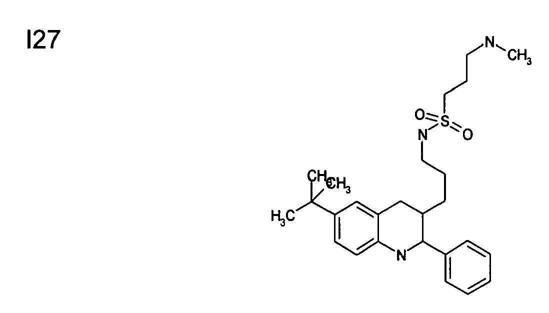

11. Composés de formule I selon les revendications 1-10, dans lesquels S désigne O.

12. Procédé de préparation de composés de formule I selon les revendications 1-11 et de sels, solvats, tautomères et stéréoisomères de ceux-ci, **caractérisé en ce que**
un composé de formule II 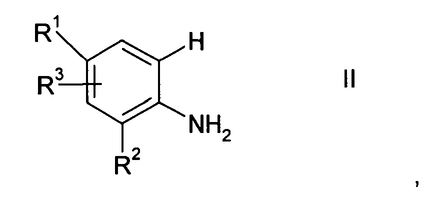 dans laquelle R¹, R² et R³ ont les significations indiquées dans la revendication 1,
est réagi avec un composé de formule III 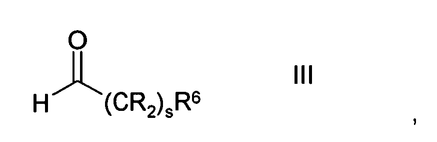 dans laquelle
R⁶ a la signification indiquée dans la revendication 1,
et
avec un composé de formule IV 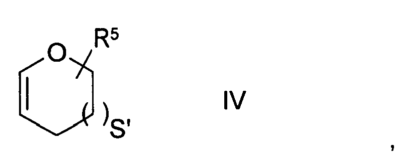 dans laquelle S' désigne 0, 1 ou 2, ou des composés substitués de manière correspondante, et les tétrahydroquinoléines résultantes sont converties en quinoléines correspondantes à l'aide d'acide, celles-ci sont ensuite réduites en composés de formule I, et le radical R⁵ est éventuellement converti en radicaux R⁵ supplémentaires par des méthodes classiques,
et, si on le souhaite, un radical R⁷ qui désigne H est converti en radical R⁷ ayant une signification autre que H,
et/ou, si on le souhaite,
une base ou un acide de formule I est converti(e) en l'un de ses sels.

13. Procédé selon la revendication 12, **caractérisé en ce que** la réaction est effectuée en présence d'un acide protonique ou d'un acide de Lewis.

14. Procédé selon la revendication 12 ou 13, **caractérisé en ce que** la réaction est effectuée en présence d'acide trifluoroacétique, d'hexafluoroisopropanol, de chlorure de bismuth(III), de triflate d'ytterbium(III), de triflate de scandium(III) ou de nitrate de cérium(IV) et d'ammonium.

15. Médicament comprenant au moins un composé de formule I selon les revendications 1 à 11 et/ou des sels, solvats, tautomères et stéréoisomères de celui-ci, y compris des mélanges de ceux-ci en toutes proportions, et éventuellement des excipients et/ou des adjuvants.

16. Mélange comprenant un ou plusieurs composés de formule I selon les revendications 1-11 et une quantité d'un ou plusieurs composés de formule V 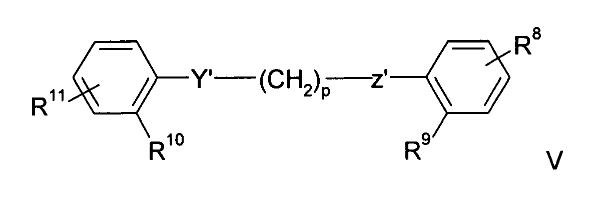 dans laquelle
Y' et Z' désignent chacun, indépendamment les uns des autres, O ou N, R⁹ et R¹⁰ désignent chacun, indépendamment les uns des autres, H, OH, halogène, OC₁₋₁₀-alkyle, OCF₃, NO₂ ou NH₂, n désigne un entier compris entre 2 et 6, chacun inclus, et R⁸ et R¹¹ sont chacun, indépendamment les uns des autres, en position méta ou para et sont choisis dans le groupe constitué par :

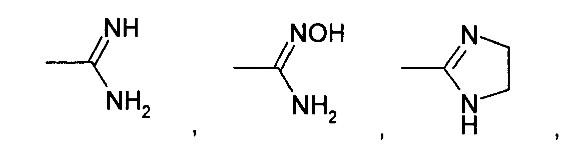

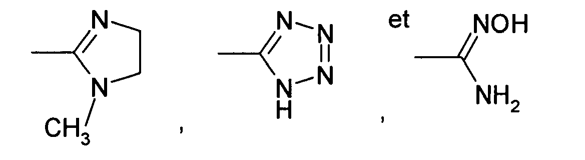

17. Mélange selon la revendication 16, où le composé de formule V utilisé est la pentamidine ou des sels de celle-ci.

18. Utilisation de composés selon les revendications 1 à 11 et de sels, solvats, tautomères et stéréoisomères de ceux-ci, y compris des mélanges de ceux-ci en toutes proportions, ou du mélange selon la revendication 16, pour la préparation d'un médicament destiné au traitement de maladies pouvant être influencées par l'inhibition, la régulation et/ou la modulation de la protéine moteur mitotique Eg5.

19. Utilisation de composés selon les revendications 1 à 11 ou du mélange selon la revendication 16, pour la préparation d'un médicament destiné au traitement et à la prophylaxie des maladies cancéreuses.

20. Utilisation selon la revendication 19, dans laquelle les maladies cancéreuses sont accompagnées d'une tumeur issue du groupe des tumeurs de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, su système lymphatique, de l'estomac, du larynx et/ou des poumons.

21. Utilisation selon la revendication 20, dans laquelle la tumeur est issue du groupe constitué par la leucémie monocytique, l'adénocarcinome du poumon, les carcinomes du poumon à petites cellules, le cancer du pancréas, les glioblastomes et le carcinome du sein et le carcinome du côlon.

22. Utilisation selon la revendication 19, dans laquelle la maladie cancéreuse à traiter est une tumeur du système sanguin et immunitaire.

23. Utilisation selon la revendication 22, dans laquelle la tumeur est issue du groupe constitué par la leucémie myéloïde aiguë, la leucémie myéloïde chronique, la leucémie lymphatique aiguë et/ou la leucémie lymphatique chronique.

24. Utilisation de composés de formule I selon les revendications 1 à 11 et/ou de sels et solvats physiologiquement acceptables de ceux-ci pour la préparation d'un médicament destiné au traitement de tumeurs en combinaison avec une quantité thérapeutiquement efficace d'un ou plusieurs composés de formule V 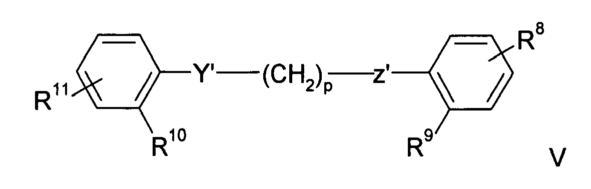 dans laquelle
Y' et Z' désignent chacun, indépendamment les uns des autres, O ou N, R⁹ et R¹⁰ désignent chacun, indépendamment les uns des autres, H, OH, halogène, OC₁₋₁₀-alkyle, OCF₃, NO₂ ou NH₂, n désigne un entier compris entre 2 et 6, chacun inclus, et R⁸ et R¹¹ sont chacun, indépendamment les uns des autres, en position méta ou para et sont choisis dans le groupe constitué par : 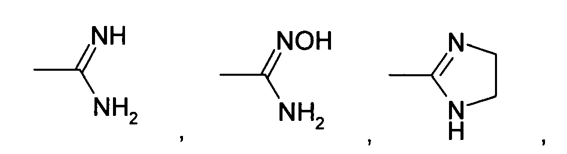 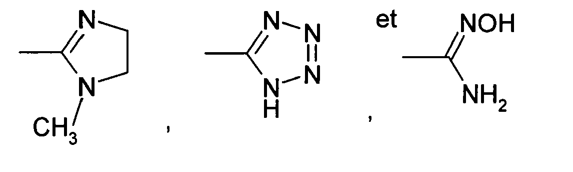 où
les composés de formule I et les composés de formule V sont administrés simultanément ou dans les 14 jours l'un par rapport à l'autre en des quantités qui sont suffisantes pour inhiber la croissance d'une tumeur ou d'autres cellules hyper-prolifératives.

25. Utilisation selon la revendication 24, dans laquelle le composé de formule V utilisé est la pentamidine ou des sels de celle-ci.

26. Composés intermédiaires de formule 1A 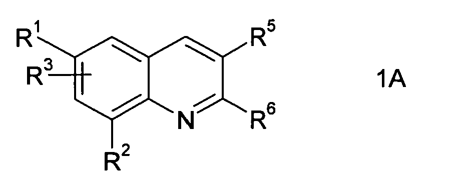 dans laquelle R¹, R², R³, R⁵ et R⁶ ont la signification indiquée dans la revendication 1.

27. Utilisation des composés de formule 1A selon la revendication 26, pour la préparation des composés de formule I en effectuant une réduction à l'aide d'hydrogène en présence de catalyseurs métalliques ou de sodium en présence d'éthanol.
